# EUROPEAN PATENT APPLICATION

(11) **EP 4 699 561 A1**
(43) Date of publication of application: **25.02.2026**
(21) Application number: 25197720.3
(22) Date of filing: 22.08.2025
(51) Int. Cl.: A61B 34/10, A61B 90/00, A61B 34/00, G06N 3/08

(54) **SYSTEMS AND METHODS FOR VISUALLY GUIDING BONE REMOVAL DURING A SURGICAL PROCEDURE ON A JOINT**

(30) Priority: 23.08.2024 US 202463686516 P
(71) Applicant: Stryker Corporation, Portage, MI 49002 (US)
(72) Inventor: ACLO, Mary Katherine, Portage, 49002 (US); FOUTS, Brian, Portage, 49002 (US); SUBRAHMANYAM, Joshua, Portage, 49002 (US); GODBEY, Ruth, Portage, 49002 (US); ZEH, Christopher, Portage, 49002 (US); LAMBERS, Floor Mariet, Portage, 49002 (US); FLOM, James, Portage, 49002 (US)
(74) Representative: V.O.

(57) **Abstract**

A computing system for guiding bone removal for a surgical procedure includes one or more programs that include instructions for receiving a two-dimensional image of bony anatomy; determining an alignment of a pre-generated three-dimensional model of the bony anatomy to the bony anatomy in the two-dimensional image; and generating and displaying a visualization comprising: a first portion comprising an overlay of at least a portion of the three-dimensional model on the two-dimensional image, wherein the overlay of at least a portion of the three-dimensional model is displayed according to the determined alignment, and a second portion comprising a representation of at least a portion of the three-dimensional model and a representation of the determined alignment of the three-dimensional model of the bony anatomy to the bony anatomy in the two-dimensional image.

## Description

### CROSS-REFERENCE TO RELATED APPLICATION

This application claims the benefit of U.S. Provisional Application 63/686,516 filed on August 23, 2024, the entire contents of which are incorporated herein by reference for all purposes.

### FIELD

This disclosure relates to orthopedics in general, and more particularly to surgical methods and systems for treating a joint.

### BACKGROUND

Orthopedics is a medical specialty that focuses on the diagnosis, correction, prevention, and treatment of patients with skeletal conditions, including, for example, conditions or disorders of the bones, joints, muscles, ligaments, tendons, nerves, and skin, which make up the musculoskeletal system. Joint injuries or conditions such as those of the hip joint or other joints can occur from overuse or over-stretching or due to other factors, including genetic factors that may cause deviations from "normal" joint morphology.

Joints are susceptible to a number of different pathologies (e.g., conditions or disorders, which may cause deviation from the normal joint morphology). These pathologies can have both congenital and injury-related origins. In some cases, the pathology can be substantial at the outset. In other cases, the pathology may be minor at the outset but, if left untreated, may worsen over time. More particularly, in many cases an existing pathology may be exacerbated, for example, by the dynamic nature of the joint, the substantial forces imposed on the joint, or a combination thereof. The pathology may, either initially or thereafter, significantly interfere with patient comfort and lifestyle and may require surgical treatment.

The current trend in orthopedic surgery is to treat joint pathologies using minimally invasive techniques such as joint arthroscopy, in which an endoscope is inserted into the joint through a small incision. Procedures performed arthroscopically include debridement of bony pathologies, in which portions of bone in a joint that deviate from a "normal" or target morphology are removed. During a debridement procedure, the surgeon uses an endoscopic camera to view the debridement area, but because the resulting endoscopic image has a limited field of view and is somewhat distorted, the surgeon cannot view the entire pathology all at once. As a result, it is generally quite difficult for the surgeon to determine exactly how much bone should be removed, and whether the shape of the remaining bone has the desired geometry.

### SUMMARY

According to various aspects, systems and methods can generate and display an overlay of a three-dimensional model of bony anatomy on a two-dimensional image of the bony anatomy to guide a surgeon during a surgical procedure on a joint, such as a bone removal surgery. The two-dimensional image can be an intra-operatively generated image (e.g., an X-ray image) showing the joint in its current state during the procedure, and the three-dimensional model can be a pre-operatively generated model of the joint that enables a surgeon to view the joint from various angles. The three-dimensional model can include a representation of planned bone removal in three-dimensional space. Thus, the overlay can illustrate where bone should be removed both at the profile of the bone captured in the two-dimensional image and portions of the joint that are beyond the two-dimensional imaging plane. By providing a three-dimensional representation of the planned bone removal as an overlay on a two-dimensional image of the current state of the joint, the surgeon can better understand where bone should be removed beyond just the profile of the bone captured in the two-dimensional image.

An overlay may be generated by determining the sufficiency of the amount of bone shown in a two-dimensional image. If the amount of bone shown in the two-dimensional image is deemed sufficient, a three-dimensional model of the joint can be aligned with the two-dimensional image using an iterative alignment optimization process. An overlay of the three-dimensional model on the two-dimensional image can then be generated based on the determined alignment and provided to a surgeon.

Various properties of the overlay can be manipulated to further assist the surgeon in the bone removal surgery. For instance, the overlay can be configured to display a visual indication of planned bone removal, representations of surgical tools in the imaged area, and/or the plane of the two-dimensional image intersecting the three-dimensional model. The transparency of the overlay can also be changed to view more or less of the two-dimensional image underneath the three-dimensional model.

A method for guiding bone removal comprises: receiving a two-dimensional image of a portion of bony anatomy; determining at least one characteristic of the portion of the bony anatomy associated with an amount of the bony anatomy in the two-dimensional image; determining a sufficiency of the amount of the bony anatomy in the two-dimensional image for determining an alignment of a pre-generated three-dimensional model of the bony anatomy to the bony anatomy in the two-dimensional image by comparing the at least one characteristic of the portion of the bony anatomy with at least one predetermined criteria; and providing a user indication associated with the sufficiency of the amount of the bony anatomy in the two-dimensional image for determining the alignment of the pre-generated three-dimensional model of the bony anatomy to the bony anatomy in the two-dimensional image.

Determining the at least one characteristic may include determining whether at least one anatomical landmark is present in the two-dimensional image. Determining the at least one characteristic may include analyzing the two-dimensional image to identify at least one anatomical landmark. Determining the at least one characteristic may include determining a ratio corresponding to at least a predetermined region of the bony anatomy. The at least one predetermined criteria may include a ratio. The at least one characteristic may include a presence or absence of one or more anatomical landmarks in the two-dimensional image, the at least one predetermined criteria may include a list of one or more anatomical landmarks, and comparing the at least one characteristic of the portion of the bony anatomy with the at least one predetermined criteria may include determining whether one or more anatomical landmarks from the list are present in the two-dimensional image.

The method may include determining the alignment of the pre-generated three-dimensional model of the bony anatomy to the bony anatomy in the two-dimensional image and displaying a visualization based on the determined alignment of the pre-generated three-dimensional model of the bony anatomy to the bony anatomy in the two-dimensional image. The visualization may include the user indication associated with the sufficiency of the amount of the bony anatomy in the two-dimensional image for determining the alignment of the pre-generated three-dimensional model of the bony anatomy to the bony anatomy in the two-dimensional image. The user indication may indicate an assessment of reliability of the alignment of the pre-generated three-dimensional model of the bony anatomy to the bony anatomy in the two-dimensional image. The visualization may include an overlay of at least a portion of the pre-generated three-dimensional model on the bony anatomy in the two-dimensional image.

Determining the sufficiency of the amount of the bony anatomy in the two-dimensional image for determining the alignment of the pre-generated three-dimensional model of the bony anatomy to the bony anatomy in the two-dimensional image may include determining that the amount of the bony anatomy in the two-dimensional image is insufficient for determining the alignment of the pre-generated three-dimensional model of the bony anatomy to the bony anatomy in the two-dimensional image. The user indication may be configured to indicate to a user that a different two-dimensional image of the bony anatomy is required. The user indication associated with the sufficiency of the amount of the bony anatomy in the two-dimensional image for determining the alignment of the pre-generated three-dimensional model of the bony anatomy to the bony anatomy in the two-dimensional image may be provided only if the amount of the bony anatomy in the two-dimensional image is determined to be sufficient for determining the alignment of the pre-generated three-dimensional model of the bony anatomy to the bony anatomy in the two-dimensional image.

A system for guiding bone removal during a surgical procedure comprises one or more processors, memory, and one or more programs, wherein the one or more programs are stored in the memory and configured to be executed by the one or more processors, the one or more programs including instructions for: receiving a two-dimensional image of a portion of bony anatomy; determining at least one characteristic of the portion of the bony anatomy associated with an amount of the bony anatomy in the two-dimensional image; determining a sufficiency of the amount of the bony anatomy in the two-dimensional image for determining an alignment of a pre-generated three-dimensional model of the bony anatomy to the bony anatomy in the two-dimensional image by comparing the at least one characteristic of the portion of the bony anatomy with at least one predetermined criteria; and providing a user indication associated with the sufficiency of the amount of the bony anatomy in the two-dimensional image for determining the alignment of the pre-generated three-dimensional model of the bony anatomy to the bony anatomy in the two-dimensional image.

Determining the at least one characteristic may include determining whether at least one anatomical landmark is present in the two-dimensional image. Determining the at least one characteristic may include analyzing the two-dimensional image to identify at least one anatomical landmark. Determining the at least one characteristic may include determining a ratio corresponding to at least a predetermined region of the bony anatomy. The at least one predetermined criteria may include a ratio. The at least one characteristic may include a presence or absence of one or more anatomical landmarks in the two-dimensional image, the at least one predetermined criteria may include a list of one or more anatomical landmarks, and comparing the at least one characteristic of the portion of the bony anatomy with the at least one predetermined criteria may include determining whether one or more anatomical landmarks from the list are present in the two-dimensional image.

The one or more programs may include instructions for determining the alignment of the pre-generated three-dimensional model of the bony anatomy to the bony anatomy in the two-dimensional image and displaying a visualization based on the determined alignment of the pre-generated three-dimensional model of the bony anatomy to the bony anatomy in the two-dimensional image. The visualization may include the user indication associated with the sufficiency of the amount of the bony anatomy in the two-dimensional image for determining the alignment of the pre-generated three-dimensional model of the bony anatomy to the bony anatomy in the two-dimensional image. The user indication may indicate an assessment of reliability of the alignment of the pre-generated three-dimensional model of the bony anatomy to the bony anatomy in the two-dimensional image. The visualization may include an overlay of at least a portion of the pre-generated three-dimensional model on the bony anatomy in the two-dimensional image.

Determining the sufficiency of the amount of the bony anatomy in the two-dimensional image for determining the alignment of the pre-generated three-dimensional model of the bony anatomy to the bony anatomy in the two-dimensional image may include determining that the amount of the bony anatomy in the two-dimensional image is insufficient for determining the alignment of the pre-generated three-dimensional model of the bony anatomy to the bony anatomy in the two-dimensional image. The user indication may be configured to indicate to a user that a different two-dimensional image of the bony anatomy is required. The user indication associated with the sufficiency of the amount of the bony anatomy in the two-dimensional image for determining the alignment of the pre-generated three-dimensional model of the bony anatomy to the bony anatomy in the two-dimensional image may be provided only if the amount of the bony anatomy in the two-dimensional image is determined to be sufficient for determining the alignment of the pre-generated three-dimensional model of the bony anatomy to the bony anatomy in the two-dimensional image.

A method for guiding bone removal for a surgical procedure comprises: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment; receiving a user request to change a transparency of the overlay of at least a portion of a three-dimensional model; and updating the visualization by changing the transparency of the overlay according to the user request.

The at least a portion of the three-dimensional model may include a representation of the bony anatomy. The at least a portion of the three-dimensional model may include a representation of planned bone removal. The user request may include a selection of a predetermined level of transparency. The user request may include an input to a graphical slider displayed in association with the visualization.

A system for guiding bone removal for a surgical procedure comprises one or more processors, memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment; receiving a user request to change a transparency of the overlay of at least a portion of a three-dimensional model; and updating the visualization by changing the transparency of the overlay according to the user request.

The at least a portion of the three-dimensional model may include a representation of the bony anatomy. The at least a portion of the three-dimensional model may include a representation of planned bone removal. The user request may include a selection of a predetermined level of transparency. The user request may include an input to a graphical slider displayed in association with the visualization.

A method for guiding bone removal for a surgical procedure comprises: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment and the overlay of at least a portion of the three-dimensional model comprises a visual indication of planned bone removal; receiving a user request to toggle off display of the visual indication of planned bone removal; and updating the visualization by ceasing to display the visual indication of planned bone removal while continuing to display the two-dimensional image.

The visual indication of planned bone removal may include a heat map. The overlay may include a representation of at least a portion of the bony anatomy and updating the visualization comprises ceasing to display the representation of the at least a portion of the bony anatomy. The overlay may include a representation of at least a portion of the bony anatomy and updating the visualization comprises continuing to display the representation of the at least a portion of the bony anatomy.

A system for guiding bone removal for a surgical procedure comprises one or more processors, memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment and the overlay of at least a portion of the three-dimensional model comprises a visual indication of planned bone removal; receiving a user request to toggle off display of the visual indication of planned bone removal; and updating the visualization by ceasing to display the visual indication of planned bone removal while continuing to display the two-dimensional image.

The visual indication of planned bone removal may include a heat map. The overlay may include a representation of at least a portion of the bony anatomy and updating the visualization comprises ceasing to display the representation of the at least a portion of the bony anatomy. The overlay may include a representation of at least a portion of the bony anatomy and updating the visualization comprises continuing to display the representation of the at least a portion of the bony anatomy.

A method for guiding bone removal for a surgical procedure comprises: receiving a two-dimensional image of bony anatomy, the two-dimensional image comprising at least a portion of a surgical tool; identifying the at least a portion of the surgical tool in the two-dimensional image; determining an alignment of a pre-generated three-dimensional model of the bony anatomy to the bony anatomy in the two-dimensional image; and generating and displaying a visualization comprising an overlay of at least a portion of the three-dimensional model on the two-dimensional image and a representation of the at least a portion of the surgical tool, wherein the at least a portion of the three-dimensional model is overlaid on the two-dimensional image according to the determined alignment.

The representation of the at least a portion of the surgical tool may be overlaid on the at least a portion of the three-dimensional model in the visualization. The method may include toggling off the overlay of the representation of the at least a portion of the surgical tool on the at least a portion of the three-dimensional model in the visualization; and toggling on an overlay of the representation of the at least a portion of the surgical tool on the two-dimensional image, behind the three-dimensional model in the visualization. The representation of the at least a portion of the surgical tool may include a silhouette of the at least a portion of the surgical tool. The representation of the at least a portion of the surgical tool may include an outline of the at least a portion of the surgical tool. The representation of the at least a portion of the surgical tool may include a three-dimensional representation of the at least a portion of the surgical tool. The representation of the at least a portion of the surgical tool may be displayed with an amount of transparency. The method may include toggling off display of the representation of the at least a portion of the surgical tool. The method may include determining a three-dimensional pose of the at least a portion of the surgical tool in the two-dimensional image, wherein the representation of the at least a portion of the surgical tool is displayed in the visualization according to the determined three-dimensional pose. The at least a portion of the three-dimensional model may include a representation of planned bone removal.

A system for guiding bone removal for a surgical procedure comprises one or more processors, memory, and one or more programs, wherein the one or more programs are stored in the memory and configured to be executed by the one or more processors, the one or more processors including instructions for: receiving a two-dimensional image of bony anatomy, the two-dimensional image comprising at least a portion of a surgical tool; identifying the at least a portion of the surgical tool in the two-dimensional image; determining an alignment of a pre-generated three-dimensional model of the bony anatomy to the bony anatomy in the two-dimensional image; and generating and displaying a visualization comprising an overlay of at least a portion of the three-dimensional model on the two-dimensional image and a representation of the at least a portion of the surgical tool, wherein the at least a portion of the three-dimensional model is overlaid on the two-dimensional image according to the determined alignment.

The representation of the at least a portion of the surgical tool may be overlaid on the at least a portion of the three-dimensional model in the visualization. The one or more programs may include instructions for toggling off the overlay of the representation of the at least a portion of the surgical tool on the at least a portion of the three-dimensional model in the visualization; and toggling on an overlay of the representation of the at least a portion of the surgical tool on the two-dimensional image, behind the three-dimensional model in the visualization. The representation of the at least a portion of the surgical tool may include a silhouette of the at least a portion of the surgical tool. The representation of the at least a portion of the surgical tool may include an outline of the at least a portion of the surgical tool. The representation of the at least a portion of the surgical tool may include a three-dimensional representation of the at least a portion of the surgical tool. The representation of the at least a portion of the surgical tool may be displayed with an amount of transparency. The one or more programs may include instructions for toggling off display of the representation of the at least a portion of the surgical tool. The one or more programs may include instructions for determining a three-dimensional pose of the at least a portion of the surgical tool in the two-dimensional image, wherein the representation of the at least a portion of the surgical tool is displayed in the visualization according to the determined three-dimensional pose. The at least a portion of the three-dimensional model may include a representation of planned bone removal.

A method for guiding bone removal for a surgical procedure comprises: receiving a two-dimensional image of bony anatomy; determining an alignment of a pre-generated three-dimensional model of the bony anatomy to the bony anatomy in the two-dimensional image; and generating and displaying a visualization comprising: a first portion comprising an overlay of at least a portion of the three-dimensional model on the two-dimensional image, wherein the overlay of at least a portion of the three-dimensional model is displayed according to the determined alignment, and a second portion comprising a representation of at least a portion of the three-dimensional model and a representation of the determined alignment of the three-dimensional model of the bony anatomy to the bony anatomy in the two-dimensional image.

The representation of the determined alignment may include a plane intersecting the at least a portion of the three-dimensional model according to the determined alignment. The representation of the determined alignment may include a representation of the two-dimensional image intersecting the at least a portion of the three-dimensional model according to the determined alignment. The orientation of the representation of at least a portion of the three-dimensional model may be fixed relative to the orientation of the representation of the determined alignment of the three-dimensional model of the bony anatomy to the bony anatomy in the two-dimensional image and the two representations together form a combined representation that is rotatable. The at least a portion of the three-dimensional model may include a representation of planned bone removal. The representation of the determined alignment may correspond to a clock face line of the bony anatomy. The method may include cutting a cross-section of the three-dimensional model according to the clock face line; and displaying the cross-section of the three-dimensional model. The method may include displaying the representation of the determined alignment with the cross-section of the three-dimensional model.

A system for guiding bone removal for a surgical procedure comprises one or more processors, a memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; and generating and displaying a visualization comprising: a first portion comprising an overlay of at least a portion of the three-dimensional model on the two-dimensional image, wherein the overlay of at least a portion of the three-dimensional model is displayed according to the determined alignment, and a second portion comprising a representation of at least a portion of the three-dimensional model and a representation of the determined alignment of the three-dimensional model of the bony anatomy to the bony anatomy in the two-dimensional image.

The representation of the determined alignment may include a plane intersecting the at least a portion of the three-dimensional model according to the determined alignment. The representation of the determined alignment may include a representation of the two-dimensional image intersecting the at least a portion of the three-dimensional model according to the determined alignment. The orientation of the representation of at least a portion of the three-dimensional model may be fixed relative to the orientation of the representation of the determined alignment of the three-dimensional model of the bony anatomy to the bony anatomy in the two-dimensional image and the two representations together form a combined representation that is rotatable. The at least a portion of the three-dimensional model may include a representation of planned bone removal. The representation of the determined alignment may correspond to a clock face line of the bony anatomy. The one or more programs may include instructions for cutting a cross-section of the three-dimensional model according to the clock face line; and displaying the cross-section of the three-dimensional model. The one or more programs may include instructions for displaying the representation of the determined alignment with the cross-section of the three-dimensional model.

Any of the methods described herein can be computer implemented methods. The methods for guiding bone removal can provide guidance to a surgeon, e.g. during a surgical procedure on a joint, such as a bone removal surgery. The methods for guiding bone removal do not include a step of a performing a surgical procedure, such as bone removal.

It will be appreciated that any of the variations, aspects, features, and options described in view of the systems apply equally to the methods and vice versa. It will also be clear that any one or more of the above variations, aspects, features, and options can be combined.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.

The invention will now be described, by way of example only, with reference to the accompanying drawings, in which:
FIGS. 1A-1D are schematic views showing various aspects of, for example, hip motion;
FIG. 2 is a schematic view showing bone structures in the region of the hip joint;
FIG. 3 is a schematic anterior view of the femur;
FIG. 4 is a schematic posterior view of the top end of the femur;
FIG. 5 is a schematic view of the pelvis;
FIGS. 6-12 are schematic views showing bone and soft tissue structures in the region of the hip joint;
FIG. 13 is a schematic view showing cam-type femoroacetabular impingement (i.e., cam-type FAI);
FIG. 14 is a schematic view showing pincer-type femoroacetabular impingement (i.e., pincer-type FAI);
FIG. 15 is a schematic view showing a labral tear;
FIG. 16 is a schematic view showing an Alpha Angle determination on the hip of a patient;
FIG. 17 is a schematic view showing a Center Edge Angle determination on the hip of a patient;
FIG. 18 is a schematic view of an exemplary surgical suite;
FIG. 19 illustrates an exemplary method 1900 for guiding a surgeon in the removal of bone from a portion of a joint during a surgical procedure;
FIG. 20 illustrates aspects of the application of method 1900 to a femur;
FIG. 21 illustrates an example of step 1904 for determining a sufficiency of bony anatomy in a two-dimensional image to be aligned with a three-dimensional model;
FIG. 22 illustrates an exemplary output of a machine learning model configured to identify and segment features of bony anatomy for determining a sufficiency of bony anatomy in a two-dimensional image;
FIG. 23 illustrates an example of step 1906 for determining an alignment of a three-dimensional model to a two-dimensional image;
FIG. 24 illustrates an exemplary overlay of a three-dimensional model on a two-dimensional image;
FIG. 25 illustrates an exemplary method for toggling display of a visual indication of planned bone removal;
FIG. 26A illustrates an exemplary visual indication of planned bone removal including a pixel mask of a surgical tool;
FIG. 26B illustrates an exemplary visual indication of planned bone removal including a silhouette of a surgical tool;
FIG. 26C illustrates an exemplary visual indication of planned bone removal including an outline of a surgical tool;
FIG. 27 illustrates an exemplary method for displaying a representation of a portion of a surgical tool on an overlay of a three-dimensional model on a two-dimensional image;
FIG. 28 illustrates an exemplary overlay of a surgical tool and a three-dimensional model on a two-dimensional image;
FIG. 29 illustrates an exemplary method for changing the transparency of a displayed overlay of a three-dimensional model on a two-dimensional image;
FIG. 30 illustrates an exemplary partially transparent three-dimensional model overlaid on a two-dimensional image;
FIG. 31 illustrates an exemplary method for displaying an overlay of a three-dimensional model on a two-dimensional image that includes a representation of a determined alignment of the three-dimensional model to the two-dimensional image;
FIG. 32A illustrates an exemplary visualization that includes an overlay of a three-dimensional model on a two-dimensional image and a plane representing a determined alignment between the three-dimensional model and the plane of the two-dimensional image;
FIG. 32B illustrates an exemplary visualization in which a two-dimensional image intersects a three-dimensional model at an imaging plane of the two-dimensional image;
FIG. 32C illustrates an exemplary visualization that includes a cross-section of a three-dimensional model cut at a two-dimensional imaging plane;
FIG. 33 illustrates an exemplary computing system;
FIGS. 34A-C illustrate exemplary visualizations of a semi-transparent three-dimensional model overlaid on a colorized two-dimensional image;
FIG. 34D illustrates an exemplary visualization in which only the visual indication of planned bone removal portion of a three-dimensional model is overlaid on the two-dimensional image;
FIG. 35 illustrates an exemplary graphical user interface for toggling three-dimensional model alignment determination on and off;
FIG. 36 illustrates an exemplary user interface that enables a user to modify an annotated two-dimensional image and store the annotated two-dimensional image in association with a checklist;
FIG. 37 illustrates an exemplary user interface that includes a checklist summary that can enable a user to visualize the progress of a surgical procedure and/or the completion of documentation for a surgical procedure;
FIG. 38A is a flow diagram of an exemplary method for using a bone removal guidance visualization to mark at least a portion of a perimeter of a planned resection of bony anatomy;
FIG. 38B illustrates an exemplary method for including virtual marks in a visualization that indicate marks made in the anatomy by the surgeon; and
FIGS. 39A-C illustrate exemplary visualizations used for marking a perimeter of a planned resection area.

### DETAILED DESCRIPTION

Reference will now be made in detail to implementations and embodiments of various aspects and variations of the invention, examples of which are illustrated in the accompanying drawings. Various devices, systems, and methods are described herein. Although at least two variations of the devices, systems, and methods are described, other variations may include aspects of the devices, systems, and methods described herein combined in any suitable manner having combinations of all or some of the aspects described. Example embodiments will now be described more fully hereinafter with reference to the accompanying drawings; however, they may be embodied in different forms and should not be construed as limited to the embodiments set forth herein. Rather, these embodiments are provided so that this disclosure will be thorough and complete, and will fully convey exemplary implementations to those skilled in the art.

According to various aspects, systems, and methods according to the principles described herein can provide an overlay of a three-dimensional model of bony anatomy on a two-dimensional image of the bony anatomy to guide a surgeon during a surgical procedure on a joint. The three-dimensional model can include a representation of planned bone removal in three-dimensional space so that a surgeon can visualize where bone should be removed both at the profile of the bone captured in the two-dimensional image and portions of the joint that are beyond the two-dimensional imaging plane. The two-dimensional image can be analyzed to determine an alignment of the three-dimensional image so that the representation of planned bone removal can be accurately positioned in the overlay.

Generating the overlay may be predicated on first determining the sufficiency of the amount of bone shown in a two-dimensional image for determining (or accurately determining) the alignment of the three-dimensional model with the two-dimensional image. A threshold amount of bony anatomy may be required to be shown in the two-dimensional image in order to accurately align the three-dimensional model to the two-dimensional image. If the two-dimensional image does not capture enough of the bony anatomy (e.g., the bony anatomy is too enlarged in the image such that too little of it is captured or the bony anatomy is too far off-center in the image), the orientation of the bony anatomy in the image may be difficult to determine, and the alignment of the overlay may be inaccurate. As such, prior to generating the overlay, a sufficiency of the amount of bony anatomy in the two-dimensional image may be assessed. Determining the sufficiency of the amount of bone in the two-dimensional image may include, for example, determining the presence or absence of one or more specific anatomical features in the image and/or determining a ratio of the bony anatomy in the image that corresponds to one or more specific anatomical features relative to other anatomical features. An indication can be provided to a user based on the determined sufficiency. For example, a user indication may be provided that indicates that there was an insufficient amount of bony anatomy in the image (and may prompt the user to obtain a new image) or a user indication may be provided that indicates an assessment of reliability of the alignment of the pre-generated three-dimensional model of the bony anatomy to the bony anatomy in the two-dimensional image. If the amount of bone shown in the two-dimensional image is insufficient for determining (or accurately determining) the alignment of the three-dimensional model with the two-dimensional image, then a notification can be provided to a user. The notification may prompt the user to obtain a better image and/or the notification may provide a warning that accuracy of the overlay may be low. If the amount of bone shown in the two-dimensional image is sufficient for accurately determining the alignment of the three-dimensional model with the two-dimensional image, the alignment can be determined, and the overlay can be generated.

Additionally, or alternatively, various properties of the overlay can be manipulated based on the preferences of the surgeon. For instance, the overlay can be configured to selectively display a visual indication of planned bone removal, such as a heat map, outline, or contour map, such that a user can select to display or not display the visual indication of planned bone removal. The overlay may be configured to display representations of one or more surgical tools captured in a two-dimensional image. The representation of the one or more surgical tools may be overlaid on top of the overlay of the three-dimensional model, such that the tool can be seen in relationship to the three-dimensional model. The overlay may be configured to display the plane of the two-dimensional image intersecting the three-dimensional model such that the alignment of the three-dimensional model with the two-dimensional image can be viewed from multiple angles. The transparency of the overlay can also be changed to view more or less of the two-dimensional image underneath the three-dimensional model according to a surgeon's preferences.

As used herein, "bone removal" encompasses any method of resecting bone, including drilling, sawing, burring, and bone removal using an osteotome. Guiding a surgeon during a surgical procedure on a joint, such as bone removal, as such excludes the step of the surgical procedure on the joint, such as the bone removal.

In the following description, it is to be understood that the singular forms "a," "an," and "the" used in the following description are intended to include the plural forms as well, unless the context clearly indicates otherwise. It is also to be understood that the term "and/or" as used herein refers to and encompasses any and all possible combinations of one or more of the associated listed items. It is further to be understood that the terms "includes," "including," "comprises," and/or "comprising," when used herein, specify the presence of stated features, integers, steps, operations, elements, components, and/or units but do not preclude the presence or addition of one or more other features, integers, steps, operations, elements, components, units, and/or groups thereof.

Certain aspects of the present disclosure include process steps and instructions described herein in the form of an algorithm. It should be noted that the process steps and instructions of the present disclosure could be embodied in software, firmware, or hardware and, when embodied in software, could be downloaded to reside on and be operated from different platforms used by a variety of operating systems. Unless specifically stated otherwise as apparent from the following discussion, it is appreciated that, throughout the description, discussions utilizing terms such as "processing," "computing," "calculating," "determining," "displaying," "generating," or the like, refer to the action and processes of a computer system, or similar electronic computing device, that manipulates and transforms data represented as physical (electronic) quantities within the computer system memories or registers or other such information storage, transmission, or display devices.

The present disclosure in some embodiments also relates to devices or systems for performing the operations herein. The devices or systems may be specially constructed for the required purposes, may comprise a general-purpose computer selectively activated or reconfigured by a computer program stored in the computer, or may include any combination thereof. Computer instructions for performing the operations herein can be stored in any combination of non-transitory, computer readable storage medium, such as, but not limited to, any type of disk, including floppy disks, USB flash drives, external hard drives, optical disks, CD-ROMs, magnetic-optical disks, read-only memories (ROMs), random access memories (RAMs), EPROMs, EEPROMs, magnetic or optical cards, or any type of media suitable for storing electronic instructions, and each coupled to a computer system bus. One or more instructions for performing the operations herein may be implemented in or executed by one or more Application Specific Integrated Circuits (ASICs), Field Programmable Gate Arrays (FPGAs), Digital Signal Processing units (DSPs), Graphics Processing Units (GPUs), or Central Processing Units (CPUs). Furthermore, the computers referred to herein may include a single processor or may be architectures employing multiple processor designs for increased computing capability.

The methods, devices, and systems described herein are not inherently related to any particular computer or other apparatus. Various general-purpose systems may also be used with programs in accordance with the teachings herein, or it may prove convenient to construct a more specialized apparatus to perform the required method steps. The required structure for a variety of these systems will appear from the description below. In addition, the present invention is not described with reference to any particular programming language. It will be appreciated that a variety of programming languages may be used to implement the teachings of the present invention as described herein.

Although the following examples often refer to hip joints, hip joint pathologies, and hip joint characteristics and measurements, it is to be understood that the systems, methods, techniques, visualizations, etc., described herein can be used for analyzing and visualizing other joints, including knees, shoulders, elbows, the spine, the ankle, etc.

According to various aspects, a physician can be provided with improved guidance with respect to the extent of a deviation of a joint morphology from a target morphology, and how much bone should be removed to achieve the target morphology, for example, during a minimally invasive arthroscopic procedure or open surgical procedure. Visualizations can provide a physician with improved guidance with respect to morphology measurements for a hip joint, including the Alpha Angle, Lateral Center Edge Angle, Acetabular Version and Femoral Torsion, Tönnis angle, neck shaft angle, and acetabular coverage that can help a practitioner gauge a deviation of the subject morphology from a target morphology.

Target joint morphology can be any joint morphology that may be desired for a given subject. Target joint morphology can be based on the anatomy representative of any reference patient population, such as a normal patient population. For example, baseline data can be a model of a "normal" joint that is derived from studies of a healthy patient population and/or from a model generated based on measurements, computer simulations, calculations, etc. The terms target, baseline, and reference are used interchangeably herein to describe joint morphology characteristics against which a subject's joint morphology is compared.

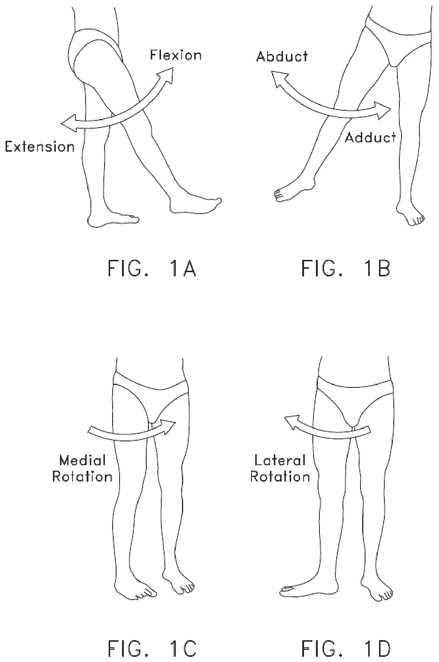

A better understanding of various joint pathologies, and the advantages provided according to various aspects described herein, can be gained from a more precise understanding of the anatomy of the joint. The hip joint is formed at the junction of the femur and the hip. The hip joint is a ball-and-socket joint and is capable of a wide range of different motions, e.g., flexion and extension, abduction and adduction, internal and external rotation, etc., as illustrated in FIGS. 1A-1D. With the possible exception of the shoulder joint, the hip joint is perhaps the most mobile joint in the body. The hip joint carries substantial loads during most of the day, in both static (e.g., standing and sitting) and dynamic (e.g., walking and running) conditions.

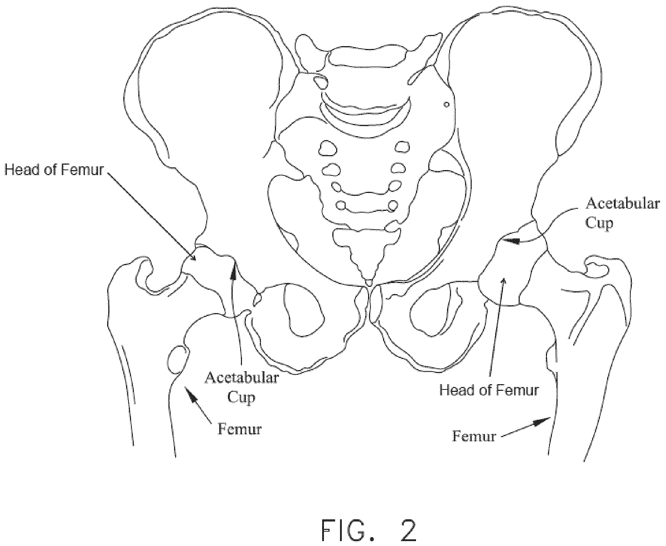

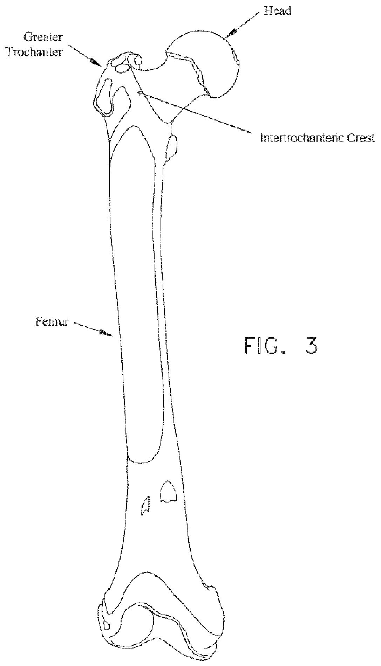

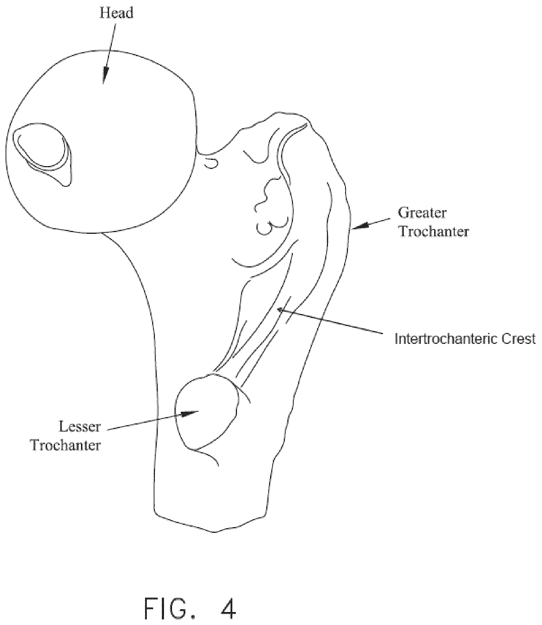

More particularly, and with reference to FIG. 2, the ball of the femur is received in the acetabular cup of the hip, with a plurality of ligaments and other soft tissue serving to hold the bones in articulating condition. As is illustrated in FIG. 3, the femur is generally characterized by an elongated body terminating, at its top end, in an angled neck that supports a hemispherical head (also sometimes referred to as the ball). As is illustrated in FIGS. 3 and 4, a large projection known as the greater trochanter protrudes laterally and posteriorly from the elongated body adjacent to the neck. A second, somewhat smaller projection known as the lesser trochanter protrudes medially and posteriorly from the elongated body adjacent to the neck. An intertrochanteric crest extends along the periphery of the femur, between the greater trochanter and the lesser trochanter.

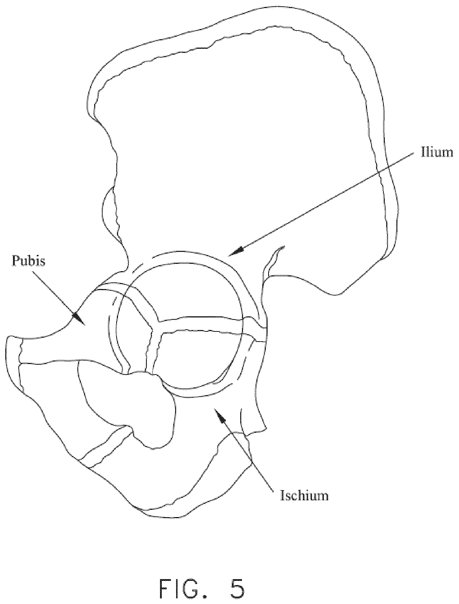

Referring to FIG. 5, the pelvis is made up of three constituent bones: the ilium, the ischium, and the pubis. These three bones cooperate with one another (they typically ossify into a single "hip bone" structure by the age of 25) so as to form the acetabular cup. The acetabular cup receives the head of the femur.

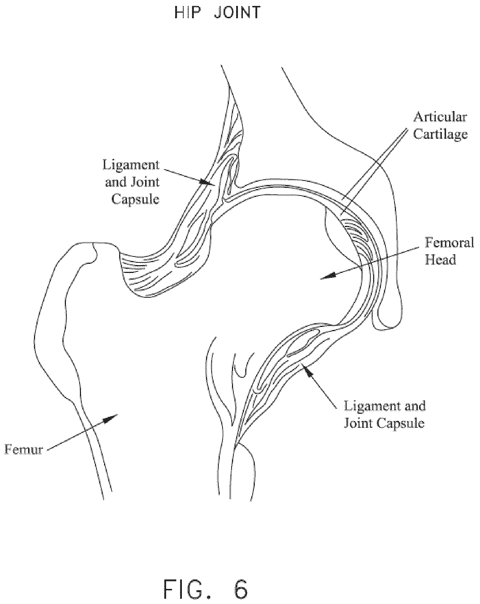

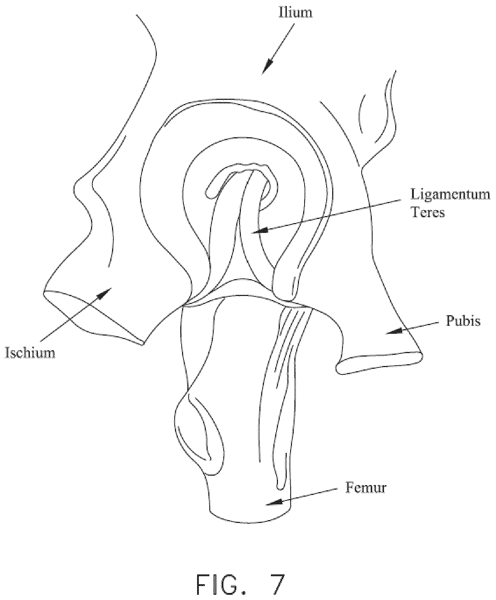

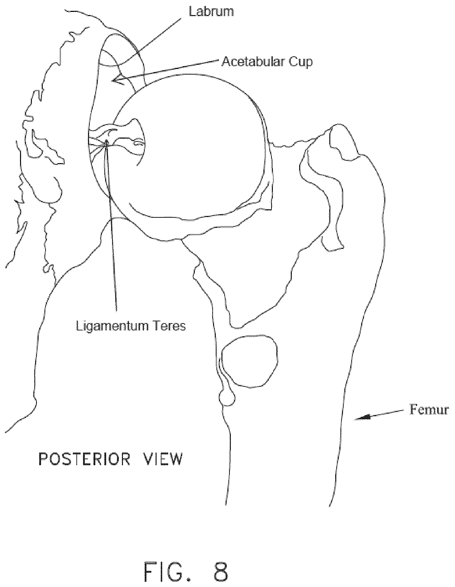

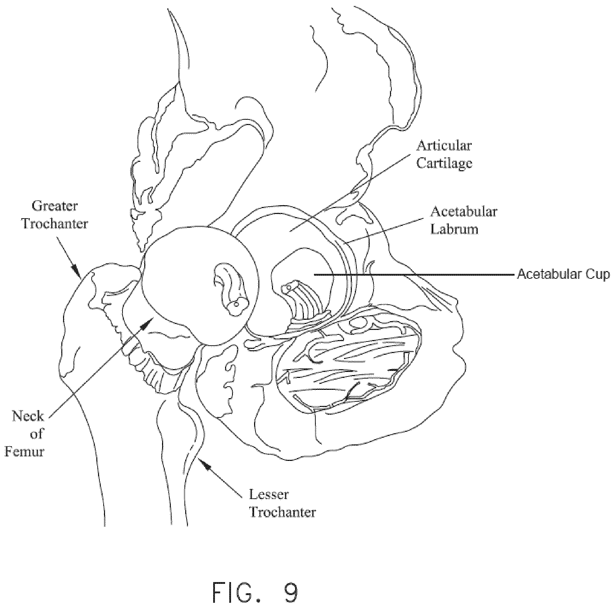

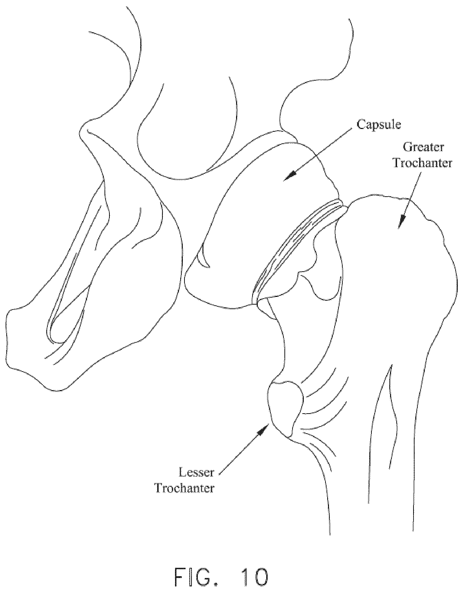

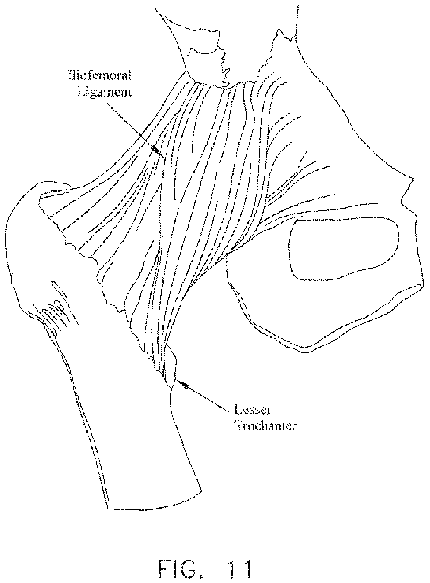

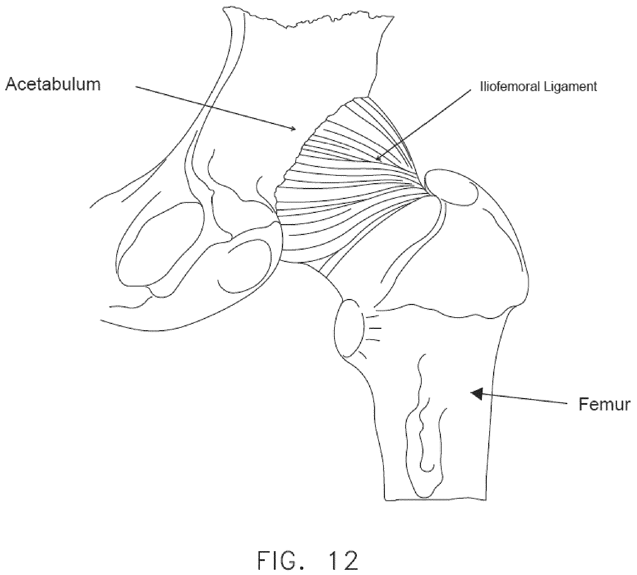

Both the head of the femur and the acetabular cup are covered with a layer of articular cartilage that protects the underlying bone and facilitates motion (see FIG. 6). Various ligaments and soft tissue serve to hold the ball of the femur in place within the acetabular cup. More particularly, and with reference to FIGS. 7 and 8, the ligamentum teres extends between the ball of the femur and the base of the acetabular cup. Referring to FIG. 9, a labrum is disposed about the perimeter of the acetabular cup. The labrum serves to increase the depth of the acetabular cup and effectively establishes a suction seal between the ball of the femur and the rim of the acetabular cup, thereby helping to hold the head of the femur in the acetabular cup. In addition, and with reference to FIG. 10, a fibrous capsule extends between the neck of the femur and the rim of the acetabular cup, effectively sealing off the ball-and-socket members of the hip joint from the remainder of the body. The foregoing structures are encompassed and reinforced by a set of three main ligaments (i.e., the iliofemoral ligament, the ischiofemoral ligament and the pubofemoral ligament) that extend between the femur and the hip (see FIGS. 11 and 12).

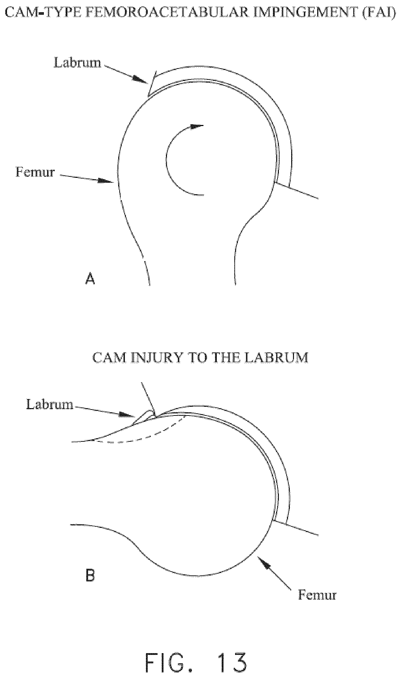

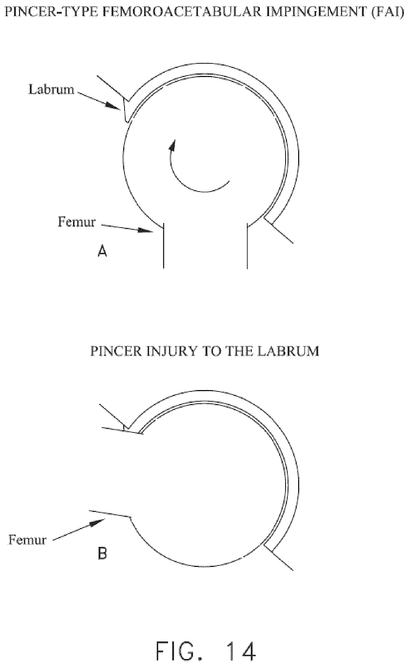

The hip joint is susceptible to a number of different pathologies. These pathologies can have, for example, both congenital and injury-related origins. For example, a congenital pathology of the hip joint involves impingement between the neck of the femur and the rim of the acetabular cup. In some cases, and with reference to FIG. 13, this impingement can occur due to irregularities in the geometry of the femur. This type of impingement is sometimes referred to as a cam-type femoroacetabular impingement (i.e., a cam-type FAI). In other cases, and with reference to FIG. 14, the impingement can occur due to irregularities in the geometry of the acetabular cup. This latter type of impingement is sometimes referred to as a pincer-type femoroacetabular impingement (i.e., a pincer-type FAI). Impingement can result in a reduced range of motion, substantial pain and, in some cases, significant deterioration of the hip joint.

Another example of congenital pathology of the hip joint involves defects in the articular surface of the ball and/or the articular surface of the acetabular cup. Defects of this type sometimes start out fairly small but often increase in size over time, generally due to the dynamic nature of the hip joint and also due to the weight-bearing nature of the hip joint. Articular defects can result in substantial pain, induce or exacerbate arthritic conditions, and, in some cases, cause significant deterioration of the hip joint.

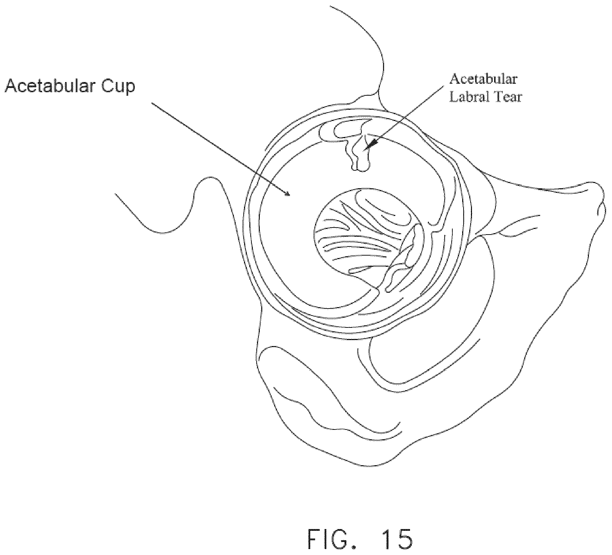

An example of injury-related pathology of the hip joint involves trauma to the labrum. In many cases, an accident or a sports-related injury can result in the labrum being torn, typically with a tear running through the body of the labrum (e.g., see FIG. 15). These types of injuries can be painful for the patient and, if left untreated, can lead to substantial deterioration of the hip joint.

The current trend in orthopedic surgery is to treat joint pathologies using minimally invasive techniques. For example, it is common to re-attach ligaments in the shoulder joint using minimally invasive, "keyhole" techniques that do not require "laying open" the capsule of the shoulder joint. Furthermore, it is common to repair, for example, torn meniscal cartilage in the knee joint, and/or to replace ruptured ACL ligaments in the knee joint, using minimally invasive techniques. While such minimally invasive approaches can require additional training on the part of the surgeon, such procedures generally offer substantial advantages for the patient and have now become the standard of care for many shoulder joint and knee joint pathologies.

In addition to the foregoing, due to the widespread availability of minimally invasive approaches for treating pathologies of the shoulder joint and knee j oint, the current trend is to provide such treatment much earlier in the lifecycle of the pathology, so as to address patient pain as soon as possible and so as to minimize any exacerbation of the pathology itself. This is in marked contrast to traditional surgical practices, which have generally dictated postponing surgical procedures as long as possible so as to spare the patient from the substantial trauma generally associated with invasive surgery.

Minimally invasive treatments for pathologies of the hip joint have lagged behind minimally invasive treatments for pathologies of the shoulder joint and knee joint. This may be, for example, due to (i) the geometry of the hip joint itself, and (ii) the nature of the pathologies that must typically be addressed in the hip joint.

The hip joint is generally considered to be a "tight" joint, in the sense that there is relatively little room to maneuver within the confines of the joint itself. This is in contrast to the knee joint, which is generally considered to be relatively spacious when compared to the hip joint. As a result, it is relatively more challenging for surgeons to perform minimally invasive procedures on the hip joint.

Furthermore, the natural pathways for entering the interior of the hip joint (i.e., the pathways that naturally exist between adjacent bones) are generally much more constraining for the hip joint than for the shoulder joint or the knee joint. This limited access further complicates effectively performing minimally invasive procedures on the hip joint.

In addition to the foregoing, the nature and location of the pathologies (e.g., conditions or disorders, which may cause deviation from the baseline anatomy of the joint) of the hip joint also complicate performing minimally invasive procedures. For example, in the case of a typical tear of the labrum in the hip joint, instruments (also referred to herein as tools) must generally be introduced into the joint space using a line of approach that is set, in some locations, at an angle of 25 degrees or more to the line of repair. This makes drilling into bone, for example, much more complex than where the line of approach is effectively aligned with the line of repair, such as is frequently the case in the shoulder joint. Furthermore, the working space within the hip joint is typically extremely limited, further complicating repairs where the line of approach is not aligned with the line of repair.

As a result of the foregoing, minimally invasive hip joint procedures continue to be relatively difficult, and patients must frequently manage their hip joint pathologies for as long as possible, until a partial or total hip replacement can no longer be avoided, whereupon the procedure is generally done as a highly invasive, open procedure, with all of the disadvantages associated with highly invasive, open procedures.

As noted above, hip arthroscopy is becoming increasingly more common in the diagnosis and treatment of various hip pathologies. However, due to the anatomy of the hip joint and the pathologies associated with the same, hip arthroscopy appears to be currently practical for only selected pathologies and, even then, hip arthroscopy has generally met with limited success.

One procedure that is sometimes attempted arthroscopically relates to femoral debridement for treatment of cam-type femoroacetabular impingement (i.e., cam-type FAI). More particularly, with cam-type femoroacetabular impingement, irregularities in the geometry of the femur can lead to impingement between the femur and the rim of the acetabular cup. Treatment for cam-type femoroacetabular impingement typically involves debriding the femoral neck and/or head, using instruments such as burrs and osteotomes, to remove the bony deformities causing the impingement. It is important to debride the femur carefully, since only bone that does not conform to the desired geometry should be removed, in order to ensure positive results as well as to minimize the possibility of bone fracture after treatment. For this reason, when debridement is performed as an open surgical procedure, surgeons generally use debridement templates having a pre-shaped curvature to guide them in removing the appropriate amount of bone from the femur.

However, when the debridement procedure is attempted arthroscopically, conventional debridement templates with their pre-shaped curvature cannot be passed through the narrow keyhole incisions, and hence debridement templates are generally not available to guide the surgeon in reshaping the bone surface. As a result, the debridement must generally be effected "freehand." In addition to the foregoing, the view of the cam pathology is also generally limited. Primarily, the surgeon uses a scope and camera to view the resection area, but the scope image has a limited field of view and is somewhat distorted. Also, because the scope is placed close to the bone surface, the surgeon cannot view the entire pathology "all at once." Secondarily, the surgeon also utilizes a fluoroscope to take X-ray images of the anatomy. These X-ray images supplement the arthroscopic view from the scope, but it is still limited to a two-dimensional representation of the three-dimensional cam pathology.

As a result of the foregoing, it is generally quite difficult for the surgeon to determine exactly how much bone should be removed, and whether the shape of the remaining bone has the desired geometry. In practice, surgeons tend to err on the side of caution and remove less bone. Significantly, under-resection of the cam pathology is the leading cause of revision hip arthroscopy.

An example of another procedure that is sometimes attempted arthroscopically relates to treatment of pincer-type femoroacetabular impingement (i.e., pincer-type FAI). More particularly, with pincer-type femoroacetabular impingement, irregularities in the geometry of the acetabulum can lead to impingement between the femur and the rim of the acetabular cup. Treatment for pincer-type femoroacetabular impingement typically involves debriding the rim of the acetabular cup using instruments such as burrs and osteotomes to remove the bony deformities causing the impingement. In some cases, the labrum is released from the acetabular bone so as to expose the underlying rim of the acetabular cup prior to debriding the rim of the acetabular cup, and then the labrum is reattached to the debrided rim of the acetabular cup. It is important to debride the rim of the acetabular cup carefully, since only bone that does not conform to the desired geometry should be removed, in order to alleviate impingement while minimizing the possibility of removing too much bone from the rim of the acetabular cup, which could cause joint instability.

However, when the debridement procedure is attempted arthroscopically, the debridement must generally be effected freehand. In this setting, it is generally quite difficult for the surgeon to determine exactly how much bone should be removed, and whether the remaining bone has the desired geometry. In practice, surgeons tend to err on the side of caution and remove less bone. Significantly, under-resection of the pincer pathology may necessitate revision hip arthroscopy.

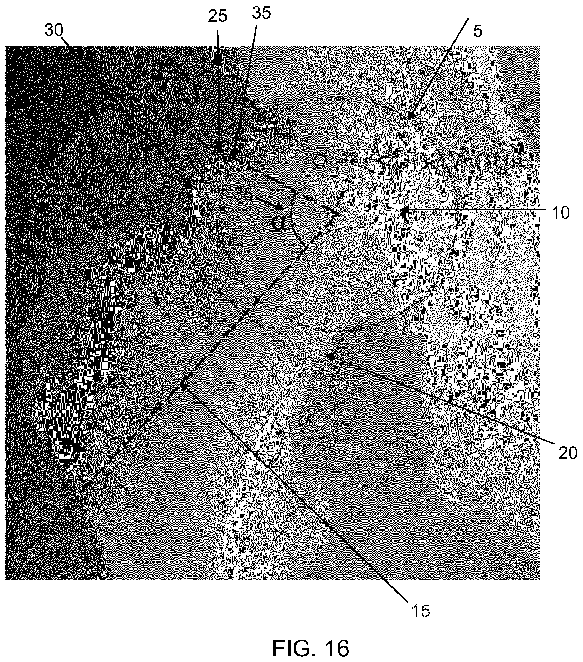

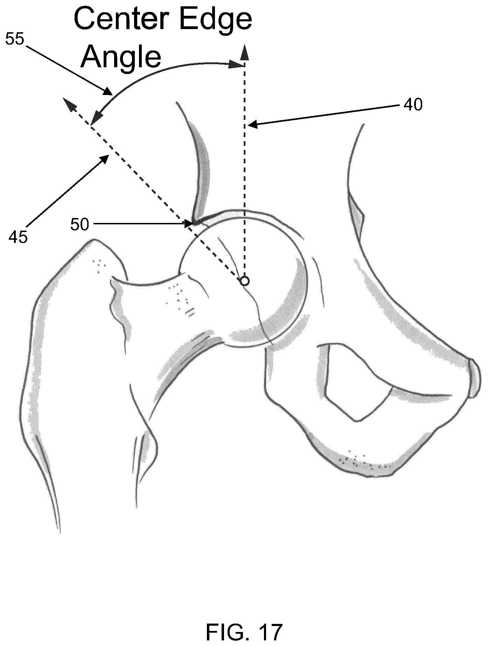

Two common anatomical measurements used in diagnosing femoroacetabular impingement (FAI) are the Alpha Angle (FIG. 16) for cam-type impingement and the Center Edge Angle (FIG. 17) for pincer-type impingement. These measurements are typically measured from pre-operative images (e.g., pre-operative X-ray images). These measurements are used to determine the degree to which the patient's hip anatomy deviates from normal (e.g., baseline), healthy hip anatomy.

For example, a healthy hip typically has an Alpha Angle of less than a threshold angle that may be defined anywhere from approximately 42 degrees to approximately 50 degrees; thus, a patient with an Alpha Angle of greater than the threshold angle (e.g. approximately 42 degrees to approximately 50 degrees) may be a candidate for FAI surgery. These are merely exemplary Alpha Angle ranges and do not limit the systems and methods herein to any particular range of Alpha Angles. During an initial examination of a patient, the surgeon will typically take an X-ray of the patient's hip. If the patient has an initial diagnosis of FAI, the patient may also obtain an MRI or CT scan of their hip for further evaluation of the bony pathology causing the FAI.

Most of today's imaging techniques (e.g., X-ray, CT, MRI) are digital, and hence the images can be imported into, and manipulated by, computer software. Using the imported digital images, the surgeon is able to measure the Alpha Angle (and/or the Center Edge Angle). For example, the surgeon imports the digital image into one of the many available software programs that use the DICOM (Digital Imaging and Communications in Medicine) standard for medical imaging. In order to make the Alpha Angle (or the Center Edge Angle) measurements with the digital image, the surgeon must first manually create and overlay geometric shapes onto the digital medical image.

For example, and with reference to FIG. 16, to measure the Alpha Angle, the surgeon manually creates a circle 5 and places it over the femoral head 10 and then manually sizes the circle such that the edge of the circle matches the edge of the femoral head. The surgeon then manually creates a line 15 and places it along the mid-line of the femoral neck 20. The surgeon then manually draws a second line 25 that originates at the center of the femoral head and passes through the location that signifies the start of the cam pathology 30 (i.e., the location where the bone first extends outside the circle set around the femoral head). The surgeon then manually selects the two lines and instructs the software to calculate the angle between the two lines; the result is the Alpha Angle 35.

Correspondingly, and with reference to FIG. 17, to measure the Center Edge Angle, the surgeon manually creates a vertical line 40 which originates at the center of the femoral head and is perpendicular to the transverse pelvic axis. The surgeon then manually draws a second line 45 that originates at the center of the femoral head and passes through the location which signifies the start of the pincer pathology 50 (i.e., the rim of the acetabular cup). The surgeon then manually selects the two lines and instructs the software to calculate the angle between the two lines; the result is the Center Edge Angle 55.

These Alpha Angle measurements (or Center Edge Angle measurements) are typically performed around the time that the patient is initially examined, which typically occurs weeks or months prior to surgery. At the time of surgery, the surgeon may bring a copy (e.g., a printout) of the Alpha Angle measurements (or the Center Edge Angle measurements) to the operating room so that the printout is available as a reference during surgery. The surgeon may also have access to these measurements with a computer located in or near the operating room, which is connected to the hospital's PACS system (Picture Archiving and Communication System). Either way, the surgeon can have the pre-operative measurements available as a reference during surgery.

However, while the surgeon is debriding bone on the cam (or pincer), the pre-operative measurements may be insufficient for adequately guiding the surgeon regarding where and how much bone should be removed due to the difficulty in comparing what the surgeon sees in the endoscopic images with the pre-operative measurements. Accordingly, as discussed further below with respect to various examples, systems and methods can guide a surgeon during a surgical procedure on a joint by displaying an overlay of a three-dimensional model of a joint on a two-dimensional image of the joint captured during the surgical procedure. The three-dimensional model may include a three-dimensional representation of planned bone removal. The three-dimensional representation of planned bone removal can indicate where bone should be removed from the joint in three-dimensional space, so that the surgeon can better understand how the planned bone removal relates to what the surgeon is seeing via the endoscopic imaging.

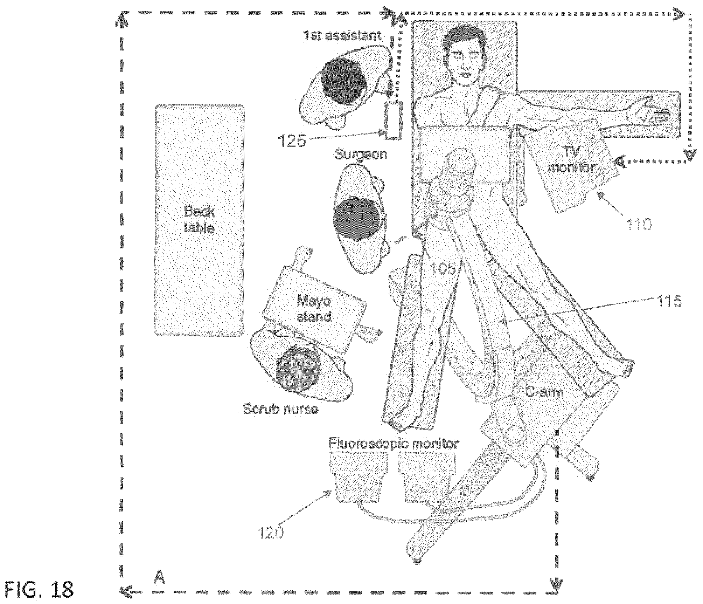

FIG. 18 illustrates an example of a surgical suite incorporating a system for guiding a surgeon in removing bone from a portion of a joint during a surgical procedure. In a typical arthroscopic surgical suite, the surgeon uses an arthroscope 105 and a display 110 to directly view an internal surgical site. In addition, the surgeon may also use a C-arm X-ray machine 115 and a fluoroscopic display 120 to image the internal surgical site. In accordance with various aspects, the surgical suite can include a visual guidance system 125 that can generate an overlay image in which a representation of bone removal extracted from a three-dimensional model of the bone is overlaid on a two-dimensional image of the bone captured intra-operatively, such as by a C-arm X-ray machine 115, according to the principles described herein, for guiding the surgeon during the surgical procedure.

Visual guidance system 125 may comprise one or more processors, memory, and one or more programs stored in the memory for causing the visual guidance system to provide the functionality disclosed herein. Visual guidance system 125 may comprise a tablet device with an integrated computer processor and user input/output functionality, e.g., a touchscreen. The visual guidance system 125 may be at least partially located in the sterile field, for example, the visual guidance system 125 may comprise a touchscreen tablet mounted to the surgical table or to a boom-type tablet support. The visual guidance system 125 may be covered by a sterile drape to maintain the surgeon's sterility as he or she operates the touchscreen tablet. Visual guidance system 125 may comprise other general purpose computers with appropriate programming and input/output functionality, e.g., a desktop or laptop computer with a keyboard, mouse, touchscreen display, heads-up display, gesture recognition device, voice activation feature, pupil reading device, etc.

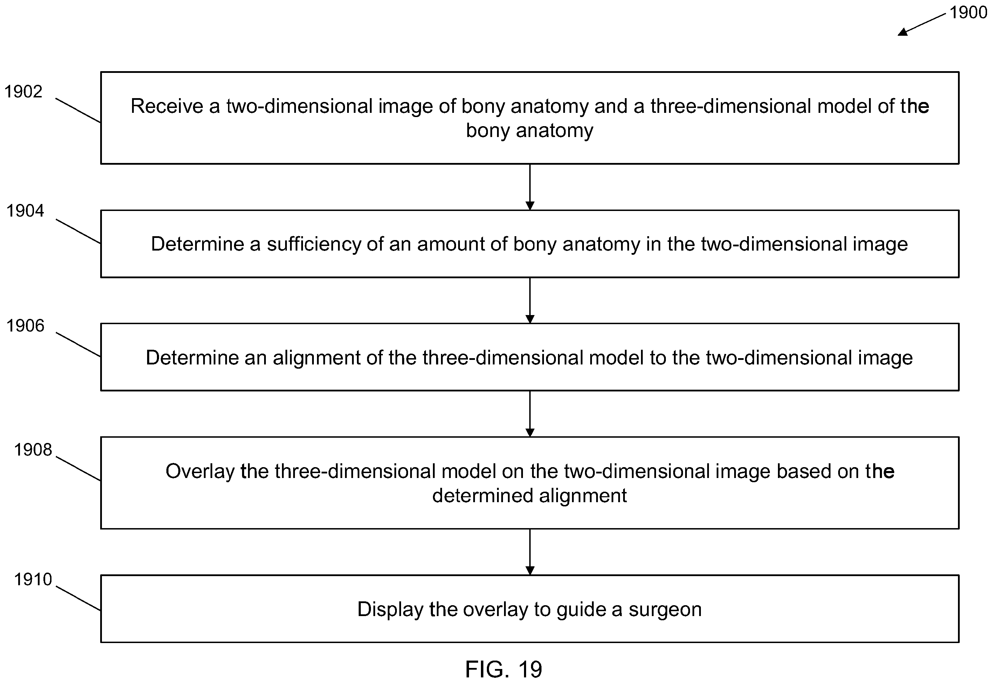

Visual guidance system 125 may be configured to generate and display visualizations that can be used to guide a surgeon in the removal of bone during surgery. FIG. 19 illustrates an exemplary method 1900 for guiding a surgeon in the removal of bone from a portion of a joint during a surgical procedure. The steps of method 1900 may, for example, be performed by one or more processors, such as the one or more processors of visual guidance system 125. Using method 1900, visual guidance system 125 may generate and display a visualization, including an overlay image that includes a three-dimensional model of a portion of a joint overlaid on a two-dimensional image (e.g., a fluoroscopic image) of the portion of the joint that is captured during the surgical procedure. The three-dimensional model shown in the overlay image can include a visualization of planned bone removal to indicate to the surgeon where and how much bone should be removed in portions of the joint that are not captured in the two-dimensional image - i.e., that are outside of the imaging plane. This can be advantageous to the surgeon by enabling the surgeon to better associate the planned bone removal with what the surgeon is seeing via endoscopic imaging. It is noted that an endoscope for the endoscopic imaging can be inserted into a lumen prior to start of the method 1900 of guiding. The method 1900 can exclude a step of inserting the endoscope. Since a two-dimensional image of the joint shows only the two-dimensional profile of the joint at the imaging plane, it is often difficult for the surgeon to identify the location in the endoscopic imaging that corresponds to the profile in the two-dimensional image and, therefore, where exactly bone should be removed. Method 1900 enables a surgeon to visualize the locations of bone for removal that are outside of the imaging plane, which enables the surgeon to better compare the planned bone removal with what the surgeon is seeing via endoscopic imaging. Method 1900 may be used for any joint of the body, including the hip joint, shoulder joint, knee joint, spinal joints, etc., and/or for any anatomy of the body.

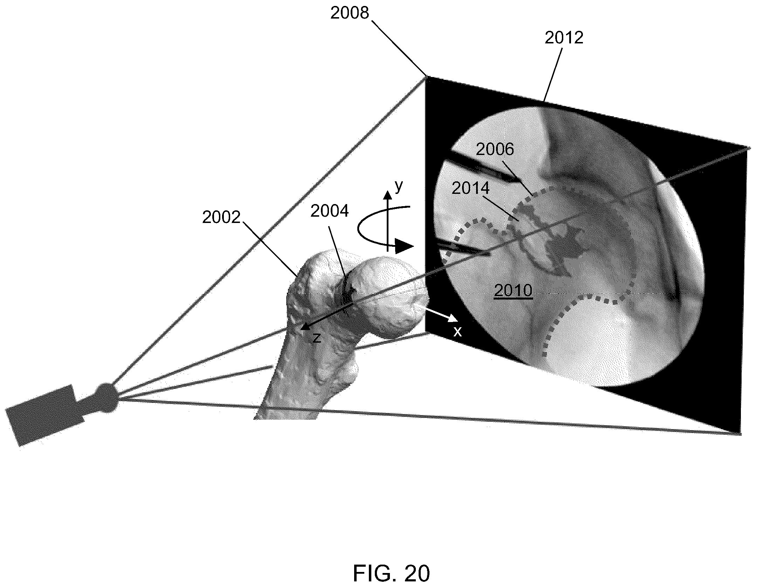

FIG. 20 illustrates aspects of the application of an example of method 1900 related to debridement of a CAM lesion on the head of a femur of a hip joint. In the illustrated example, a three-dimensional model 2002 of the upper portion of a particular patient's femur includes a representation of planned bone removal in the form of a heat map 2004 that covers the portion of bone that deviates from a target morphology and, as such, is identified for removal (e.g., via debridement). The heat map 2004 is color coded according to the planned depth of bone removal. As discussed further below, method 1900 includes steps for aligning the three-dimensional model 2002 so that a projection 2006 of the model onto a two-dimensional plane 2008 that corresponds with the imaging plane aligns with the femur 2010 in the two-dimensional image 2012. Conceptually speaking, the model 2002 is manipulated according to the available degrees of freedom - such as translations in the x, y, and z directions, rotations about these axes, and scaling relative to the viewpoint of a simulated camera (depicted as a camera in FIG. 20) - until its projection 2006 aligns sufficiently with the femur 2010 in the two-dimensional image 2012. Once this satisfactory alignment is achieved, and with continued reference to the example of FIG. 20, the three-dimensional heat map 2004 is projected onto the two-dimensional plane 2008 and rendered as an overlay 2014 on the two-dimensional image 2012 that is displayed to the surgeon intra-operatively.

Returning to FIG. 19, method 1900 may include receiving (e.g., by one or more processors of visual guidance system 125) a two-dimensional image of bony anatomy and a three-dimensional model of the bony anatomy at step 1902. The two-dimensional image generally includes the portion of the bone that is being or will be surgically treated, as well as surrounding portions of the bone that enable the surgeon to generally compare what is shown in the image to what the surgeon is seeing endoscopically. For instance, in examples involving debridement to address a CAM pathology, the two-dimensional image may include the head and neck of the femur. The two-dimensional image can be received from an intra-operative imaging system, such as an X-ray imager that is communicatively connected with the surgical guidance system performing method 1900 (e.g., C-arm X-ray machine 115 of FIG. 18). The two-dimensional image can be pre-generated (i.e., generated prior to performance of method 1900) and retrieved from a suitable image storage system. Optionally, one or more pre-processing operations may be applied to the two-dimensional image, such as one or more scaling operations, cropping operations, down-sampling, up-sampling, etc. A dewarping operation may be applied to the two-dimensional image to correct for warping caused by the imaging system. Dewarping of a two-dimensional image may be performed based on the determined relationship between a known pattern of reference markers attached to the detector of the imaging system and the reference markers visible in the two-dimensional image. For example, the reference markers in a two-dimensional image may be detected, a non-rigid transformation that maps the known positions of the reference markers to the markers visible in the image may be calculated, and the transformation may be applied to the image, resulting in a dewarped image. The reference markers may then be removed from the image.

The three-dimensional model represents at least a portion of the joint that is targeted for surgical treatment. The three-dimensional model may be generated based on a pre-operative three-dimensional scan of the patient's joint. For example, for a hip surgery, a patient's hip joint can be imaged and a model can be built from the imaging that includes various portions of the hip joint, including, for example, the femoral head, the femoral neck, the acetabular cup, the pelvis, femoral condyles, etc. The joint may be imaged using any suitable imaging system for generating three-dimensional image data, including, for example, a CT imaging system, an ultrasound tomography system, an O-arm^{™} imaging system, a beam cone CT imaging system, a C-arm imaging system, and an MRI imaging system. The three-dimensional model may be pre-generated and stored for use during the surgical session. The three-dimensional model may be generated intraoperatively, such as using an O-arm^{™} imaging system. The three-dimensional model may be updated based on images captured during the surgical procedure to reflect the removal of bone during the surgical procedure.

The three-dimensional model may include a representation of at least one region of the portion of the joint that deviates from a target morphology. This model can be used to assist a practitioner in planning for a surgical procedure on region(s) of the joint, such as by indicating to the practitioner where and how much bone should be removed. For example, a three-dimensional model of a portion of a hip joint of a subject can be generated that includes information identifying a location of a hip joint pathology (e.g., a condition or a disorder), such as an FAI, and an amount of bone that may be removed to match a baseline anatomy. The three-dimensional model can be used to generate a rendering of the portion of the joint that includes a visual representation of the location and amount of planned bone removal, such as a heat map covering the portion of bone that deviates from a target and including variation in color, contrast, or other suitable visual indicator, that indicates a degree of deviation from the target morphology. The three-dimensional model and information regarding deviations from a baseline/target anatomy can be used to generate a three-dimensional rendering of the joint that includes a visual representation of the deviations from the baseline/target anatomy. The visual representation can indicate the location and amount of bone that should be removed to achieve a target morphology. A user, such as the surgeon or a third party, can tailor the visual representation for surgical planning purposes, such as by altering one or more parameters that determine the deviations from the target bone morphology, which can increase or decrease the size of the region indicated for bone removal and/or increase or decrease the amount of bone indicated for removal.

At step 1904, a sufficiency of an amount of bony anatomy in the two-dimensional image for determining (or accurately determining) the alignment of the three-dimensional model with the two-dimensional image is determined. A threshold amount of bony anatomy may be required to be shown in the two-dimensional image in order to accurately align the three-dimensional model to the two-dimensional image. If the two-dimensional image is too close-up on the bony anatomy, the orientation of the bony anatomy in the image may be difficult to determine. For example, in the case of two-dimensional image of just a femoral head, it may be difficult to determine the orientation of the femoral head since the femoral head is roughly spherical and, thus, looks similar from different angles. This may lead to difficulty in determining the orientation of the femoral head, resulting in inaccurate alignment between the two-dimensional image of the femoral head and a corresponding three-dimensional model of the femoral head. In contrast, the presence of additional features of the femur (such as the femoral neck, the upper and lower trochanters, etc.) in the two-dimensional image may improve the ability to determine the orientation of the femur in the two-dimensional image, and thereby, improve the alignment between the two-dimensional image of the femoral head and the three-dimensional model. Accordingly, it may be advantageous to analyze a two-dimensional image before alignment with a three-dimensional model in order to determine whether there is a sufficient amount of bony anatomy in the image to enable accurate alignment.

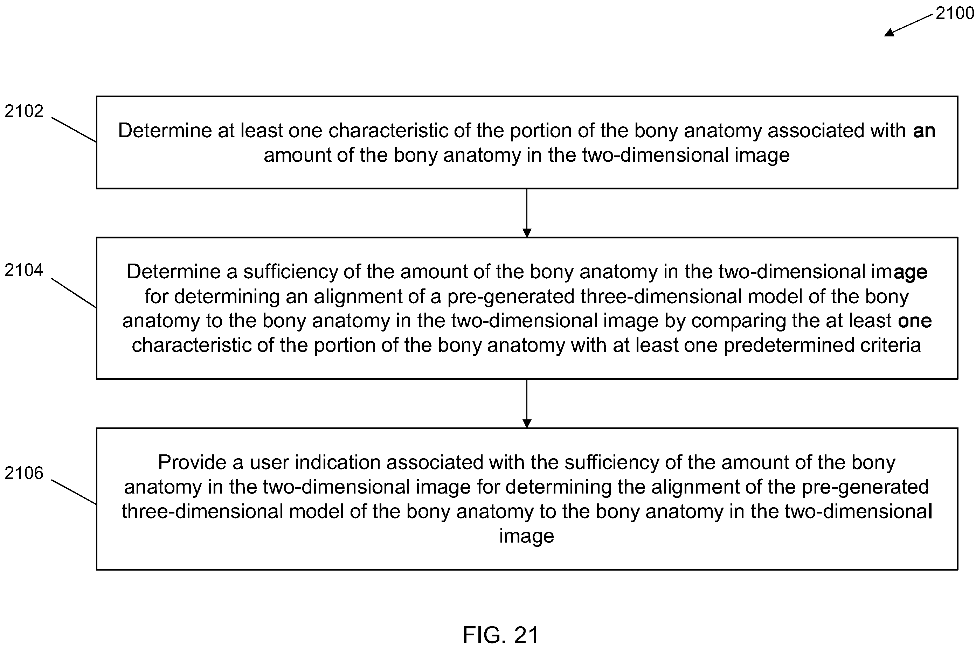

FIG. 21 illustrates an exemplary method 2100 for determining the sufficiency of the bony anatomy in a two-dimensional image, which may be used for step 1904 of method 1900. If method 2100 determines that there is sufficient bony anatomy in the two-dimensional image, then method 1900 may proceed to step 1906. If method 2100 determines that there is not sufficient bony anatomy in the two-dimensional image, then method 1900 may stop prematurely and/or restart from step 1902 using a different two-dimensional image of the bony anatomy. Method 2100 may be performed by one or more processors, such as the one or more processors of visual guidance system 125 of FIG. 18.

At step 2102, at least one characteristic of the portion of the bony anatomy associated with an amount of the bony anatomy in the two-dimensional image is determined. The characteristic may include a presence or absence of one or more specific anatomical landmarks in the two-dimensional image. As such, determining the characteristic(s) may include determining whether at least one anatomical landmark is present in the two-dimensional image. An anatomical landmark may be any biologically meaningful feature that can be detected by an intra-operative imaging system and can help guide a surgeon. For example, anatomical landmarks associated with a hip surgery (e.g., surgery to treat FAI) may include a femoral head, a femoral neck, a greater trochanter, a lesser trochanter, an intertrochanteric crest, a linea aspera, a condyle, and/or an epicondyle. The two-dimensional image may be analyzed in order to identify at least one anatomical landmark, for example by one or more machine learning models configured to identify anatomical landmarks.

Additionally, or alternatively, determining the at least one characteristic of the portion of the bony anatomy associated with an amount of the bony anatomy in the two-dimensional image may include determining a ratio of at least a predetermined region of the bony anatomy that is in the two-dimensional image relative to one or more other regions of the bony anatomy in the two-dimensional image. For example, in the example of a femur described above, the predetermined region may correspond to the femoral neck, the greater trochanter, and/or the lesser trochanter, and determining the at least one characteristic may include determining the ratio of the femoral neck, the greater trochanter, and/or the lesser trochanter to the femoral head. The ratio may be determined by summing the number of pixels in the image corresponding to the predetermined region(s) to the number of pixels in the image corresponding to regions other than the predetermined region(s). For example, if the two-dimensional image shows the femoral head, the femoral neck, and the greater and lesser trochanters, and the predetermined region is the region corresponding to the greater and lesser trochanters, the number of pixels corresponding to the greater and lesser trochanters may be compared to the number of pixels corresponding to the femoral head and neck to determine the ratio of the bony anatomy corresponding to the greater and lesser trochanters.

The number of pixels corresponding to a given region may be determined using at least one or more machine learning models. One or more machine learning models may be used to determine features of bony anatomy shown in a two-dimensional image and/or segment the features of the bony anatomy. For example, one or more processors, such as the one or more processors of visual guidance system 125, may be configured to implement one or more machine learning models to determine the number of pixels corresponding to a given region. Features of bony anatomy determined using one or more machine learning models can include regions of the bony anatomy (e.g., landmarks or groups of landmarks) and/or parameters of regions of the bony anatomy. For example, regions of a femur that may be identified by one or more machine learning models may include the femoral head, the femoral neck, and the greater and lesser trochanters, and parameters of such regions that may be the location of the center of the femoral head and/or the radius of the femoral head.

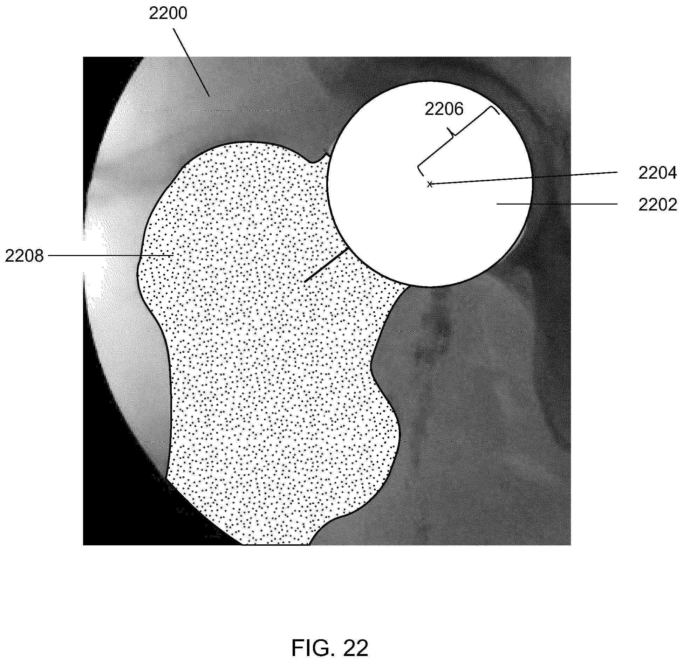

An exemplary result of the use of one or more machine learning models to determine features of a femur in a two-dimensional image is shown in FIG. 22. The machine learning model(s) may be used to identify the bony anatomy 2200 and/or one or more particular regions of the bony anatomy 2200, such as the femoral head 2202 and/or non-femoral head anatomy 2208 (e.g., the femoral neck and the greater and lesser trochanters). Optionally, one or more machine learning models are used to determine one or more parameters of one or more regions of bony anatomy that are then used to identify particular regions of the bony anatomy. For example, a machine learning model may be used to determine the location of the femoral head center 2204 and/or the femoral head radius 2206. These parameters may be used to segment the femoral head 2202, such as by segmenting a circle centered at femoral head center 2204 and having the femoral head radius 2206. The rest of the bony anatomy 2200 may then be identified as the non-femoral head anatomy 2208.

Once the machine learning model(s) have been used to identify which portions of the two-dimensional image correspond to femoral head 2202 and non-femoral head anatomy 2208, the number of pixels corresponding to each may be determined, such as by determining the number of pixels that the machine learning models have classified as associated with the femoral head 2202 or the non-femoral head anatomy 2208. Once the number of pixels corresponding to each region is determined, the ratio of the non-femoral head bony anatomy to the femoral head bony anatomy in the image may be calculated.

Returning to FIG. 21, step 2104 of method 2100 includes determining a sufficiency of the amount of the bony anatomy in the two-dimensional image for determining an alignment of a pre-generated three-dimensional model of the bony anatomy to the bony anatomy in the two-dimensional image. As described above with reference to step 1902 of FIG. 19, the three-dimensional model to be aligned with the two-dimensional image may be generated based on a three-dimensional scan of the region targeted for surgical treatment. The three-dimensional model may be generated pre-operatively and stored for use during a surgical session. Optionally, the pre-operatively generated three-dimensional model may be updated during surgery based on images captured during the surgical procedure in order to reflect changes to the anatomy during surgery (e.g., the removal of bone).

Sufficiency of the amount of bony anatomy in the two-dimensional image may be determined by comparing the characteristic(s) of the portion of the bony anatomy identified in step 2102 with at least one predetermined criteria. The predetermined criteria may include a presence or absence of one or more anatomical landmarks in the two-dimensional image. For example, in the context of hip surgery, the predetermined criteria may include the presence of a femoral head, a femoral neck, a greater trochanter, a lesser trochanter, an intertrochanteric crest, a linea aspera, a condyle, and/or an epicondyle. The predetermined criteria may include a list of one or more anatomical landmarks and comparing the characteristic(s) of the portion of the bony anatomy with at least one predetermined criteria may include determining whether one or more anatomical landmarks from the list are present in the two-dimensional image. For example, if only a femoral head and neck are identified in a two-dimensional image and the predetermined criteria includes a list of the femoral head and neck and the greater and lesser trochanters, then a determination may be made that the amount of bony anatomy in the two-dimensional image is insufficient.

Alternatively, or additionally, the predetermined criteria may be a ratio of a particular region of bony anatomy relative to other bony anatomy in the image. For instance, in examples involving debridement of the femoral head, the predetermined criteria may require that a certain ratio of the bony anatomy in the image corresponds to bony anatomy other than the femoral head and neck. For instance, using the hip surgery example described above, the predetermined criteria may require that the ratio of the amount of bony anatomy not corresponding to the femoral head and neck to the amount of bony anatomy corresponding to the femoral head and neck is about 5:1, about 4:1, about 3:1, about 2.5:1, about 2:1, about 1.8:1, about 1.5:1, or about 1:1. Preferably, the ratio may be about 1.5:1 to about 1.8:1.

At step 2106, a user indication associated with the sufficiency of the amount of the bony anatomy in the two-dimensional image for determining the alignment of the pre-generated three-dimensional model of the bony anatomy to the bony anatomy in the two-dimensional image is provided. The user indication may be provided to a user (e.g., a surgeon or other medical personnel), for example, via visual guidance system 125 of FIG. 18. The user indication may include, for example, a dialog box, including text indicating whether the amount of bony anatomy in the two-dimensional image is sufficient to determine alignment with the three-dimensional model or an assessment of reliability of the alignment of the pre-generated three-dimensional model of the bony anatomy to the bony anatomy in the two-dimensional image. If a determination is made at step 2104 that the amount of bony anatomy is not sufficient, a user indication may include a message indicating to the surgeon that a different two-dimensional image is required. Optionally, the user indication may only be provided if the amount of the bony anatomy in the two-dimensional image is determined to be insufficient for determining the alignment of the pre-generated three-dimensional model of the bony anatomy to the bony anatomy in the two-dimensional image. The user indication may indicate an assessment of reliability of an alignment of a three-dimensional model with the two-dimensional image for a displayed overlay. The assessment of the reliability may be qualitative (e.g., high, medium, low confidence) or may be quantitative (e.g., 90% confidence, 80% confidence, etc.).

Returning to FIG. 19, once the sufficiency of the amount of bony anatomy in the two-dimensional image is determined at step 1904 (e.g., using method 2100), an alignment of the three-dimensional model to the two-dimensional image may be determined at step 1906. Method 1900 may proceed to step 1906 only if the amount of bony anatomy in the two-dimensional image was determined to be sufficient in step 1904 (e.g., if the amount of bony anatomy in the two-dimensional image met a predetermined criterion). If the amount of bony anatomy was determined to be insufficient, a different two-dimensional image may be obtained and method 1900 may return to step 1902 for the different two-dimensional image. Alternatively, if the amount of bony anatomy was determined to be insufficient, method 1900 may continue to step 1906, with the user indication provided in step 1904 serving to notify the user that the alignment and overlay may not be accurately aligned (or providing another type of qualitative assessment or a quantitative assessment of the reliability of the alignment).

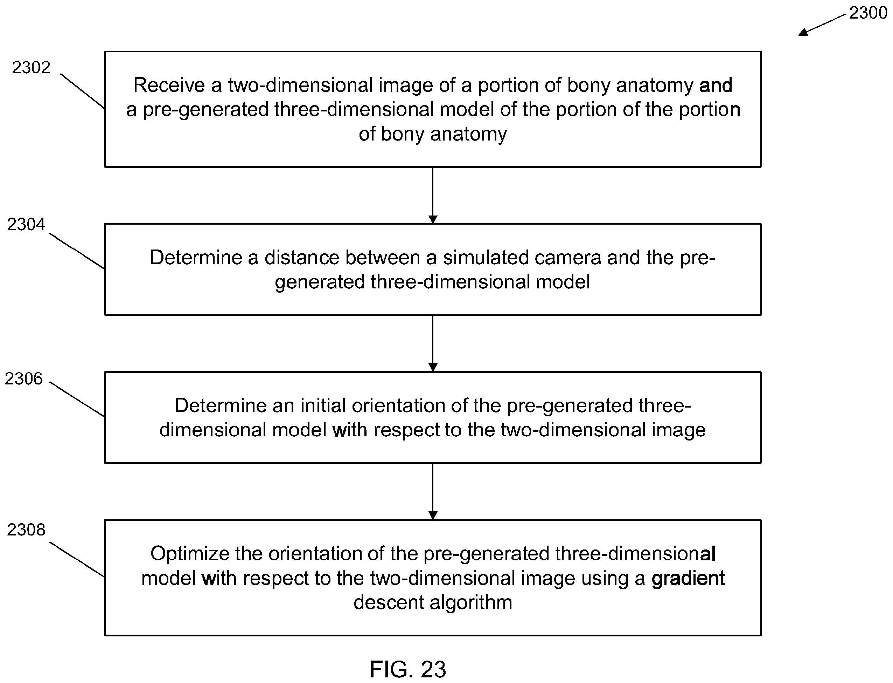

Determining the alignment of the three-dimensional model to the two-dimensional image generally includes manipulating the three-dimensional model according to the available spatial degrees of freedom until a projection of that model onto a two-dimensional plane aligns with the bone in the two-dimensional image. FIG. 23 illustrates an exemplary method 2300 for determining the alignment of a three-dimensional model to a two-dimensional image, according to step 1906. Method 2300 can be performed by one or more processors, such as the one or more processors of visual guidance system 125 of FIG. 18.

Step 2302 includes receiving a two-dimensional image of a portion of bony anatomy and a pre-generated three-dimensional model of the portion of bony anatomy. The two-dimensional image may be received from an intra-operative imaging system, such as an X-ray image received from an X-ray imager that is communicatively connected with the surgical guidance system performing method 2300 (e.g., C-arm X-ray machine 115 of FIG. 18). The two-dimensional image may be a two-dimensional image for which the sufficiency of the bony anatomy in the image for alignment was previously determined (for example, via method 2100 described above with reference to FIG. 21). The pre-generated three-dimensional model may be generated pre-operatively based on a three-dimensional scan of the portion of bony anatomy and stored for use during a surgical session.

At step 2304, a distance between a simulated camera and the pre-generated three-dimensional model is determined. Determining the distance between the simulated camera and the pre-generated three-dimensional model allows the visual guidance system (e.g., visual guidance system 125 of FIG. 18) to determine a position of the pre-generated three-dimensional model between the simulated camera and the plane of the two-dimensional image, such that a projection of the pre-generated three-dimensional model onto a two-dimensional plane aligns with the two-dimensional image. The distance between the simulated camera and the pre-generated three-dimensional model may be computed based on features of the bony anatomy in the two-dimensional image and the pre-generated three-dimensional model. For example, in examples involving debridement of a femoral head, the distance between the simulated camera and the pre-generated three-dimensional model may be based on the radius of the femoral head in the two-dimensional image and the radius of the femoral head in the pre-generated three-dimensional model. The simulated camera distance may be determined by computing the distance between the pre-generated three-dimensional model and the simulated camera that causes the radius of the three-dimensional femoral head shown in the pre-generated three-dimensional model to become the radius of the two-dimensional femoral head shown in the two-dimensional image when projected into a two-dimensional plane.

Once the position of the pre-generated three-dimensional model with respect to the simulated camera is determined, an initial orientation of the three-dimensional model with respect to the two-dimensional model may be determined at step 2306. The initial orientation may be determined by performing a grid search. The grid search may identify a plurality of rotated positions of the three-dimensional model and compute a similarity metric between the two-dimensional image and a two-dimensional projection of the three-dimensional model for each position. The grid search may compute a similarity metric for multiple rotations of the three-dimensional model between the rotated positions (-90, -90, - 90) and (90, 90, 90) by changing one variable at a time, e.g. in predetermined increments, such as in 20-degree increments. For example, the grid search may check the rotated positions (-90, -90, -90), (-90, -90, -70), (-90, -90, -50), and so on until reaching (90, 90, 90). The rotated position for which the similarity metric indicates the highest degree of similarity between the projection of the three-dimensional model and the two-dimensional image may be selected as the initial orientation of the 3D model.

The similarity metric computed to evaluate the alignment of the two-dimensional image and the three-dimensional model in each rotated position may be a cost function such as the intersection over union (IoU). IoU is a measure of overlap. To calculate IoU, the number of pixels that are shared between a pixel mask corresponding to the two-dimensional image and a pixel mask corresponding to the projection of the three-dimensional model onto the two-dimensional plane of the image is calculated. The total number of unique pixels contained in both masks combined is also calculated. IoU is then computed by dividing the number of shared pixels (the intersection) by the total number of unique pixels across both masks (the union). The possible values of IoU range from 0 (indicating there is no overlap between the pixel masks) to 1 (indicating a perfect overlap between the pixel masks).

Cost functions other than IoU may be used to evaluate alignment, such as reprojection error, iterative closest point (ICP) error, mutual information (MI), sum of squared distances (SSD), or normalized cross correlation (NCC). Optionally, the cost function used may have smooth gradients (e.g., IoU, MI, SSD, NCC) such that local minima do not interfere with locating the absolute minimum of the cost function (which may indicate the optimal orientation of the pre-generated three-dimensional model with respect to the two-dimensional image).

At step 2308, the orientation of the pre-generated three-dimensional model with respect to the two-dimensional image is optimized using a gradient descent algorithm. Optionally, the gradient descent algorithm may be the Adam algorithm. The gradient descent algorithm may compute the approximate gradient of the cost function with respect to each variable. In the context of orientation, the variables include the six degrees of freedom: rotation in the x, y, and z directions and translation in the x, y, and z directions. The gradient descent algorithm may use the computed gradients to optimize each variable. The gradient descent algorithm may optimize each variable by using the finite difference method, partial differentiation, or any other suitable technique.

Using translation in the x-direction as an example, if the initial orientation of the three-dimensional model has an x-axis translation value of 10 mm, and the IoU for that value is 90, the gradient descent algorithm may compute the IoU for an x-axis translation value of 9 mm and an x-axis translation value of 11 mm. If the IoU for 9 mm is less than 90 and the IoU for 11 mm is greater than 90, the gradient descent algorithm may recognize that increasing the x-axis translation value above 10 mm improves IoU. The gradient descent algorithm may subsequently use an x-axis translation value greater than 10 mm in the next iteration. The subsequent x-axis translation value may be based on the computed gradient with respect to x-axis translation and the learning rate of the gradient descent algorithm. The learning rate may decay over time.

The gradient descent algorithm may continue to iteratively guess values for each variable using the techniques described above until the cost function for each variable is minimized or satisfies a predetermined criterion. For example, if IoU is used as the cost function, the gradient descent algorithm may continue to iteratively guess values for each variable until the IoU is equal to 1 (indicating a perfect alignment) or until the IoU exceeds a predetermined threshold (e.g., about 0.95). Alternatively, the gradient descent algorithm may continue to iterate for a predetermined amount of time or for a predetermined number of iterations. The end result of the iterative process is a determined three-dimensional model alignment that, when projected into the two-dimensional plane, sufficiently aligns with the two-dimensional image.

Returning to FIG. 19, once the alignment of the three-dimensional model to the two-dimensional image is determined in step 1906, the three-dimensional model may be overlaid on the two-dimensional image based on the determined alignment at step 1908. A projection of the model according to the alignment determined in step 1906 is generated and at least a portion of the projection is overlaid on the two-dimensional image, which is then displayed to guide a surgeon in step 1910. As noted above, the three-dimensional model may include a representation of the portion of the bone that deviates from a target bone state. This representation can serve as a plan for the surgical procedure that indicates to the surgeon where and how much bone should be removed to achieve the target state of the bone. A representation of planned bone removal may include a heat map that is color-coded according to the planned depth of bone removal, an outline of the planned bone removal area, a contour map indicating amounts of bone removal, or any other suitable visual aid. In step 1908, at least a portion of the representation of planned bone removal may be included in the overlay to provide the surgeon with information regarding planned bone removal. The model may include portions of bone that are outside of the planned bone removal and the overlay may include at least some of these portions of bone, or the overlay may include only the representation of planned bone removal. Optionally, the overlay may include additional information, such as an indication of the reliability of the alignment of the three-dimensional model to the two-dimensional image determined in step 1906.

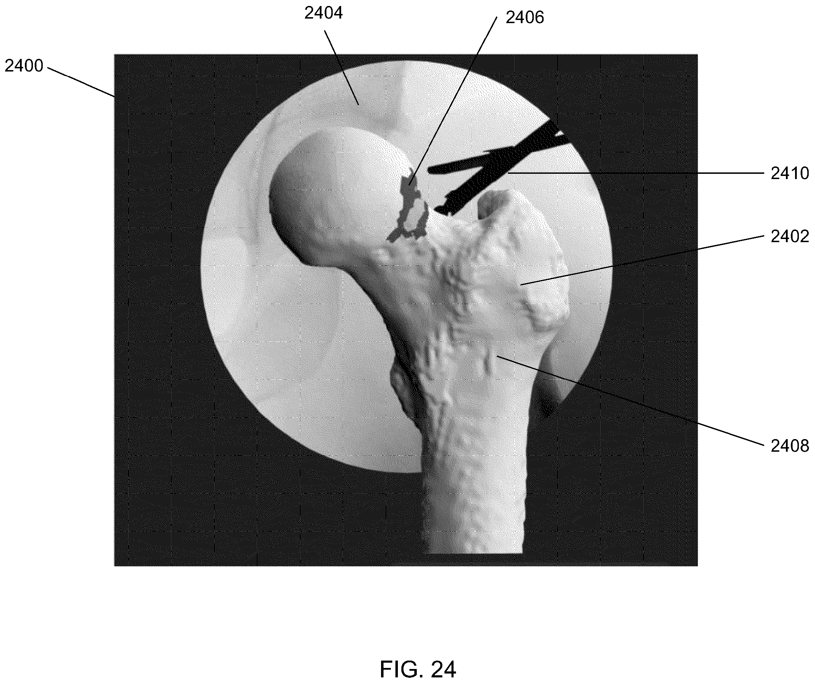

FIG. 24 illustrates a visualization 2400 (e.g., displayed in a graphical user interface by a visual guidance system) that includes an exemplary overlay of a rendering 2402 of a projection of a three-dimensional model of the upper portion of the femur on a two-dimensional image 2404 of the hip joint. The rendering 2402 includes a representation 2406 of planned bone removal - in the form of a heat map - and portions 2408 of bone outside of the planned bone removal. The heat map illustrated in FIG. 24 is merely exemplary of a representation of planned bone removal. As noted above, any suitable representation may be used, including, for example, an outline of the planned bone removal area, a contour map indicating amounts of bone removal, or any combinations of such visual aids.

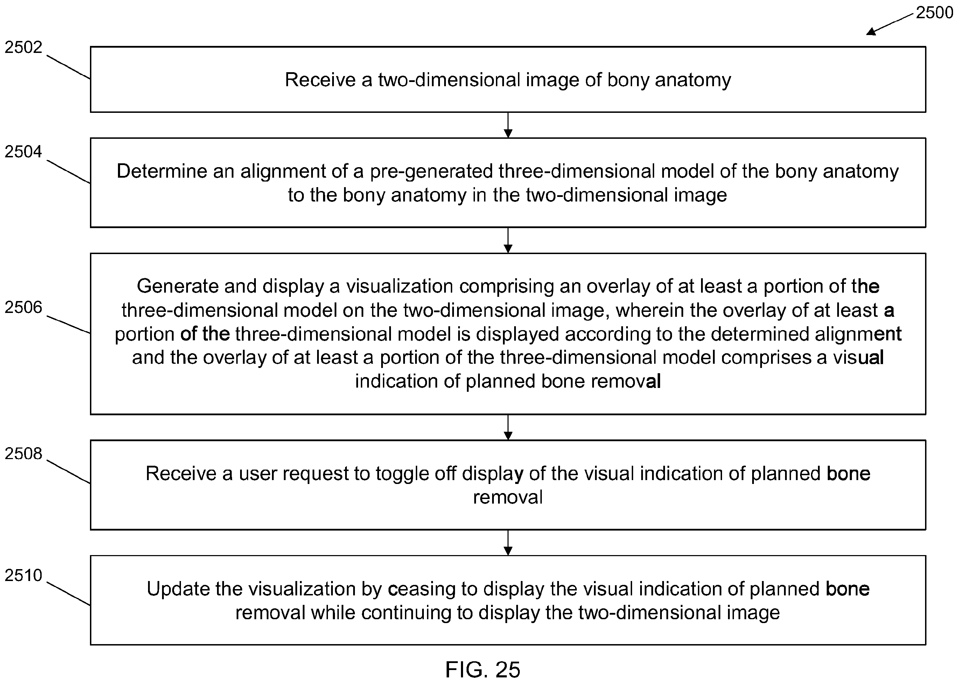

Optionally, the representation 2406 of planned bone removal may be toggled on and off based on a request from a user (e.g., a surgeon). FIG. 25 illustrates an exemplary method 2500 for toggling a visual indication of planned bone removal. Method 2500 may be performed by a visual guidance system, such as visual guidance system 125 of FIG. 18.

At step 2502, a two-dimensional image of bony anatomy is received. The two-dimensional image may be received from an intra-operative imaging system, such as an X-ray image received from an X-ray imager that is communicatively connected with the surgical guidance system performing method 2500 (e.g., C-arm X-ray machine 115 of FIG. 18). The two-dimensional image may be a two-dimensional image for which the sufficiency of the bony anatomy in the image for alignment was previously determined (for example, via method 2100 described above with reference to FIG. 21).

At step 2504, an alignment of a pre-generated three-dimensional model of the bony anatomy to the bony anatomy in the two-dimensional image is determined. The pre-generated three-dimensional model may be produced pre-operatively based on a three-dimensional scan of the portion of the bony anatomy to receive surgical treatment and stored for use during a surgical session. The alignment of the pre-generated three-dimensional model to the two-dimensional image may be determined according to method 2300 described above with reference to FIG. 23.

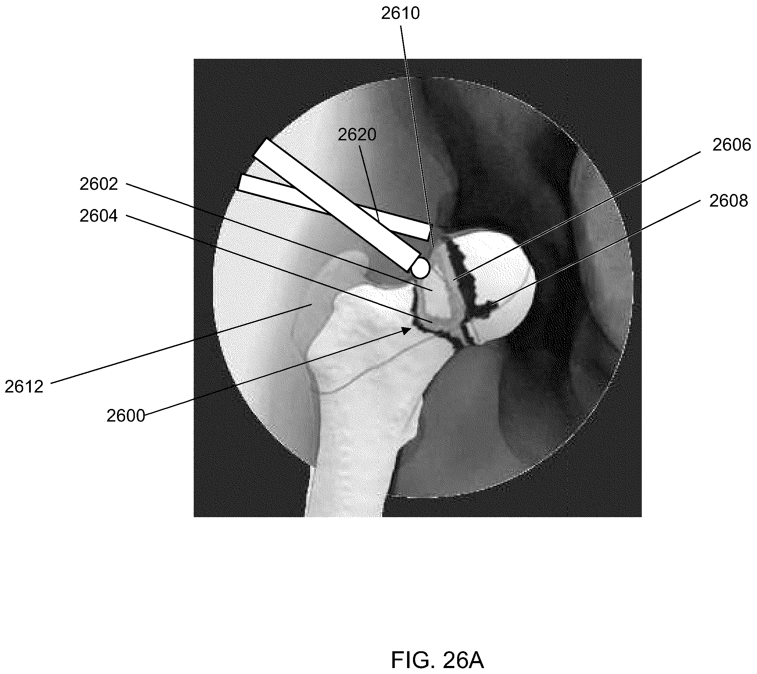

At step 2506, a visualization comprising an overlay of at least a portion of the three-dimensional model on the two-dimensional image is generated and displayed. The overlay of at least a portion of the three-dimensional model is displayed according to the determined alignment. The overlay of at least a portion of the three-dimensional model includes a visual indication of planned bone removal. The visual indication of planned bone removal may include, but is not limited to, an outline of the planned bone removal area, a contour map indicating amounts of bone removal, or a heat map. The visual indication of planned bone removal may indicate the planned depth of bone removal. For example, a heat map may indicate different depths of bone removal using color-coding or varying degrees of shading. FIG. 26A illustrates an exemplary heat map 2600 that includes a first color portion 2602 indicating where bone removal should be the deepest, a second color portion 2604 indicating where bone removal should be the second deepest, a third color portion 2606 indicating where bone removal should be the third deepest, and a fourth color portion 2608 indicating where bone removal should be the shallowest. In some examples, the transition between color portions in heat map 2600 may be indicated by discrete borders (e.g., as shown in FIG. 26A). In other examples, the transition between color portions in heat map 2600 may be smooth, indicating that the depth of the bone removed should change gradually in accordance with the color transitions.

Optionally, a visual indication of planned bone removal, such as the heat map shown in FIG. 26A, may indicate which portions of the heat map correspond to anatomy shown in the two-dimensional image and which portions do not. Portions of the heat map that do not correspond to anatomy shown in the two-dimensional image may be represented differently from portions of the heat map that do correspond to anatomy shown in the two-dimensional image, e.g. in a different color (palette), hatching, different shading, or muted in color. For example, portion 2610 of heat map 2600 is muted in color (e.g., darker or less vibrant in color) compared to the rest of the heat map to indicate that portion 2610 corresponds to a portion of the anatomy not located within the plane of the two-dimensional image (e.g., a portion of the anatomy that is behind the imaging plane corresponding to the two-dimensional image). Optionally, all portions of the three-dimensional model that are not in the plane of the two-dimensional image may be muted in color rather than only the heat map. Muted portions of the three-dimensional model may be distinguished from the two-dimensional image using different color schemes for the three-dimensional model and two-dimensional image. In some examples, the two-dimensional image may be presented in grayscale, while the three-dimensional model may be colorized. For example, in FIG. 26A, the two-dimensional model is shown in grayscale, while the three-dimensional image is an off-white color (with a colorized heat map). The portions of the three-dimensional model that are outside of the plane of the two-dimensional image are still colorized (e.g., portion 2612 of the three-dimensional model and portion 2610 of the heat map), but the colors are muted compared to the portions of the three-dimensional model in the imaging plane.

At step 2508, a user request to toggle off display of the visual indication of planned bone removal is received. The user request may be provided by a surgeon or other user via a user interface, such as a user interface of visual guidance system 125 of FIG. 18. The user request may be communicated by toggling a switch, checking a checkbox, or selecting an option button.

At step 2510, the visualization is updated by ceasing to display the visual indication of planned bone removal while continuing to display the two-dimensional image. The overlay may include a representation of at least a portion of the bony anatomy, and updating the visualization may cause the visualization to cease displaying the representation of the bony anatomy altogether. Alternatively, updating the visualization may cause the visualization to toggle off the visual indication of planned bone removal but continue to display the representation of the bony anatomy.

An overlay may include additional features and/or information. For instance, with reference to FIG. 26A, an overlay may optionally include a representation of a surgical tool 2620 or a portion thereof that is overlaid on a portion of the overlay corresponding to the three-dimensional model (i.e., on top of the three-dimensional model). Overlaying a representation of a surgical tool on top of the overlay can be advantageous because if a two-dimensional image includes a surgical tool that is at least partially shown on top of bone, the surgical tool may be blocked when the three-dimensional model is overlaid on the portion of the two-dimensional image corresponding to the bone. For example, in FIG. 24, surgical tool 2410 is blocked by the overlay of the three-dimensional model rendering 2402. As a result, a surgeon may not be able to see where the surgical tool is with respect to the planned bone removal, which may make it difficult for the surgeon to orient the tool in the surgical cavity for removal of bone according to the planned bone removal. By overlaying a representation of the surgical tool on a portion of the three-dimensional model overlay, the surgeon may be better guided in the bone removal.

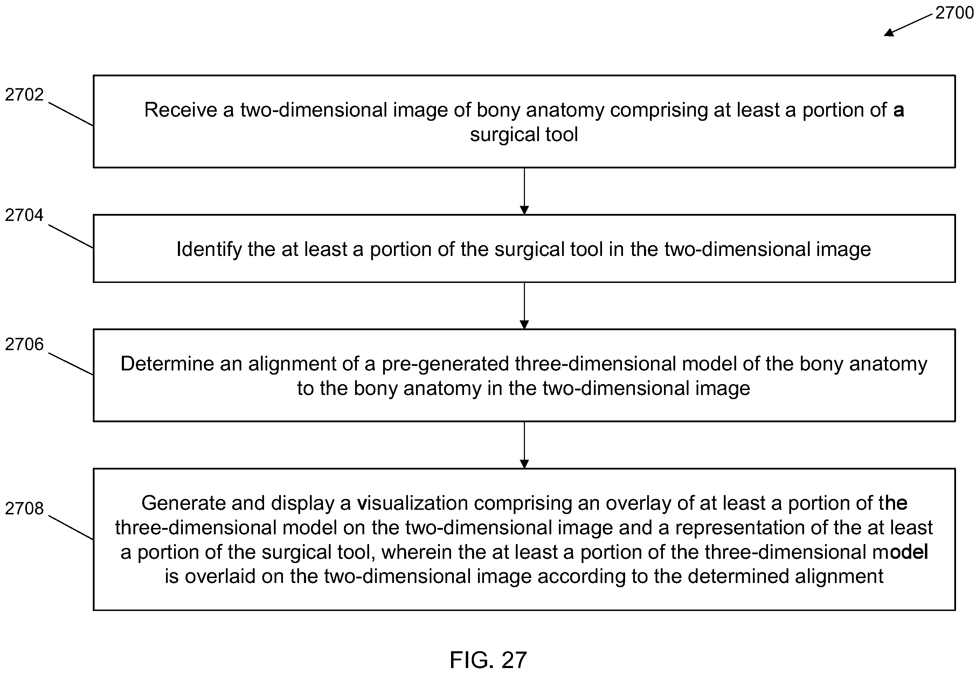

An exemplary method for generating and displaying an overlay visualization, including a representation of a surgical tool, is illustrated in FIG. 27. Method 2700 may be performed by one or more processors, such as the one or more processors of visual guidance system 125 of FIG. 18. At step 2702, a two-dimensional image of bony anatomy comprising at least a portion of a surgical tool is received. The two-dimensional image may be received from an intra-operative imaging system, such as an X-ray image received from an X-ray imager that is communicatively connected with the surgical guidance system performing method 2700 (e.g., C-arm X-ray machine 115 of FIG. 18). The two-dimensional image may be a two-dimensional image for which the sufficiency of the bony anatomy in the image for alignment was previously determined (for example, via method 2100 described above with reference to FIG. 21). The surgical tool or portion thereof pictured in the two-dimensional image may be a bone removal tool (e.g., a bur, a chisel, a file, forceps, etc.) or any other orthopedic surgical tool.

At step 2704, at least a portion of the surgical tool may be identified in the two-dimensional image. The surgical tool may be identified using one or more machine learning models. For example, one or more processors, such as the one or more processors of visual guidance system 125, may be configured to implement one or more semantic segmentation algorithms configured to segment a surgical tool from the rest of an image. A representation of the identified surgical tool or portion thereof may then be created based on the identified surgical tool or portion thereof. The representation may include a segmented portion of the two-dimensional image corresponding to the surgical tool or portion thereof or a pixel mask corresponding to the surgical tool or portion thereof. Alternatively, the representation may include a silhouette or outline corresponding to the surgical tool or portion thereof.

Optionally, identifying at least a portion of the surgical tool may include identifying the type of surgical tool in the two-dimensional image. The type of surgical tool may be identified using one or more machine learning models configured to identify surgical tool types. For example, one or more processors, such as the one or more processors of visual guidance system 125, may be configured to implement one or more machine learning models to identify the type of surgical tool(s) in an image. Alternatively, the type of surgical tool type may be identified based on a unique fiducial marker associated with type tool (e.g., different types or sizes of burs may have different fiducial markers). Alternatively, the type of surgical tool may be identified based on a surgical tool selection made by a surgeon on a user interface (e.g., visual guidance system 125 of FIG. 18) prior to surgery.

At step 2706, an alignment of a pre-generated three-dimensional model of the bony anatomy to the bony anatomy in the two-dimensional image is determined. The pre-generated three-dimensional model may be produced pre-operatively based on a three-dimensional scan of the portion of bony anatomy to be surgically treated and stored for use during a surgical session. The alignment of the pre-generated three-dimensional model to the two-dimensional image may be determined according to method 2300 described above with reference to FIG. 23.

At step 2708, a visualization comprising an overlay of at least a portion of the three-dimensional model on the two-dimensional image and a representation of at least a portion of the surgical tool is generated and displayed. An exemplary visualization is illustrated in FIG. 26A. The representation of the surgical tool or portion thereof may be overlaid on the three-dimensional model. For example, in FIG. 26A, the representation of surgical tool 2620 is a pixel mask overlaid on the heat map 2600 portion of the three-dimensional model. Alternatively, the representation of the surgical tool or portion thereof may be overlaid on the two-dimensional image, behind the three-dimensional model, as shown in FIG. 24.

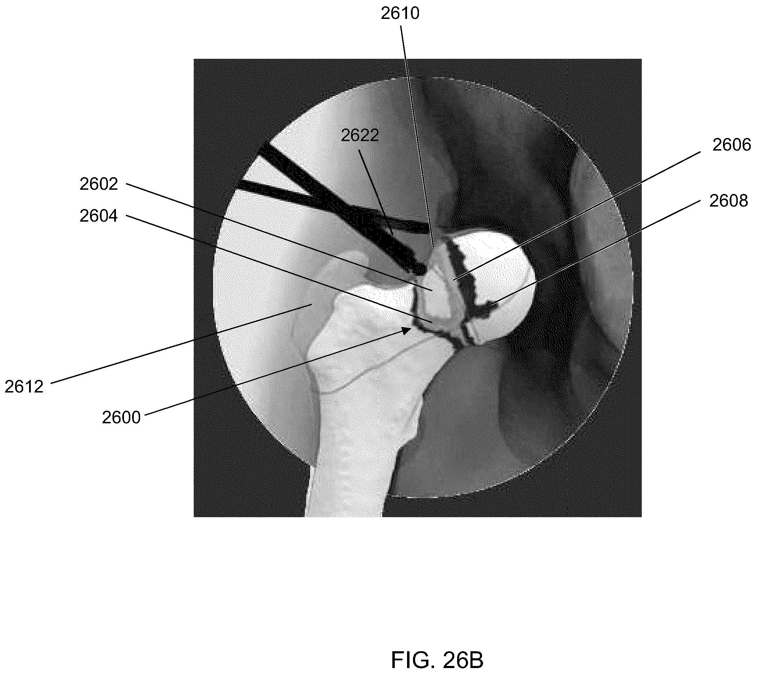

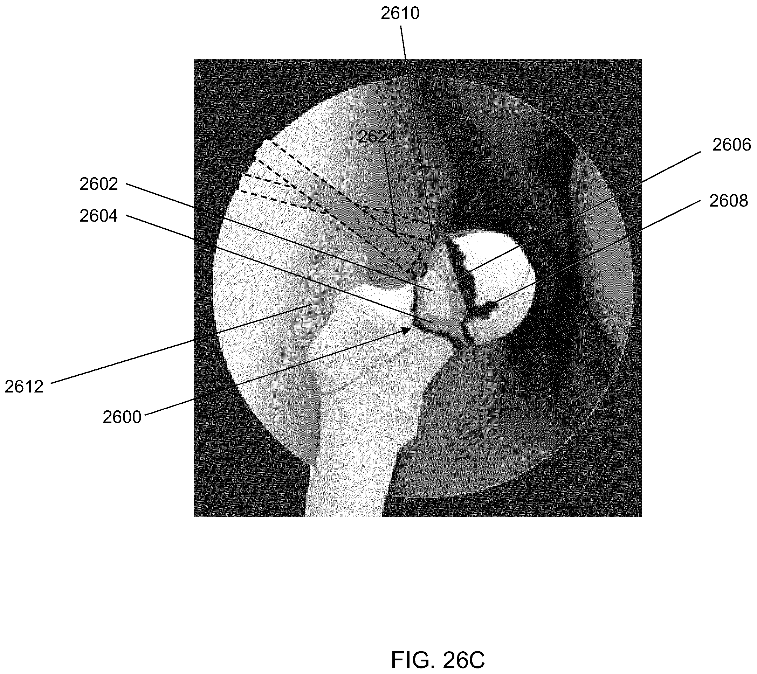

The representation of the surgical tool or portion thereof may be a two-dimensional representation. For example, the representation of the surgical tool or portion thereof may include the segmented portion of the two-dimensional image or pixel mask corresponding to the surgical tool or portion thereof generated in step 2704. FIG. 26A shows an exemplary visualization in which the representation of a surgical tool 2620 is a pixel mask. Alternatively, the representation of the surgical tool or portion thereof may include a silhouette, such as silhouette 2622 shown in FIG. 26B. Alternatively, the representation of the surgical tool or portion thereof may include an outline, such as outline 2624 shown in FIG. 26C. Optionally, the representation of the surgical tool is a graphical object (e.g., a circle, an "x", a bullseye, etc.) representing the location of the tip of the surgical tool. The segmented portion, mask, silhouette, outline, or other representation of the surgical tool or portion thereof may be overlaid on the three-dimensional model, such that the position of the surgical tool is visible in the overlay image. Alternatively, the representation of the surgical tool or portion thereof may be formed as a cut-out in the overlay of the at least a portion of the three-dimensional model on the two-dimensional image. Alternatively, generic two-dimensional representations of various surgical tools may be stored in the system (e.g., visual guidance system 125 of FIG. 18), and a generic two-dimensional representation corresponding to the type of tool shown in the two-dimensional image may be selected to overlay on the three-dimensional model.

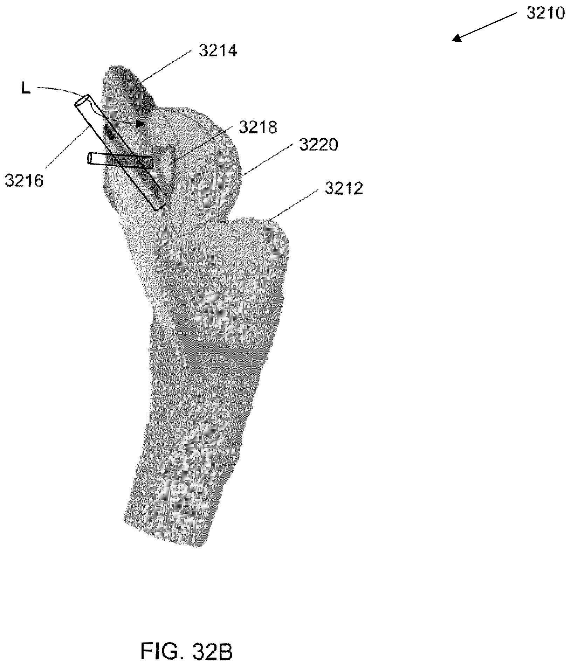

Alternatively, the representation of the surgical tool or portion thereof may be a three-dimensional representation. Optionally, the three-dimensional pose of the surgical tool in the two-dimensional image may be determined. The determination of the three-dimensional pose may be based on the type of surgical tool in the image identified in step 2704. Generic three-dimensional models of various surgical tools may be stored in the system (e.g., visual guidance system 125 of FIG. 18). A generic three-dimensional model corresponding to the type of surgical tool identified in step 2704 may be selected. The three-dimensional pose of the surgical tool may then be determined based on a projection of the generic three-dimensional model into two dimensions, such that the projection of the three-dimensional model lines up with the surgical tool or portion thereof shown in the two-dimensional image. The three-dimensional representation of the surgical tool may subsequently be displayed in the visualization according to the determined three-dimensional pose (e.g., see tool 3216 in FIG. 32B, which is discussed further below). In some aspects, the three-dimensional representation of the surgical tool may include an indication of which portions of the surgical tool are in the two-dimensional imaging plane. For example, the portions of the surgical tool in the two-dimensional imaging plane may be indicated using color (e.g., by making the portions of the surgical tool outside of the two-dimensional imaging plane muted in color compared to the portions of the surgical tool in the two-dimensional imaging plane), by using different shades of grayscale, and/or by outlining the portions of the surgical tool in the two-dimensional imaging plane.

Optionally, only a portion of the surgical tool may be displayed in an overlay visualization. For example, only the cutting head of a bone cutting tool may be displayed in the overlay visualization without the shaft supporting the cutting head. The head of the bone cutting tool may be displayed by segmenting the portion of the two-dimensional image corresponding to the head and overlaying it on the three-dimensional model, overlaying an outline of the head on the three-dimensional model, or overlaying a generic two-dimensional or three-dimensional representation of the head on the three-dimensional model. In some examples, a generic two-dimensional or three-dimensional representation of the head may be a circle or a sphere, rather than an actual representation of the shape of the tool head. Optionally, a user may choose which portion(s) of a surgical tool to display in an overlay visualization. For instance, the user may choose to display only the cutting head of a bone cutting tool or the cutting head and shaft of the bone cutting tool.

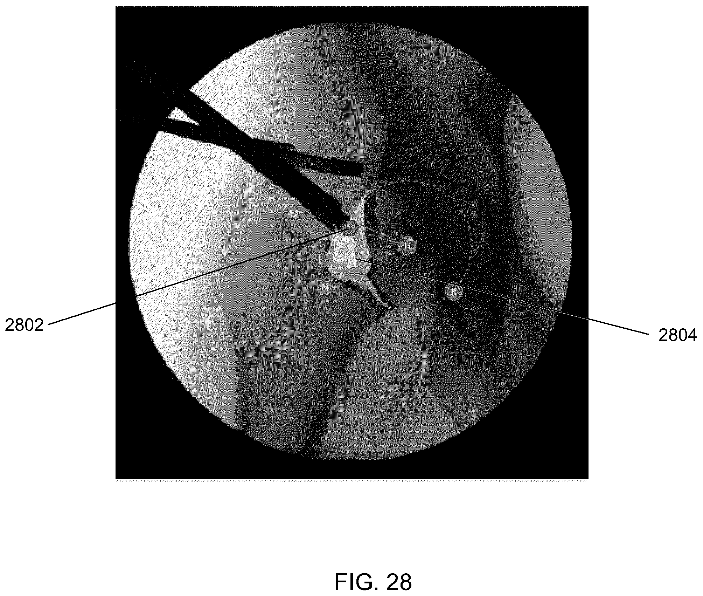

Optionally, the representation of the surgical tool or portion thereof may be displayed with an amount of transparency. For example, as shown in FIG. 28, the distal tip 2802 of the surgical tool may be partially transparent, such that the heat map 2804 of planned bone removal remains visible underneath the surgical tool. In some examples, the entire surgical tool may be partially transparent, rather than only the distal tip. In other examples, a predetermined portion of the surgical tool may be partially transparent, such as a set number of millimeters at the distal end of the surgical tool or the portion of the surgical tool inside the body of the patient. Displaying the representation of the surgical tool in the visualization may be optional and may be turned on and off, for example, by toggling a switch. Other aspects of a representation of a surgical tool may be selectively displayed (e.g., by toggling a switch), such as transparency versus no transparency or adjusting transparency of the surgical tool or the portion of the surgical tool that overlays the bony anatomy.

Returning to FIG. 24, the user interface (e.g., visual guidance system 125 of FIG. 18) may be configured to enable a user to change a transparency of the rendering 2402 of the projection of the three-dimensional model and/or the representation 2406 of planned bone removal. Increasing the transparency of rendering 2402 and/or representation 2406 may be advantageous because it may allow a surgeon to see more of the two-dimensional image 2404 behind the rendering 2402 and/or representation 2406.

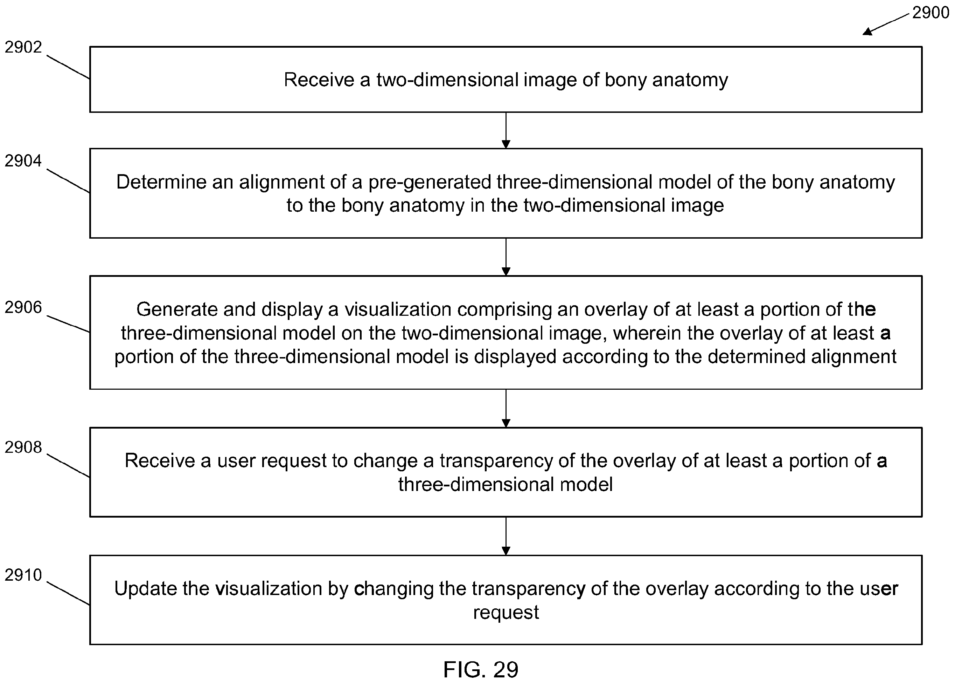

An exemplary method 2900 for changing the transparency of an overlay is illustrated in FIG. 29. Method 2900 may be performed by one or more processors of a visual guidance system, such as the one or more processors of visual guidance system 125 of FIG. 18. At step 2902, a two-dimensional image of bony anatomy is received. Step 2902 may be substantially the same as step 2502 of method 2500 and, thus, the details of step 2902 are omitted for brevity. At step 2904, an alignment of a pre-generated three-dimensional model of the bony anatomy to the bony anatomy in the two-dimensional image is determined. The alignment of the pre-generated three-dimensional model to the two-dimensional image may be determined according to method 2300 described above with reference to FIG. 23. At step 2906, a visualization comprising an overlay of at least a portion of the three-dimensional model on the two-dimensional image is generated and displayed. The overlay of at least a portion of the three-dimensional model is displayed according to the determined alignment. The three-dimensional model may include a representation of the bony anatomy. The three-dimensional model may also include a representation of planned bone removal (e.g., a heat map, an outline of the planned bone removal area, or a contour map indicating amounts of planned bone removal).

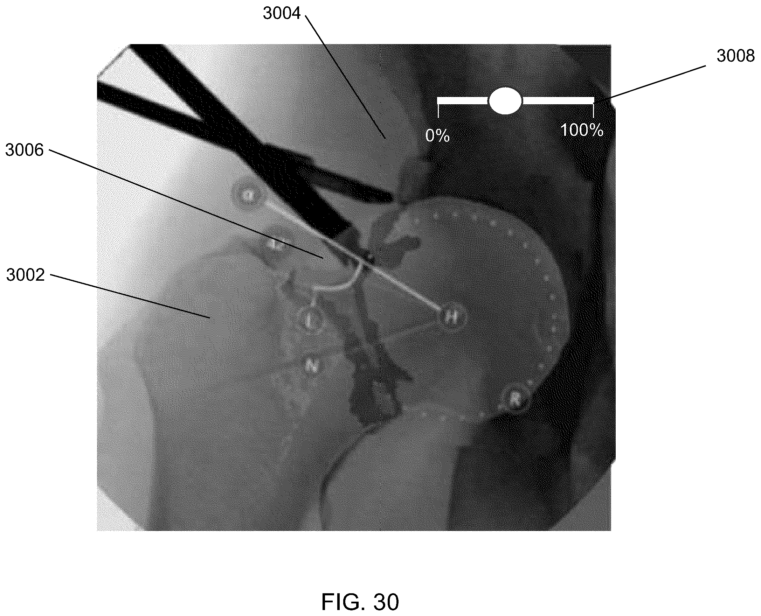

At step 2908, a user request to change the transparency of the overlay of at least a portion of the three-dimensional model is received. The user request may include a selection of a predetermined level of transparency. The user request may include interacting with a user interface control (e.g., moving a graphical slider) associated with the visualization to indicate a desired level of transparency. The visualization is then updated by changing the transparency of the overlay according to the user request in step 2910. FIG. 30 shows an exemplary semi-transparent three-dimensional model overlay. A portion of the three-dimensional model 3002 is overlaid on the two-dimensional image 3004. The overlay further includes a representation of planned bone removal in the form of a heat map 3006. Both the portion of the three-dimensional model 3002 and the heat map 3006 are provided in a semi-transparent state, such that the bony anatomy pictured in the two-dimensional image 3004 is visible underneath the portion of the three-dimensional model 3002 and the heat map 3006. The transparency of the three-dimensional model 3002 and the heat map 3006 may be adjusted using a user interface control, such as a graphical slider 3008. Graphical slider 3008 is only one example of a user interface control. Other suitable user interface controls may include radio buttons, toggle switches, text entry boxes, and the like.

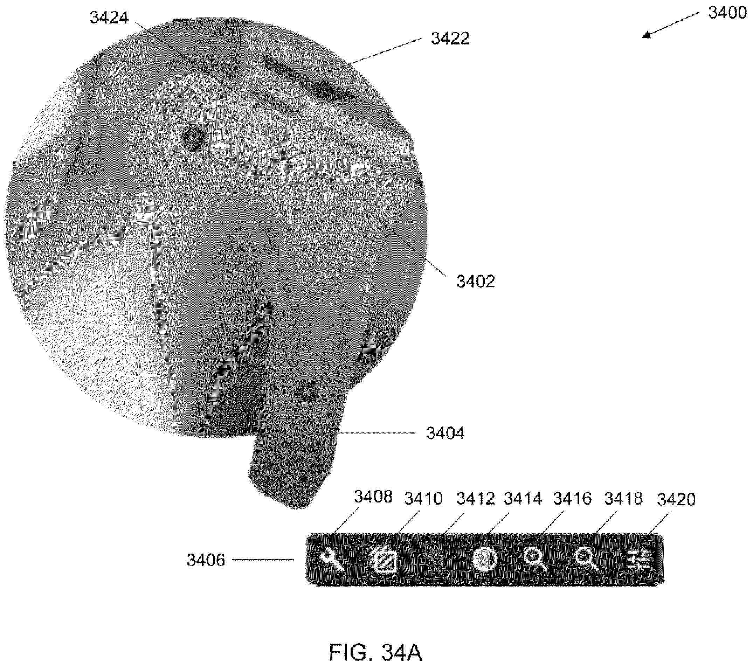

Distinguishing between a semi-transparent representation of the three-dimensional model and the two-dimensional image on which the three-dimensional model is overlaid may be challenging. Accordingly, the three-dimensional model may be identified by an outline. Additionally, or alternatively, the three-dimensional model may be shaded or colorized, while the two-dimensional image may be shown in black and white or grayscale in order to aid in distinguishing the model from the image. For instance, in FIG. 26A, the three-dimensional model is shown in a first shade or color (e.g., an off-white color), while the two-dimensional image is shown in a second shade or color (e.g., grayscale or a different shade of gray within a grayscale). Optionally, the bony anatomy in the two-dimensional image may be shaded or colorized. FIG. 34A illustrates an example of an overlay visualization 3400 of a semi-transparent three-dimensional model 3404 overlaid on a colorized two-dimensional image 3402. The bony anatomy in the two-dimensional image may be shown in a first color or shade (e.g., pink or light grayscale shade), such that the bony anatomy corresponding to the two-dimensional image is clearly visible through the semi-transparent three-dimensional model overlay. Optionally, the coloration or shading of the bony anatomy in the two-dimensional image may be toggled on and off according to the preferences of the user.

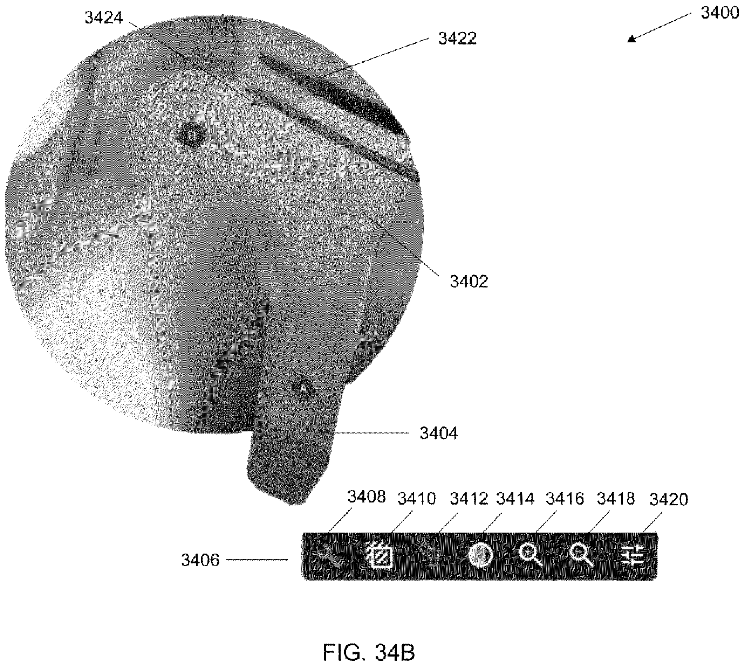

Various aspects of the overlay visualization 3400 may be adjusted by a user, such as by using user interface control panel 3406. For example, the display of surgical instrument 3422 may be changed using the surgical instrument user interface control 3408. For example, surgical instrument user interface control 3408 may comprise a toggle switch, radio buttons, or graphical slider allowing a user to select whether to display surgical instrument 3422 in front of three-dimensional model 3404 (as shown in FIG. 34B) or behind three-dimensional model 3404 (as shown in FIG. 34A). Optionally, the representation of the surgical instrument 3422 may be turned off entirely using surgical instrument user interface control 3408.

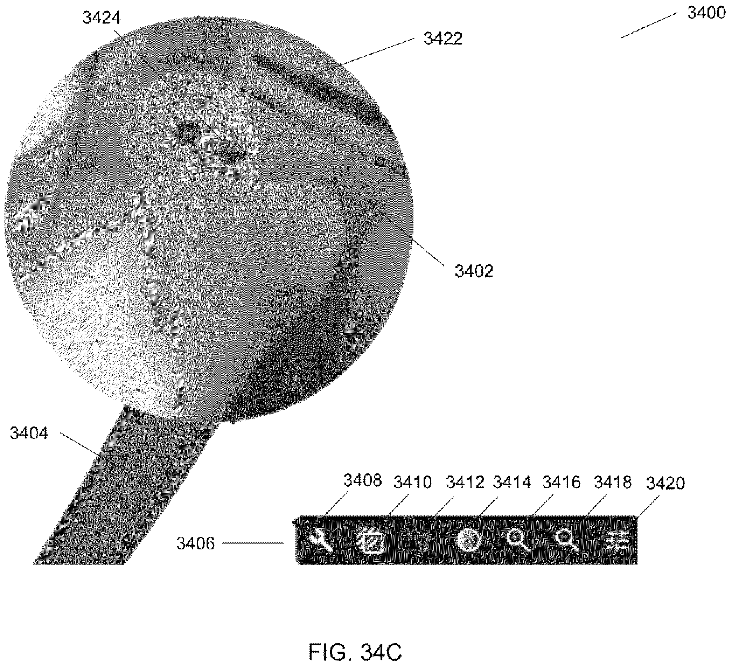

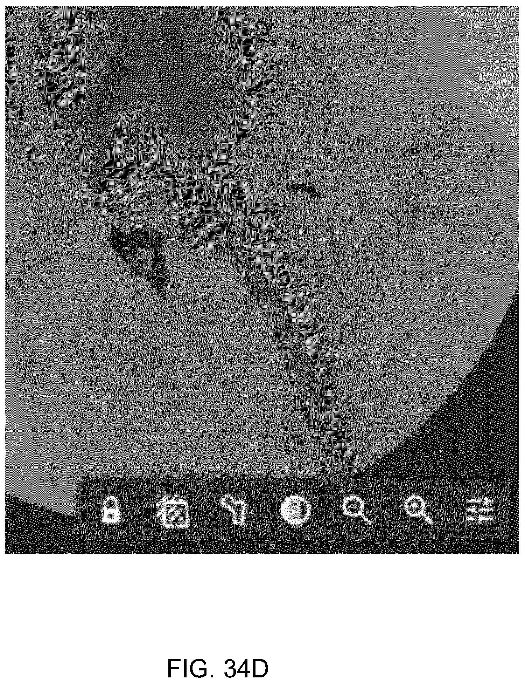

Additional aspects of the overlay visualization 3400 may be adjusted using additional controls on user interface control panel 3406. For example, opacity user interface control 3410 may be used to adjust the degree of opacity of three-dimensional model 3404. Opacity user interface control 3410 may include a graphical slider, radio buttons, checkboxes, or other suitable user interface controls corresponding to various degrees of opacity. Optionally, selecting an opacity less than 100% (i.e., making three-dimensional model 3404 at least partially transparent) may automatically colorize the bony anatomy shown in the two-dimensional image. User interface control panel 3406 may further include a three-dimensional model user interface control 3412, which may be used to toggle the three-dimensional model on and off entirely or to leave the representation of planned bone removal on while toggling on and off the other portions of the three-dimensional model (e.g., so that only the representation of planned bone removal is displayed overlaying the two-dimensional image, as shown in FIG. 34D). User interface control panel 3406 may also include a planned bone removal user interface control 3414, which may be used to toggle on and off a representation of planned bone removal, such as heat map 3424 or any other suitable form of representation (e.g., an outline or contour map). User interface control panel 3406 may also include user interface controls for zooming in (3416) and zooming out (3418) on visualization 3400. User interface control panel 3406 may also include an adjustment user interface control 3420. Adjustment user interface control 3420 may be used to adjust various parameters of three-dimensional model 3404, such as the rotation and position of three-dimensional model 3404 relative to two-dimensional image 3402. For example, FIG. 34C shows the three-dimensional model 3404 rotated out of alignment with the bony anatomy shown in two-dimensional image 3402.

An overlay may additionally, or alternatively, include a representation of the determined alignment between the three-dimensional model and the two-dimensional image. For example, the overlay may include a representation of the two-dimensional plane corresponding to the plane of the two-dimensional image. The representation of the two-dimensional plane may include the two-dimensional image itself (e.g., as shown in FIG. 32B discussed below). This may provide a surgeon with additional information to help guide the surgeon in removing bone.

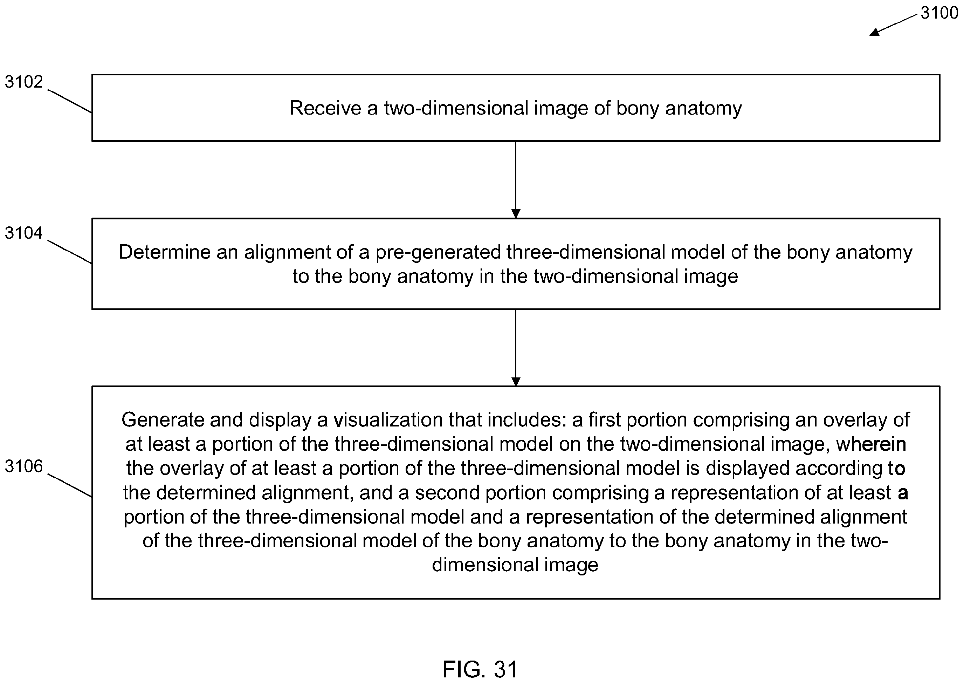

FIG. 31 illustrates an exemplary method 3100 for displaying a representation of the alignment between a three-dimensional model and a two-dimensional image. Method 3100 may be performed by one or more processors, such as the one or more processors of visual guidance system 125 of FIG. 18. At step 3102, a two-dimensional image of bony anatomy is received. Step 3102 may be substantially the same as step 2502 of method 2500 and, thus, the details of step 3102 are omitted for brevity. At step 3104, an alignment of a, e.g., pre-generated three-dimensional model of the bony anatomy to the bony anatomy in the two-dimensional image is determined. The alignment of the pre-generated three-dimensional model to the two-dimensional image may be determined according to method 2300 described above with reference to FIG. 23.

At step 3106, a visualization is generated and displayed. The visualization includes a first portion comprising an overlay of at least a portion of the three-dimensional model on the two-dimensional image. The overlay of at least a portion of the three-dimensional model is displayed according to the alignment determined in step 3104. The three-dimensional model may include a representation of planned bone removal (e.g., a heat map, an outline, or a contour map). The visualization may also include a second portion comprising a representation of at least a portion of the three-dimensional model and a representation of the determined alignment of the three-dimensional model of the bony anatomy to the bony anatomy in the two-dimensional image. The representation of the determined alignment may include a plane intersecting a portion of the three-dimensional model according to the determined alignment. The plane may be the plane of the two-dimensional image. Displaying the plane of the two-dimensional image as intersecting the three-dimensional model can allow a surgeon to visualize the location of the bony anatomy shown in the two-dimensional image relative to the three-dimensional model, even when the three-dimensional model is manipulated (e.g., when a surgeon rotates the three-dimensional model via a user interface to view the bony anatomy from different angles).

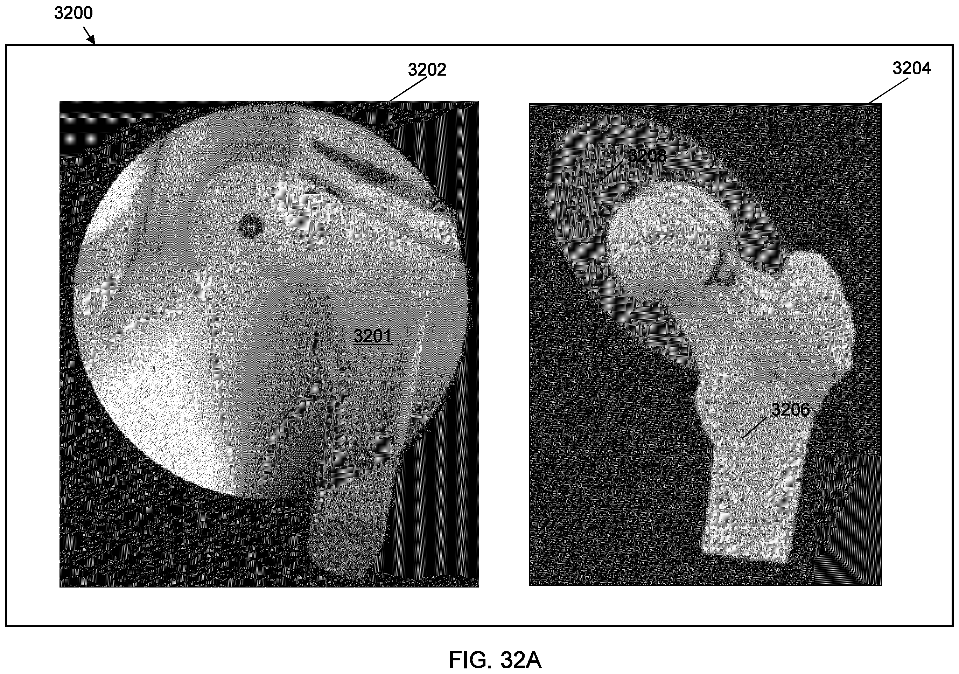

An exemplary visualization 3200 that may be generated according to method 3100 is illustrated in FIG. 32A. The visualization 3200 includes a first portion 3202 that includes an overlay 3201 of the three-dimensional model on the two-dimensional image. The visualization 3200 includes a second portion 3204 that includes a representation of the three-dimensional model 3206 and a generic plane 3208 that represents the determined alignment of the three-dimensional model 3206 to the bony anatomy in the two-dimensional image. The three-dimensional model 3206 may be rotatable by a user. The orientation of plane 3208 may be fixed relative to the orientation of three-dimensional model 3206, such that when the three-dimensional model 3206 is rotated, plane 3208 rotates with the three-dimensional model 3206. Thus, the intersection of plane 3208 with the three-dimensional model 3206 can be visualized from various angles. Second portion 3204 may be displayed alongside first portion 3202 or may be displayed by itself, independently of first portion 3202 (e.g., on a different display).

Optionally, the representation of the determined alignment of the three-dimensional model to the two-dimensional image may include a representation of the two-dimensional image intersecting the three-dimensional model according to the determined alignment. FIG. 32B illustrates an example of a visualization 3210 that may be displayed instead of or in addition to second portion 3204 of FIG. 32A. FIG. 32B illustrates a three-dimensional model 3212 that is intersected by a two-dimensional image 3214. Three-dimensional model 3212 includes a heat map 3218 showing planned bone removal. Two-dimensional image 3214 intersects three-dimensional model 3212 at the portion of three-dimensional model 3212 that corresponds to the plane in which two-dimensional image 3214 was taken (i.e., at the two-dimensional imaging plane).

Optionally, a representation of a surgical instrument captured in two-dimensional image 3214 may be shown in visualization 3210. The representation may show the surgical instrument two-dimensionally (e.g., the surgical instrument may be displayed as-is in two-dimensional image 3214, or a generic two-dimensional representation of a surgical instrument may be displayed) or three-dimensionally, as shown in FIG. 32B. A three-dimensional representation of a surgical tool 3216 may be generated by determining the type of surgical instrument in the two-dimensional image and the three-dimensional pose of that surgical instrument. Once the type of surgical instrument is known, a generic three-dimensional model corresponding to the surgical instrument may be selected from a repository of generic three-dimensional surgical instrument models stored in the system (e.g., visual guidance system 125 of FIG. 18). The three-dimensional pose of the surgical instrument may then be determined based on a projection of the generic three-dimensional model into two dimensions, such that the projection of the three-dimensional model aligns with the surgical instrument or portion thereof shown in the two-dimensional image. The three-dimensional representation of the surgical instrument may subsequently be displayed in the visualization 3210 according to the determined three-dimensional pose. Optionally, the representation of the surgical instrument may be toggled on and off by a user.

Optionally, visualization 3210 may include one or more clock face lines 3220 displayed on three-dimensional model 3212. Clock face lines may be used to help surgeons identify positions within the hip joint (e.g., for identifying rotational positions about the femoral head, the acetabular cup, etc.). For example, a "zero degree" clock face line may correspond to the plane of the surgical table, while a "twenty degree" clock face line may correspond to the plane tilted twenty degrees from the plane of the surgical table. In some examples, the two-dimensional imaging plane may correspond to a clock face line. In FIG. 32B, clock face line L corresponds to the two-dimensional imaging plane.

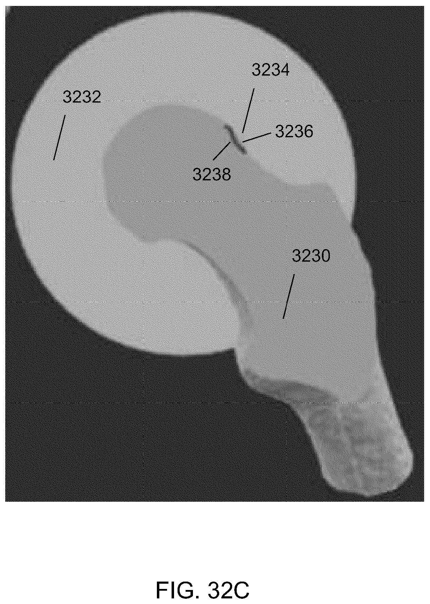

Optionally, a cross-section of the three-dimensional model may be cut along the clock face line corresponding to the two-dimensional imaging plane and displayed to a user. Displaying a cross-section of the three-dimensional model may help a surgeon visualize the depth of bone that needs to be resected in the plane of the two-dimensional image. FIG. 32C illustrates a cross-section 3230 of three-dimensional model 3212 cut along clock face line L. Optionally, the plane according to which cross-section 3230 was cut may be displayed along with the cross section. In some examples, plane 3232 may be a generic representation of a plane, as shown in FIG. 32C, or may be the two-dimensional image itself.

In some examples, cross-section 3230 includes (a cross section of) an indication of bone to be removed from a particular location, such as a cross-section of a heat map 3234. The cross-section of the heat map 3234 indicates bone to be removed from a particular location. In the three-dimensional model 3212 from which cross-section 3230 was cut, different colors or shading within the heat map 3218 indicate different depths of bone to be removed. For instance, in FIG. 32B, a portion of heat map 3218 shown in a first color may correspond to a first depth, while a portion of heat map 3218 shown in a second color may correspond to a different second depth. In cross-section 3230 of the three-dimensional model, however, the different colors or shading in the heat map may not be needed to indicate depth - when using cross-section 3230 to guide bone removal surgery, a surgeon removes bone according to the *shape* of the cross-section of the heat map 3234, rather than the coloration. For example, in FIG. 32C, the bone to be removed is all bone corresponding to portions 3236 and 3238 of heat map 3234-i.e., the entire cross-section of the heat map 3234 shown in the visualization. The cross-section of the heat map 3234 may still be colorized according to the depth of bone to be removed (e.g., portion 3236 may be shown in a first color, and portion 3238 may be shown in a different second color). However, the colors may be included in order to match the heat map from other visualizations where color corresponds to depth (e.g., visualization 3210 of FIG. 32B), not necessarily to show bone depth. This may help a surgeon reconcile what they see in the cross-sectional view with the full three-dimensional model view.

Returning to method 1900 of FIG. 19, a surgeon may desire to capture a new two-dimensional image, and a visualization (e.g., provided in a graphical user interface) may be updated to include the newly captured two-dimensional image with the three-dimensional model overlayed on the two-dimensional image. For example, a surgeon may move a tool to different locations proximate the bony anatomy and may capture two-dimensional images with the tool at the different locations, and the visualization may update to include the three-dimensional model overlaid on newly captured two-dimensional images as the two-dimensional images are captured, thereby displaying the location of the tool (as captured in the two-dimensional image) relative to the three-dimensional model. In some examples, the visualization is updated based on a newly captured and received two-dimensional image by repeating the determination of the alignment of the three-dimensional model to the two-dimensional image (e.g., step 1906 of method 1900). However, in situations in which neither the body moves, nor the camera moves, this repeated alignment determination may not be needed (because there is no relative movement between the body and the camera). As such, in some examples, the visualization is updated to include a newly captured two-dimensional image without re-determining the alignment of the model, which may avoid the computational expense associated with the alignment determination process and may improve the responsiveness of the updating of the visualization in instances in which new two-dimensional images are captured in quick succession.

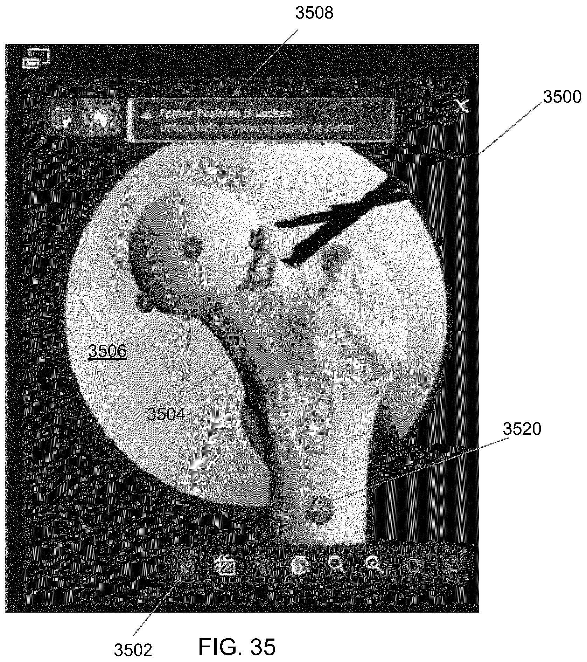

In some examples, computing system, such as visual guidance system 125, may automatically determine whether the alignment of the three-dimensional model should be re-determined when a newly captured two-dimensional image is captured. For example, the computing system may be configured to compare a newly received two-dimensional image with a previously received two-dimensional image to determine whether there has been gross movement that may correspond with movement of the bony anatomy and the camera relative to one another (e.g., because the body moved and/or because the camera moved). In other examples, a user may provide a user input to the computing system that selects a mode in which the alignment of the three-dimensional model relative to the two-dimensional image is not updated. For example, a graphical user interface may include a graphical control that can be selected by a user to toggle between a three-dimensional model alignment determination mode in which the alignment of the three-dimensional model is determined for newly received two-dimensional images and a three-dimensional model lock mode in which the alignment of the three-dimensional model is not determined for newly received two-dimensional image. An example of such a graphical user interface is illustrated in FIG. 35. Graphical user interface 3500 includes a graphical control 3502 in the exemplary form of a lock icon that a user may select to "lock" and "unlock" the three-dimensional model 3504. When "locked" (e.g., the lock icon is in a lock configuration, such as illustrated), the three-dimensional model alignment is not re-determined for newly received two-dimensional images 3506 such that the three-dimensional model 3504 does not move within the graphical user interface 3500 (unless manually manipulated). When "unlocked" (e.g., the lock icon is in an unlocked configuration), the three-dimensional model 3504 alignment is re-determined for newly received two-dimensional images 3506 such that the three-dimensional model position and orientation in the graphical user interface 3500 may change. Optionally, a notification 3508 may be provided in the graphical user interface 3500 indicating that the three-dimensional model position is locked, and optionally, a warning that the three-dimensional mode position should be unlocked before moving the subject or the intra-operative imaging system. The form of the graphical control 3502 illustrated in FIG. 35 is merely exemplary. Any suitable user control can be used to provide similar functionality, including any other type of graphical icon, a selection in a menu, a key combination, a voice command, a gesture, or a dedicated switch.

As noted above, a user may manually manipulate the position and/or orientation of the three-dimensional model in a visualization. A graphical user interface that includes a visualization may include one or more graphical controls that enable a user to select how user inputs map to adjustments in the position and/or orientation of the three-dimensional model in the visualization. Returning to FIG. 35 as an example, graphical user interface 3500 includes a user control 3520 that a user may use to toggle between different modes of manual manipulation of the three-dimensional model 3504. In the illustrated example, the user control 3520 can be used to switch between two modes. In a first mode, a user input for manipulating the orientation of the three-dimensional model, such as a slide or drag on a touchscreen, may control flexion/extension and internal/external rotation of the three-dimensional model, but not adduction/abduction. Taking the femur example shown in FIG. 35, the femur may be pinned at a particular location (e.g., the center of the femoral head). In a first mode, drag user inputs associated with the femur in the graphical user interface 3500 in a first set of directions (e.g., left-right) may control internal/external rotation of the femur, and drag user inputs associated with the femur in a second set of directions (e.g., up-down) may control extension/flexion of the femur. In a second mode (e.g., initiated via a user input to the user control 3520), drag user inputs associated with the femur may control, e.g., only abduction and adduction of the femur (and not flexion/extension and internal/external rotation). The user control 3520 may change in appearance to indicate the selected mode. For example, a portion of the user control 3520 associated with a selected mode (e.g., the top portion in the illustrated example may be associated with the flexion/extension and internal/external rotation mode) may be emphasized in a particular color.

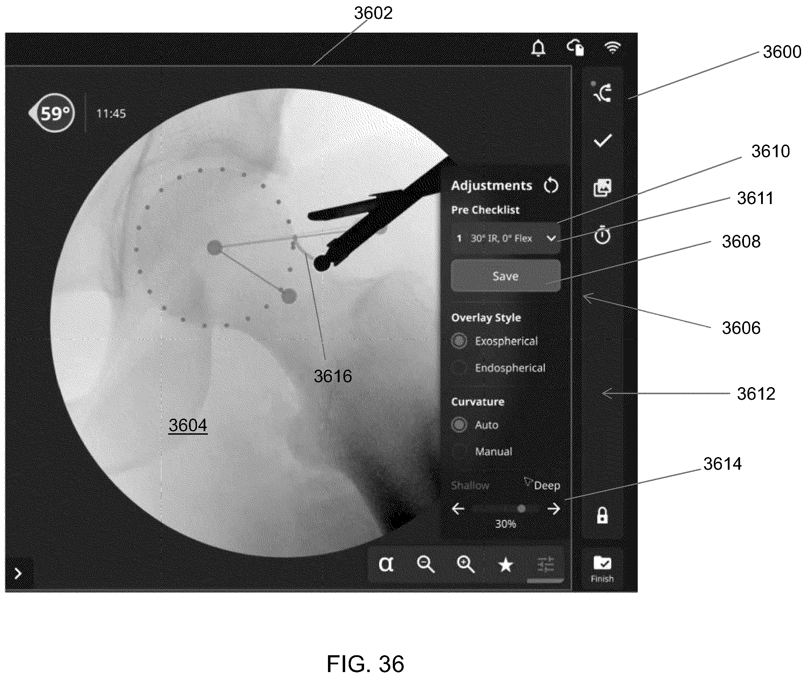

According to an aspect, a graphical user interface may enable a user to store images associated with a surgical procedure in association with one or more predefined checklists. FIG. 36 illustrates an exemplary user interface 3600 that enables a user to modify an annotated two-dimensional image and store the annotated two-dimensional image in association with a checklist. User interface 3600 includes a first region 3602 that displays an annotated two-dimensional image 3604 and a second region 3606 that includes controls for saving an annotated image in a predefined checklist. The second region 3606 may include a save object 3608 that a user can select to save the annotated image 3604 to a particular portion of a checklist indicated in a checklist region 3610. The checklist region 3610 may include a selector 3611 (e.g., a dropdown menu) that enables a user to select a portion of a checklist to which the annotated image 3604 is to be saved, thereby enabling a user to save different annotated images to different portions of a checklist. Optionally, upon saving an annotated image to a given portion of a checklist, the selector 3611 may automatically update to a suggested next portion of the checklist to which an annotated image needs to be saved, which may guide a user in progressing through a checklist without the user having to switch to a different user interface screen.

The user interface 3600 may include a third region 3612 that includes one or more controls 3614 for modifying the annotated image 3604. The one or more controls 3614 may enable a user to adjust how an annotation (a resection curve 3616) is generated, such as by adjusting one or more parameters used by the computing system to generate the annotation. The graphical user interface 3600 may enable a user to provide inputs directly to an annotation of the annotated image 3604 displayed in the first region 3602 to modify the annotation (for example, a user may tap or drag a particular portion of an annotation to adjust that portion of the annotation). By simultaneously providing the annotated image 3604, the ability to modify the annotation of the annotated image 3604, and the ability to save the annotated image 3604 to a checklist, user interface 3600 enables a user to view an annotated image, modify the annotated image, and save the annotated image to a checklist without switching between different user interface screens. Thus, user interface 3600 provides an improved user interface that can reduce the cognitive burden on the user and reduce errors associated with switching between different screens (e.g., saving the wrong image to the checklist or saving an image to the wrong portion of a checklist).

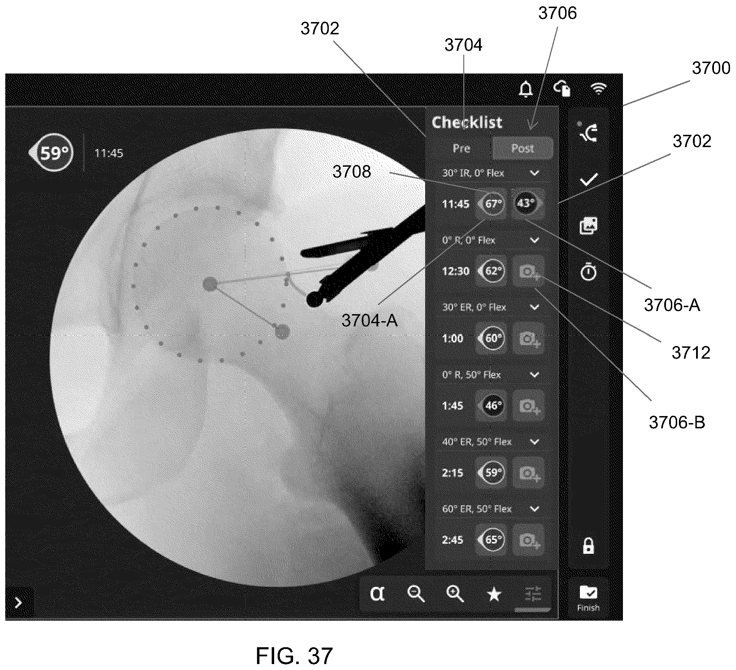

As noted above, a user may save different annotated images to different portions of a checklist. According to various aspects, a graphical user interface may display an overview of a checklist that enables a user to quickly and easy discern which portions of a checklist have been completed. FIG. 37 illustrates an exemplary user interface 3700 that includes a checklist summary 3702 that provides a simple and intuitive summary of a surgical procedure checklist that can enable a user to visualize the progress of a surgical procedure and/or the completion of documentation for a surgical procedure. The illustrated example is associated with a femoral resection procedure (e.g., resection associated with treating a cam-type FAI). Slots for saving annotated images of the femur, for example, at six different leg positions (e.g., a 30 degrees internal rotation and 0 degree flexion position, 0 degree rotation and 0 degree flexion position, etc.) are included in the checklist summary 3702. The slots may include a first set of slots 3704 associated with a pre-resection checklist and a second set of slots 3706 associated with a post-resection checklist. Each slot may include an icon that may indicate whether an annotated image has been saved for the given leg position and/or various information associated with the annotated image. In the illustrated example, a slot 3704-A of the first set of slots 3704 includes an icon 3708 that indicates that an annotated image has been stored in the corresponding portion of the checklist and an indication of the Alpha angle (67 degrees) determined for the image. The icon 3708 in the slot 3704-A may have a color, or other graphical indicator, that indicates whether (and, optionally, to what degree) the Alpha angle is out of a target range. For example, the icon 3708 may have a first color (e.g., green) indicating that the Alpha angle is within a target range (e.g., not needing resection), a second color (e.g., yellow) indicating that the Alpha angle is outside of the target range within a first out-of-target range, a third color (e.g., red) indicating that the Alpha angle is further outside of the target range within a second out-of-target range that is beyond the first range, etc.. If an annotated image has not been saved for a particular portion of a checklist, the corresponding slot may include an icon configured to indicate that no image has been saved. For example, slot 3706-B of the second set of slots 3706 includes an icon 3712 that indicates that no image has been saved to that portion of the checklist.

As noted above, the first set of slots 3704 may be associated with a pre-resection checklist and the second set of slots 3706 may be associated with a post-resection checklist. Thus, the checklist summary 3702 provides a simple graphical summary of both the pre- and post- resection checklists simultaneously. To the extent the checklist is used as the resection progresses, the checklist summary 3702 can indicate the progress of the resection (e.g., slots with icon 3712 may indicate that the associated portion of the resection has not yet been completed). The simultaneous display of the pre- and post- resection checklist information provided by checklist summary 3702 can also provide a user with a simple comparison between the anatomy before the resection and after the resection. For example, the icon in slot 3704-A indicates that the Alpha angle for the femur at the associated leg position was 67 degrees before resection and the icon in slot 3706-A indicates that the Alpha angle for the femur at that same leg position after resection is 43 degrees, enabling the user to quickly and easily see the progress of the resection.

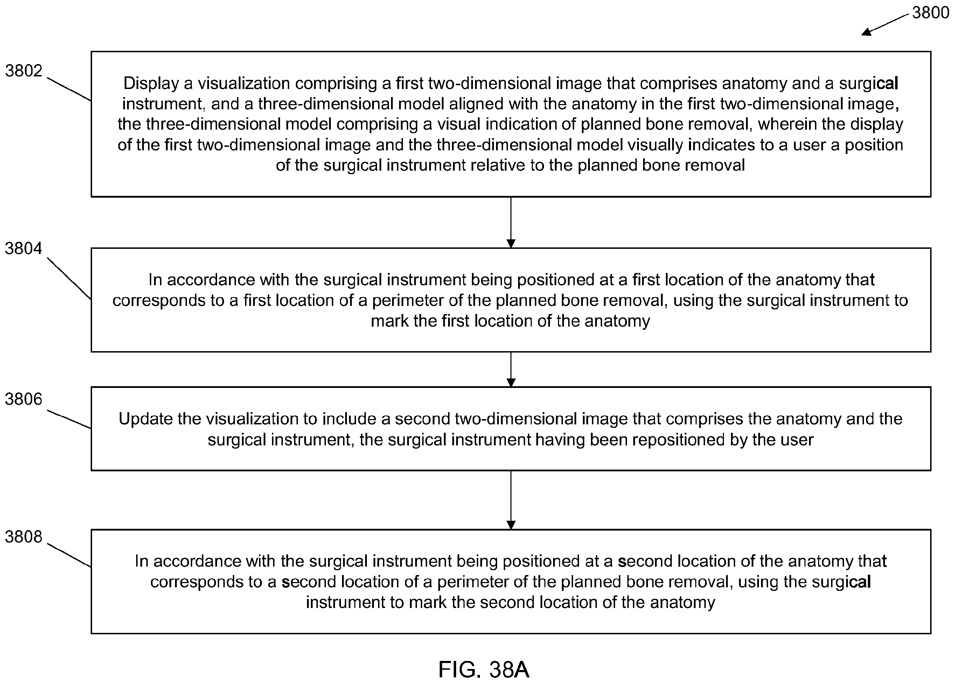

According to various aspects, graphical user interfaces and visualizations disclosed herein, such as visualization 2400 of FIG. 24, provide a surgeon with visual guidance that guides a surgeon through a surgical procedure involving resection of bony anatomy by displaying a model of planned bone removal aligned with bony anatomy in a two-dimensional image. Optionally, a surgeon may utilize the visual guidance to guide in marking at least a portion a perimeter of a region of bone resection with a surgical instrument, thereby enabling the surgeon to resect the region of bone without needing to reference the visual guidance throughout the resection process, which may reduce the amount of time required for the resection. FIG. 38A is a flow diagram of a method 3800 for using a bone removal guidance visualization to mark at least a portion of a perimeter of a planned resection of bony anatomy. Method 3800 can be used in conjunction with any of the methods described here.

At step 3802, a computing system, such as a visual guidance system 125, displays a visualization that includes a first two-dimensional image of anatomy. The visualization may be generated and displayed according to any of the methods described herein. The two-dimensional image of the anatomy may include a surgical instrument located in the surgical cavity proximate the anatomy. The surgical instrument may be a tissue removal instrument, such as an RF ablation instrument for ablating soft tissue or a bur for removing bony tissue. The graphical user interface includes a three-dimensional model aligned with the anatomy in the first two-dimensional image (using any of the techniques described herein). The three-dimensional model includes a visual indication of planned bone removal (e.g., a heat map). The display of the three-dimensional model aligned to the two-dimensional image visually indicates to a surgeon a position of the surgical instrument relative to the planned bone removal.

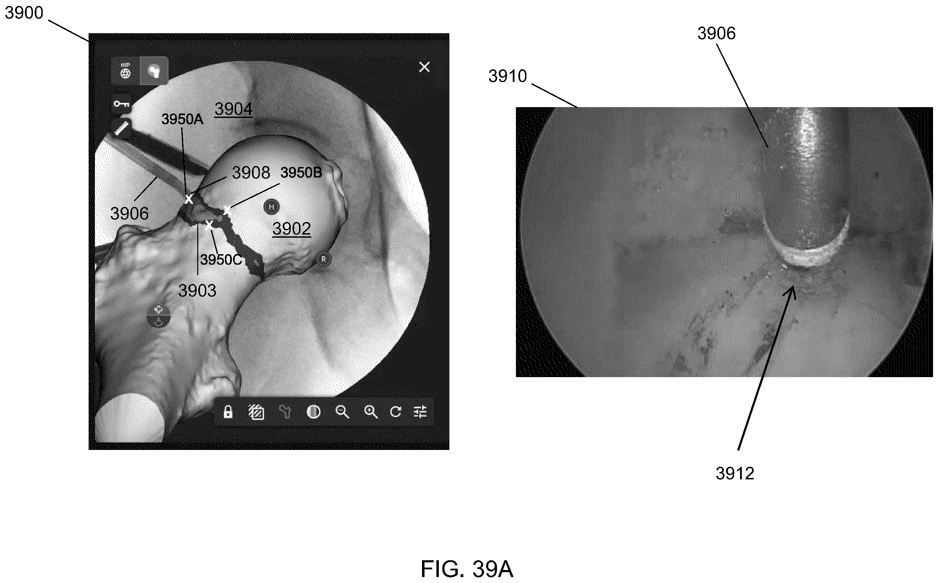

FIG. 39A illustrates an exemplary visualization 3900 that includes a three-dimensional model 3902 overlaying a two-dimensional image 3904, with the three-dimensional model 3902 including a visual indication of planned bone removal in the form of a heat map 3903. The two-dimensional image 3904 includes a surgical instrument 3906. In the illustrated example, the surgical instrument 3906 was positioned near bony anatomy such that, in the visualization 3900, the surgical instrument 3906 appears proximate the three-dimensional model 3902.

In step 3804 of method 3800, if the surgeon observes from the displayed visualization, that the surgical instrument is positioned at a location of the anatomy that corresponds to a perimeter of the planned bone removal, the surgeon may mark that location of the anatomy using the surgical instrument. For example, in the example of FIG. 39A, the surgical instrument 3906 is positioned proximate a first location 3908 of a perimeter of the heat map 3903. As such, a surgeon may use the surgical instrument 3906 while at that position to mark the anatomy at the corresponding location. FIG. 39A illustrates an endoscopic view 3910 that corresponds to the visualization 3900 and includes the surgical instrument 3906 positioned at a position of the anatomy corresponding to its location in the two-dimensional image 3904 (i.e., the two-dimensional image 3904 was captured when the surgical instrument 3906 was at the location that it is in the endoscopic view). The surgeon may use the surgical instrument 3906 to mark the location 3912 of the anatomy (e.g., by burning the soft tissue with an RF ablation instrument, as shown, or removing bone with a bur).

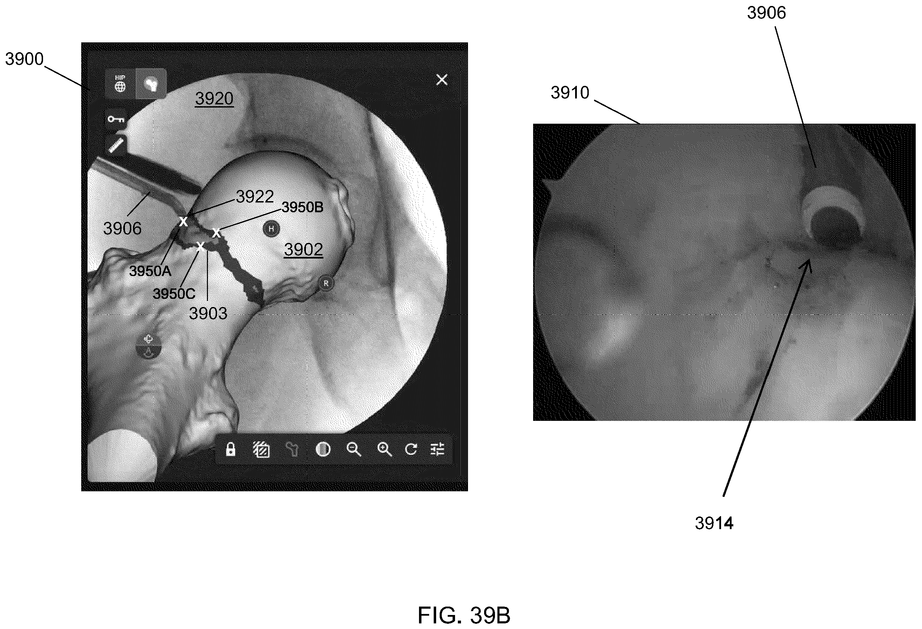

After marking the location 3912, the surgeon may move the surgical instrument 3906 to a different location relative to the anatomy and may capture a two-dimensional with the surgical instrument 3906 in the new location. At step 3806, after the surgical instrument has been repositioned, the computing system may update the visualization to include to include a two-dimensional image captured with the surgical instrument at the new location. The three-dimensional model may be re-aligned with the anatomy in the newly captured two-dimensional image (or, the three-dimensional model alignment may not be re-aligned, if for example, a user has selected to "lock" the model, as described above). FIG. 39B illustrates the visualization 3900 after being updated with a two-dimensional image 3920 captured after the surgical instrument was moved a new location of the anatomy, as shown in the endoscopic view 3910 in which the surgical instrument 3906 is at a different location 3914.

At step 3808, if the surgical instrument is positioned at a second location of the anatomy that corresponds to a second location of a perimeter of the planned bone removal, the surgeon may use the surgical instrument to mark the second location of the anatomy. For example, in the example of FIG. 39B, the surgical instrument 3906 is positioned at a second location of the anatomy that corresponds to a second location 3922 of a perimeter of the heat map 3903. As such, a surgeon may use the surgical instrument 3906 while at that position to mark the anatomy at that location (3914 in endoscopic view 3910.

Steps 3802 to 3808 can be repeated as many times as desired, such as to mark the entire perimeter of the resection area or just a portion of the perimeter of the resection area. For example, a surgeon may mark all, or just a portion of, the medial side of the resection area, the anterior side of the resection area, and/or the posterior side of the resection area The surgeon may make a mark at the greatest extent of each side of the resection area in a particular direction, or the surgeon may mark the most superior part of the cam, the most anterior part of the cam, and/or the most anterior part of the cam. One or more portions of the perimeter of the resection area may be marked in any suitable fashion (which may depend on the surgeon's preferences), such as with a plurality of discrete marks or with a continuous line. Method 3000 may include additional marking of the anatomy that does not include the capture and display of an updated two-dimensional image. For example, the surgeon may mark one or more locations at one or more steps that occur between step 3804 and step 3806. The surgeon may move the instrument a relatively small amount that does not require updated visualization guidance. The amount of movement without updated visualization guidance may depend on the experience of the surgeon. For example, a more experienced surgeon may be able to mark the perimeter of a resection area with fewer updates to the visualization (i.e., fewer two-dimensional images) than a less experienced surgeon.

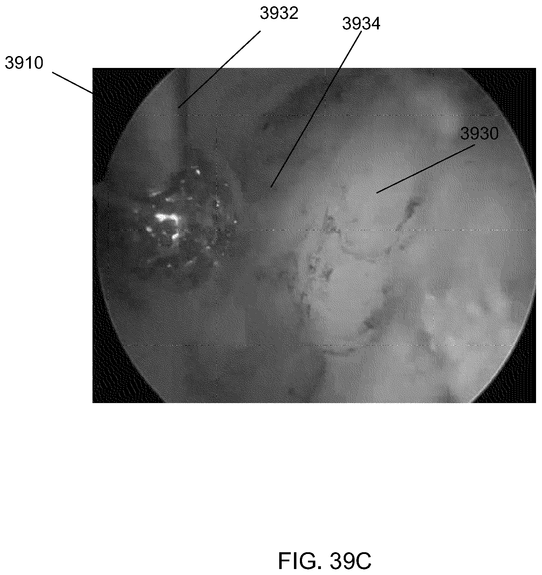

With the perimeter of the resection area marked, the surgeon may resect the portion of the anatomy within the marked perimeter and may do so without needing to reference the visualization 3900. The surgeon may use the same instrument that was used to mark the perimeter to resect the bone (e.g., if the perimeter was marked with a bur) or may use a different instrument to resect the bone (e.g., if an RF ablation instrument was used to mark the tissue and a bur is used to resect the bone). FIG. 39C shows the endoscopic view 3910 when a surgeon is using a bur 3932 to remove bone from a region 3934 of bony anatomy within a perimeter 3936 marked according to method 3800.

According to an aspect, method 3800 may include representing, in a visualization, one or more of the markings that are made by the surgeon, such as visualization 3900. The computing system may identify the instrument in the two-dimensional image, determine the location of a tip of the instrument relative to the three-dimensional model, and include a visual representation (a virtual mark) in the visualization at a location relative to the three-dimensional model that corresponds to the location of the tip of the instrument relative to the bony anatomy. This may assist a surgeon in tracking where the surgeon has already marked the resection area and what portions of the resection area still need marking (if any).

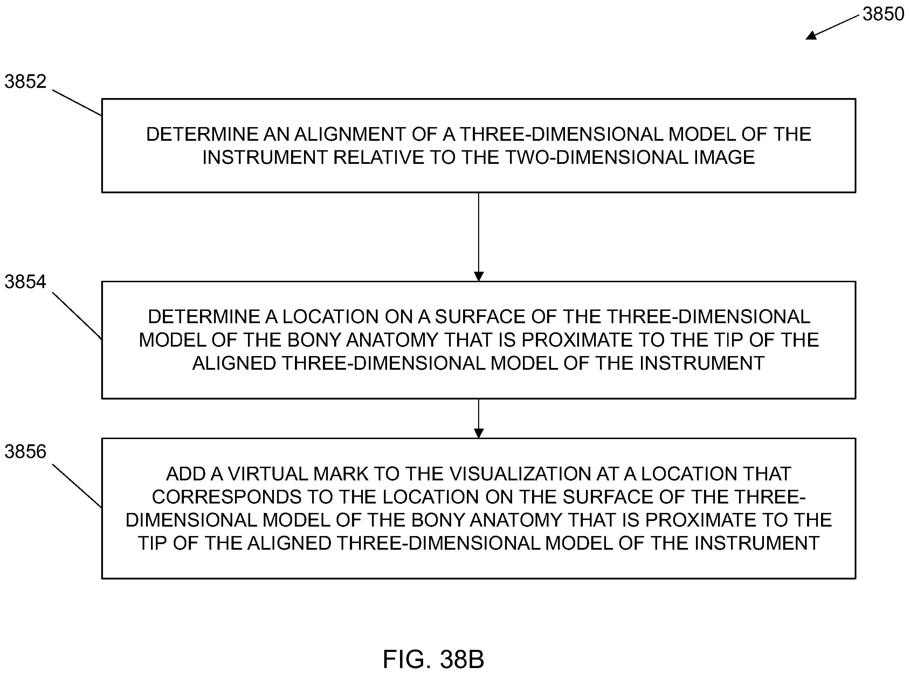

FIG. 38B illustrates a method 3850 that may be performed by a computing system (e.g., such as a visual guidance system 125) for including virtual marks in a visualization that indicate marks made in the anatomy by the surgeon. Method 3850 may be performed in conjunction with any of the methods described herein. Method 3850 may be performed along with steps 3802-3808 of FIG. 38A and may be repeated for any number of virtual marks. At step 3850, the two-dimensional image displayed in the visualization in step 3802 of method 3800 is analyzed to identify the surgical instrument in the image. For example, the computing system may utilize a machine learning model trained to identify the surgical instrument in X-ray images, such as discussed above with respect to step 2704 of method 200.

At step 3852, an alignment of a three-dimensional model of the instrument relative to the two-dimensional image may be determined. Step 3852 is analogous to step 1906 of method 1900 and, as such, may include using method 2300 to determine the alignment of a three-dimensional model of the instrument relative to the two-dimensional image (optional aspects of determining the alignment of the instrument relative to the two-dimensional image are discussed above with respect to step 2708 of method 2700). Given the alignment of the three-dimensional model of the bony anatomy relative to the two-dimensional image determined at step 1900 and the alignment of the three-dimensional model of the instrument relative to the two-dimensional image at step 3852, the alignment of the three-dimensional model of the instrument and the three-dimensional model of the bony anatomy relative to one another (e.g., in a common reference frame) is known. Based on the relative alignment between the three-dimensional model of the instrument and the three-dimensional model of the bony anatomy resulting from step 3852, at step 3854, a location on a surface of the three-dimensional model of the bony anatomy that is proximate to the tip of the aligned three-dimensional model of the instrument is determined. Step 3854 may include determining the location on the surface of the three-dimensional model of the bony anatomy where the three-dimensional model of the instrument intersects with the three-dimensional model of the bony anatomy or a nearest point of the three-dimensional model of the bony anatomy to the tip of the three-dimensional model of the instrument. Optionally, the location on the surface of the three-dimensional model that is determined in step 3854 is constrained to be a location on or near to a perimeter of the representation of the planned bone removal. Any other suitable machine vision techniques may be used to determine the location of the tip of the instrument with respect to the three-dimensional model, according to various aspects.

At step 3856, a virtual mark is added to the visualization at a location that corresponds to the location on the surface of the three-dimensional model of the bony anatomy that is proximate to the tip of the aligned three-dimensional model of the instrument. An exemplary virtual mark 3950A is illustrated in FIGS. 39A and 39B and may result from the performance of method 3850 after the user marks location 3912 at step 3804 of method 3800. FIGS. 39A and 39B also include exemplary virtual marks 3950B and 3950C, which correspond to markings made by the surgeon previously during the procedure. The form of the virtual marks in FIGS. 39A and 39B is merely exemplary. Any shapes, symbols, icons, or other graphical feature could be used as a virtual mark.

Optionally, one or more virtual marks may include a label indicating the anatomical position of the location of the corresponding marking of the anatomy. For example, a virtual mark may include a label such as posterior, anterior, lateral, or medial that represents the relative location of the marking of the anatomy. A label may be added manually by a user, such as via a voice command or menu selection, or may be added automatically based on information embedded in the three-dimensional model of the bony anatomy.

In some examples, virtual marks are added to a visualization, such as visualization 3900, in response to an input to the computing system that indicates that the user has marked the anatomy. The input may be, for example, a user input, such as a button press (e.g., a button of the instrument) or a voice command. Alternatively, the input may be a signal from a controller of the instrument that indicates use of the instrument. Additionally, or alternatively, the computing system may process one or more endoscopic images to detect marking of the anatomy by the surgeon and add the virtual mark to the visualization in response to the detection of the marking.

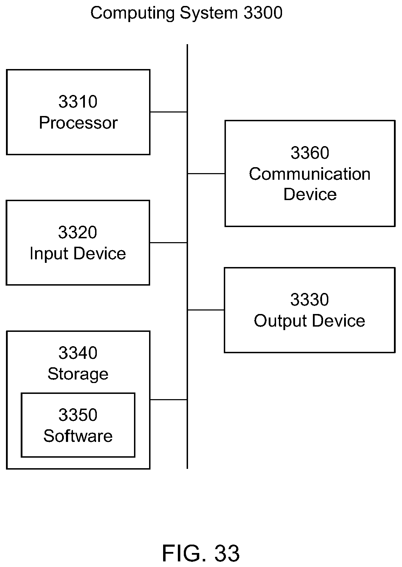

FIG. 33 illustrates an example of a computing system 3300. System 3300 may be used, for example, for visual guidance system 125. System 3300 can be a computer connected to a network. System 3300 can be a client computer or a server. As shown in FIG. 33, system 3300 can be any suitable type of microprocessor-based system, such as a personal computer, workstation, server, or handheld computing device (portable electronic device) such as a phone or tablet. The system can include, for example, one or more of a processor 3310, input device 3320, output device 3330, storage 3340, and communication device 3360. Input device 3320 and output device 3330 can generally correspond to those described above and can either be connectable or integrated with the computer.

Input device 3320 can be any suitable device that provides input, such as a touch screen, keyboard or keypad, mouse, gesture recognition component of a virtual/augmented reality system, or voice-recognition device. Output device 3330 can be or include any suitable device that provides output, such as a touch screen, haptics device, virtual/augmented reality display, or speaker.

Storage 3340 can be any suitable device that provides storage, such as an electrical, magnetic, or optical memory, including a RAM, cache, hard drive, removable storage disk, or other non-transitory computer readable medium. Communication device 3360 can include any suitable device capable of transmitting and receiving signals over a network, such as a network interface chip or device. The components of the computer can be connected in any suitable manner, such as via a physical bus or wirelessly.

Software 3350, which can be stored in storage 3340 and executed by processor 3310, can include, for example, the programming that embodies the functionality of the present disclosure (e.g., as embodied in the devices as described above). For example, software 3350 can include one or more programs for performing one or more of the steps of method 1900, method 2100, method 2300, method 2500, method 2700, method 2900, and/or method 3100.

Software 3350 can also be stored and/or transported within any non-transitory computer-readable storage medium for use by or in connection with an instruction execution system, apparatus, or device, such as those described above, that can fetch instructions associated with the software from the instruction execution system, apparatus, or device and execute the instructions. In the context of this disclosure, a computer-readable storage medium can be any medium, such as storage 3340, that can contain or store programming for use by or in connection with an instruction execution system, apparatus, or device.

Software 3350 can also be propagated within any transport medium for use by or in connection with an instruction execution system, apparatus, or device, such as those described above, that can fetch instructions associated with the software from the instruction execution system, apparatus, or device and execute the instructions. In the context of this disclosure, a transport medium can be any medium that can communicate, propagate, or transport programming for use by or in connection with an instruction execution system, apparatus, or device. The transport readable medium can include, but is not limited to, an electronic, magnetic, optical, electromagnetic, or infrared wired or wireless propagation medium.

System 3300 may be connected to a network, which can be any suitable type of interconnected communication system. The network can implement any suitable communications protocol and can be secured by any suitable security protocol. The network can comprise network links of any suitable arrangement that can implement the transmission and reception of network signals, such as wireless network connections, T1 or T3 lines, cable networks, DSL, or telephone lines.

System 3300 can implement any operating system suitable for operating on the network. Software 3350 can be written in any suitable programming language, such as C, C++, Java, or Python. In various aspects, application software embodying the functionality of the present disclosure can be deployed in different configurations, such as in a client/server arrangement or through a Web browser as a Web-based application or Web service, for example.

The foregoing description, for the purpose of explanation, has been described with reference to specific aspects. However, the illustrative discussions above are not intended to be exhaustive or to limit the invention to the precise forms disclosed. Many modifications and variations are possible in view of the above teachings. The aspects were chosen and described in order to best explain the principles of the techniques and their practical applications. Others skilled in the art are thereby enabled to best utilize the techniques and various aspects with various modifications as are suited to the particular use contemplated.

Although the disclosure and examples have been fully described with reference to the accompanying figures, it is to be noted that various changes and modifications will become apparent to those skilled in the art. Such changes and modifications are to be understood as being included within the scope of the disclosure and examples as defined by the claims. Finally, the entire disclosure of any patents and publications referred to in this application are hereby incorporated herein by reference.

### CLAUSES

The following clauses are exemplary and are not intended to limit the scope of the disclosure provided herein.

Clause 1. A method for guiding bone removal comprising: receiving a two-dimensional image of a portion of bony anatomy; determining at least one characteristic of the portion of the bony anatomy associated with an amount of the bony anatomy in the two-dimensional image; determining a sufficiency of the amount of the bony anatomy in the two-dimensional image for determining an alignment of a pre-generated three-dimensional model of the bony anatomy to the bony anatomy in the two-dimensional image by comparing the at least one characteristic of the portion of the bony anatomy with at least one predetermined criteria; and providing a user indication associated with the sufficiency of the amount of the bony anatomy in the two-dimensional image for determining the alignment of the pre-generated three-dimensional model of the bony anatomy to the bony anatomy in the two-dimensional image.

Clause 2. The method of clause 1, wherein determining the at least one characteristic comprises determining whether at least one anatomical landmark is present in the two-dimensional image.

Clause 3. The method of clause 1 or 2, wherein determining the at least one characteristic comprises analyzing the two-dimensional image to identify at least one anatomical landmark.

Clause 4. The method of any one of the preceding clauses, wherein determining the at least one characteristic comprises determining a ratio corresponding to at least a predetermined region of the bony anatomy.

Clause 5. The method of any one of the preceding clauses, wherein the at least one predetermined criteria comprises a ratio.

Clause 6. The method of any one of the preceding clauses, wherein the at least one characteristic comprises a presence or absence of one or more anatomical landmarks in the two-dimensional image, the at least one predetermined criteria comprises a list of one or more anatomical landmarks, and comparing the at least one characteristic of the portion of the bony anatomy with the at least one predetermined criteria comprises determining whether one or more anatomical landmarks from the list are present in the two-dimensional image.

Clause 7. The method of any one of the preceding clauses, comprising determining the alignment of the pre-generated three-dimensional model of the bony anatomy to the bony anatomy in the two-dimensional image and displaying a visualization based on the determined alignment of the pre-generated three-dimensional model of the bony anatomy to the bony anatomy in the two-dimensional image, wherein the visualization comprises the user indication associated with the sufficiency of the amount of the bony anatomy in the two-dimensional image for determining the alignment of the pre-generated three-dimensional model of the bony anatomy to the bony anatomy in the two-dimensional image.

Clause 8. The method of clause 7, wherein the user indication indicates an assessment of reliability of the alignment of the pre-generated three-dimensional model of the bony anatomy to the bony anatomy in the two-dimensional image.

Clause 9. The method of clause 7 or 8, wherein the visualization comprises an overlay of at least a portion of the pre-generated three-dimensional model on the bony anatomy in the two-dimensional image.

Clause 10. The method of any one of the preceding clauses, wherein determining the sufficiency of the amount of the bony anatomy in the two-dimensional image for determining the alignment of the pre-generated three-dimensional model of the bony anatomy to the bony anatomy in the two-dimensional image comprises determining that the amount of the bony anatomy in the two-dimensional image is insufficient for determining the alignment of the pre-generated three-dimensional model of the bony anatomy to the bony anatomy in the two-dimensional image, and wherein the user indication is configured to indicate to a user that a different two-dimensional image of the bony anatomy is required.

Clause 11. The method of any one of the preceding clauses, wherein the user indication associated with the sufficiency of the amount of the bony anatomy in the two-dimensional image for determining the alignment of the pre-generated three-dimensional model of the bony anatomy to the bony anatomy in the two-dimensional image is provided only if the amount of the bony anatomy in the two-dimensional image is determined to be sufficient for determining the alignment of the pre-generated three-dimensional model of the bony anatomy to the bony anatomy in the two-dimensional image.

Clause 12. A system for guiding bone removal during a surgical procedure, the system comprising one or more processors, memory, and one or more programs, wherein the one or more programs are stored in the memory and configured to be executed by the one or more processors, the one or more programs including instructions for: receiving a two-dimensional image of a portion of bony anatomy; determining at least one characteristic of the portion of the bony anatomy associated with an amount of the bony anatomy in the two-dimensional image; determining a sufficiency of the amount of the bony anatomy in the two-dimensional image for determining an alignment of a pre-generated three-dimensional model of the bony anatomy to the bony anatomy in the two-dimensional image by comparing the at least one characteristic of the portion of the bony anatomy with at least one predetermined criteria; and providing a user indication associated with the sufficiency of the amount of the bony anatomy in the two-dimensional image for determining the alignment of the pre-generated three-dimensional model of the bony anatomy to the bony anatomy in the two-dimensional image.

Clause 13. The system of clause 12, wherein determining the at least one characteristic comprises determining whether at least one anatomical landmark is present in the two-dimensional image.

Clause 14. The system of clause 12 or 13, wherein determining the at least one characteristic comprises analyzing the two-dimensional image to identify at least one anatomical landmark.

Clause 15. The system of any one of clauses 12-14, wherein determining the at least one characteristic comprises determining a ratio corresponding to at least a predetermined region of the bony anatomy.

Clause 16. The system of any one of clauses 12-15, wherein the at least one predetermined criteria comprises a ratio.

Clause 17. The system of any one of clauses 12-16, wherein the at least one characteristic comprises a presence or absence of one or more anatomical landmarks in the two-dimensional image, the at least one predetermined criteria comprises a list of one or more anatomical landmarks, and comparing the at least one characteristic of the portion of the bony anatomy with the at least one predetermined criteria comprises determining whether one or more anatomical landmarks from the list are present in the two-dimensional image.

Clause 18. The system of any one of clauses 12-17, wherein the one or more programs include instructions for determining the alignment of the pre-generated three-dimensional model of the bony anatomy to the bony anatomy in the two-dimensional image and displaying a visualization based on the determined alignment of the pre-generated three-dimensional model of the bony anatomy to the bony anatomy in the two-dimensional image, wherein the visualization comprises the user indication associated with the sufficiency of the amount of the bony anatomy in the two-dimensional image for determining the alignment of the pre-generated three-dimensional model of the bony anatomy to the bony anatomy in the two-dimensional image.

Clause 19. The system of clause 18, wherein the user indication indicates an assessment of reliability of the alignment of the pre-generated three-dimensional model of the bony anatomy to the bony anatomy in the two-dimensional image.

Clause 20. The system of clause 18 or 19, wherein the visualization comprises an overlay of at least a portion of the pre-generated three-dimensional model on the bony anatomy in the two-dimensional image.

Clause 21. The system of any one of clauses 12-20, wherein determining the sufficiency of the amount of the bony anatomy in the two-dimensional image for determining the alignment of the pre-generated three-dimensional model of the bony anatomy to the bony anatomy in the two-dimensional image comprises determining that the amount of the bony anatomy in the two-dimensional image is insufficient for determining the alignment of the pre-generated three-dimensional model of the bony anatomy to the bony anatomy in the two-dimensional image, and wherein the user indication is configured to indicate to a user that a different two-dimensional image of the bony anatomy is required.

Clause 22. The system of any one of clauses 12-21, wherein the user indication associated with the sufficiency of the amount of the bony anatomy in the two-dimensional image for determining the alignment of the pre-generated three-dimensional model of the bony anatomy to the bony anatomy in the two-dimensional image is provided only if the amount of the bony anatomy in the two-dimensional image is determined to be sufficient for determining the alignment of the pre-generated three-dimensional model of the bony anatomy to the bony anatomy in the two-dimensional image.

Clause 23. A method for guiding bone removal for a surgical procedure, the method comprising: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment; receiving a user request to change a transparency of the overlay of at least a portion of a three-dimensional model; and updating the visualization by changing the transparency of the overlay according to the user request.

Clause 24. The method of clause 23, wherein the at least a portion of the three-dimensional model comprises a representation of the bony anatomy.

Clause 25. The method of clause 23 or 24, wherein the at least a portion of the three-dimensional model comprises a representation of planned bone removal.

Clause 26. The method of any one of clauses 23-25, wherein the user request comprises a selection of a predetermined level of transparency.

Clause 27. The method of any one of clauses 23-26, wherein the user request comprises an input to a graphical slider displayed in association with the visualization.

Clause 28. A system for guiding bone removal for a surgical procedure, the system comprising one or more processors, memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment; receiving a user request to change a transparency of the overlay of at least a portion of a three-dimensional model; and updating the visualization by changing the transparency of the overlay according to the user request.

Clause 29. The system of clause 28, wherein the at least a portion of the three-dimensional model comprises a representation of the bony anatomy.

Clause 30. The system of clause 28 or 29, wherein the at least a portion of the three-dimensional model comprises a representation of planned bone removal.

Clause 31. The system of any one of clauses 28-30, wherein the user request comprises a selection of a predetermined level of transparency.

Clause 32. The system of any one of clauses 28-31, wherein the user request comprises an input to a graphical slider displayed in association with the visualization.

Clause 33. A method for guiding bone removal for a surgical procedure, the method comprising: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment and the overlay of at least a portion of the three-dimensional model comprises a visual indication of planned bone removal; receiving a user request to toggle off display of the visual indication of planned bone removal; and updating the visualization by ceasing to display the visual indication of planned bone removal while continuing to display the two-dimensional image.

Clause 34. The method of clause 33, wherein the visual indication of planned bone removal comprises a heat map.

Clause 35. The method of clause 33 or 34, wherein the overlay comprises a representation of at least a portion of the bony anatomy and updating the visualization comprises ceasing to display the representation of the at least a portion of the bony anatomy.

Clause 36. The method of clause 33 or 34, wherein the overlay comprises a representation of at least a portion of the bony anatomy and updating the visualization comprises continuing to display the representation of the at least a portion of the bony anatomy.

Clause 37. A system for guiding bone removal for a surgical procedure, the system comprising one or more processors, memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; generating and displaying a visualization comprising an overlay of at least a portion of the three-dimensional model on the two-dimensional image, wherein the overlay of at least a portion of the three-dimensional model is displayed according to the determined alignment and the overlay of at least a portion of the three-dimensional model comprises a visual indication of planned bone removal; receiving a user request to toggle off display of the visual indication of planned bone removal; and updating the visualization by ceasing to display the visual indication of planned bone removal while continuing to display the two-dimensional image.

Clause 38. The system of clause 37, wherein the visual indication of planned bone removal comprises a heat map.

Clause 39. The system of clause 37 or 38, wherein the overlay comprises a representation of at least a portion of the bony anatomy and updating the visualization comprises ceasing to display the representation of the at least a portion of the bony anatomy.

Clause 40. The system of any one of clauses 37 or 38, wherein the overlay comprises a representation of at least a portion of the bony anatomy and updating the visualization comprises continuing to display the representation of the at least a portion of the bony anatomy.

Clause 41. A method for guiding bone removal for a surgical procedure, the method comprising: receiving a two-dimensional image of bony anatomy, the two-dimensional image comprising at least a portion of a surgical tool; identifying the at least a portion of the surgical tool in the two-dimensional image; determining an alignment of a pre-generated three-dimensional model of the bony anatomy to the bony anatomy in the two-dimensional image; and generating and displaying a visualization comprising an overlay of at least a portion of the three-dimensional model on the two-dimensional image and a representation of the at least a portion of the surgical tool, wherein the at least a portion of the three-dimensional model is overlaid on the two-dimensional image according to the determined alignment.

Clause 42. The method of clause 41, wherein the representation of the at least a portion of the surgical tool is overlaid on the at least a portion of the three-dimensional model in the visualization.

Clause 43. The method of clause 42, comprising: toggling off the overlay of the representation of the at least a portion of the surgical tool on the at least a portion of the three-dimensional model in the visualization; and toggling on an overlay of the representation of the at least a portion of the surgical tool on the two-dimensional image, behind the three-dimensional model in the visualization.

Clause 44. The method of any one of clauses 41-43, wherein the representation of the at least a portion of the surgical tool comprises a silhouette of the at least a portion of the surgical tool.

Clause 45. The method of any one of clauses 41-43, wherein the representation of the at least a portion of the surgical tool comprises an outline of the at least a portion of the surgical tool.

Clause 46. The method of any one of clauses 41-43, wherein the representation of the at least a portion of the surgical tool comprises a three-dimensional representation of the at least a portion of the surgical tool.

Clause 47. The method of any one of clauses 41-46, wherein the representation of the at least a portion of the surgical tool is displayed with an amount of transparency.

Clause 48. The method of any one of clauses 41-47, comprising toggling off display of the representation of the at least a portion of the surgical tool.

Clause 49. The method of any one of clauses 41-48, comprising determining a three-dimensional pose of the at least a portion of the surgical tool in the two-dimensional image, wherein the representation of the at least a portion of the surgical tool is displayed in the visualization according to the determined three-dimensional pose.

Clause 50. The method of any one of clauses 41-49, wherein the at least a portion of the three-dimensional model comprises a representation of planned bone removal.

Clause 51. A system for guiding bone removal for a surgical procedure, the system comprising one or more processors, memory, and one or more programs, wherein the one or more programs are stored in the memory and configured to be executed by the one or more processors, the one or more processors including instructions for: receiving a two-dimensional image of bony anatomy, the two-dimensional image comprising at least a portion of a surgical tool; identifying the at least a portion of the surgical tool in the two-dimensional image; determining an alignment of a pre-generated three-dimensional model of the bony anatomy to the bony anatomy in the two-dimensional image; and generating and displaying a visualization comprising an overlay of at least a portion of the three-dimensional model on the two-dimensional image and a representation of the at least a portion of the surgical tool, wherein the at least a portion of the three-dimensional model is overlaid on the two-dimensional image according to the determined alignment.

Clause 52. The system of clause 51, wherein the representation of the at least a portion of the surgical tool is overlaid on the at least a portion of the three-dimensional model in the visualization.

Clause 53. The system of clause 52, wherein the one or more programs include instructions for: toggling off the overlay of the representation of the at least a portion of the surgical tool on the at least a portion of the three-dimensional model in the visualization; and toggling on an overlay of the representation of the at least a portion of the surgical tool on the two-dimensional image, behind the three-dimensional model in the visualization.

Clause 54. The system of any one of clauses 51-53, wherein the representation of the at least a portion of the surgical tool comprises a silhouette of the at least a portion of the surgical tool.

Clause 55. The system of any one of clauses 51-53, wherein the representation of the at least a portion of the surgical tool comprises an outline of the at least a portion of the surgical tool.

Clause 56. The system of any one of clauses 51-53, wherein the representation of the at least a portion of the surgical tool comprises a three-dimensional representation of the at least a portion of the surgical tool.

Clause 57. The system of any one of clauses 51-56, wherein the representation of the at least a portion of the surgical tool is displayed with an amount of transparency.

Clause 58. The system of any one of clauses 51-57, wherein the one or more programs include instructions for toggling off display of the representation of the at least a portion of the surgical tool.

Clause 59. The system of any one of clauses 51-58, wherein the one or more programs include instructions for determining a three-dimensional pose of the at least a portion of the surgical tool in the two-dimensional image, wherein the representation of the at least a portion of the surgical tool is displayed in the visualization according to the determined three-dimensional pose.

Clause 60. The system of any one of clauses 51-59, wherein the at least a portion of the three-dimensional model comprises a representation of planned bone removal.

Clause 61. A method for guiding bone removal for a surgical procedure, the method comprising: receiving a two-dimensional image of bony anatomy; determining an alignment of a pre-generated three-dimensional model of the bony anatomy to the bony anatomy in the two-dimensional image; and generating and displaying a visualization comprising: a first portion comprising an overlay of at least a portion of the three-dimensional model on the two-dimensional image, wherein the overlay of at least a portion of the three-dimensional model is displayed according to the determined alignment, and a second portion comprising a representation of at least a portion of the three-dimensional model and a representation of the determined alignment of the three-dimensional model of the bony anatomy to the bony anatomy in the two-dimensional image.

Clause 62. The method of clause 61, wherein the representation of the determined alignment comprises a plane intersecting the at least a portion of the three-dimensional model according to the determined alignment.

Clause 63. The method of clause 61 or 62, wherein the representation of the determined alignment comprises a representation of the two-dimensional image intersecting the at least a portion of the three-dimensional model according to the determined alignment.

Clause 64. The method of any one of clauses 61-63, wherein the orientation of the representation of at least a portion of the three-dimensional model is fixed relative to the orientation of the representation of the determined alignment of the three-dimensional model of the bony anatomy to the bony anatomy in the two-dimensional image and the two representations together form a combined representation that is rotatable.

Clause 65. The method of any one of clauses 61-64, wherein the at least a portion of the three-dimensional model comprises a representation of planned bone removal.

Clause 66. The method of any one of clauses 61-65, wherein the representation of the determined alignment corresponds to a clock face line of the bony anatomy.

Clause 67. The method of clause 66, comprising: cutting a cross-section of the three-dimensional model according to the clock face line; and displaying the cross-section of the three-dimensional model.

Clause 68. The method of clause 67, comprising displaying the representation of the determined alignment with the cross-section of the three-dimensional model.

Clause 69. A system for guiding bone removal for a surgical procedure, the system comprising one or more processors, a memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; and generating and displaying a visualization comprising: a first portion comprising an overlay of at least a portion of the three-dimensional model on the two-dimensional image, wherein the overlay of at least a portion of the three-dimensional model is displayed according to the determined alignment, and a second portion comprising a representation of at least a portion of the three-dimensional model and a representation of the determined alignment of the three-dimensional model of the bony anatomy to the bony anatomy in the two-dimensional image.

Clause 70. The system of clause 69, wherein the representation of the determined alignment comprises a plane intersecting the at least a portion of the three-dimensional model according to the determined alignment.

Clause 71. The system of clause 69 or 70, wherein the representation of the determined alignment comprises a representation of the two-dimensional image intersecting the at least a portion of the three-dimensional model according to the determined alignment.

Clause 72. The system of any one of clauses 69-71, wherein the orientation of the representation of at least a portion of the three-dimensional model is fixed relative to the orientation of the representation of the determined alignment of the three-dimensional model of the bony anatomy to the bony anatomy in the two-dimensional image and the two representations together form a combined representation that is rotatable.

Clause 73. The system of any one of clauses 69-72, wherein the at least a portion of the three-dimensional model comprises a representation of planned bone removal.

Clause 74. The system of any one of clauses 69-73, wherein the representation of the determined alignment corresponds to a clock face line of the bony anatomy.

Clause 75. The system of clause 74, wherein the one or more programs include instructions for: cutting a cross-section of the three-dimensional model according to the clock face line; and displaying the cross-section of the three-dimensional model.

Clause 76. The system of clause 75, wherein the one or more programs include instructions for displaying the representation of the determined alignment with the cross-section of the three-dimensional model.

Clause 77. A method comprising: receiving a two-dimensional image of bony anatomy; determining an alignment of a pre-generated three-dimensional model of the bony anatomy to the bony anatomy in the two-dimensional image; and determining an alignment of a three-dimensional model of an instrument relative to the two-dimensional image; determining, based on the alignments of the pre-generated three-dimensional models of the bony anatomy and instrument to the bony anatomy in the two-dimensional image, a location on a surface of the three-dimensional model of the bony anatomy that is proximate to the tip of the three-dimensional model of the instrument; and generating and displaying a visualization comprising: an overlay of at least a portion of the three-dimensional model on the two-dimensional image, and a virtual mark in the visualization at a location that corresponds to the location on the surface of the three-dimensional model of the bony anatomy that is proximate to the tip of the aligned three-dimensional model of the instrument.

Clause 78. A system for guiding bone removal for a surgical procedure, the system comprising one or more processors, a memory, and one or more programs, wherein the one or more programs are stored in the memory and configured to be executed by the one or more processors, the one or more programs including instructions for: receiving a two-dimensional image of bony anatomy; determining an alignment of a pre-generated three-dimensional model of the bony anatomy to the bony anatomy in the two-dimensional image; and determining an alignment of a three-dimensional model of an instrument relative to the two-dimensional image; determining, based on the alignments of the pre-generated three-dimensional models of the bony anatomy and instrument to the bony anatomy in the two-dimensional image, a location on a surface of the three-dimensional model of the bony anatomy that is proximate to the tip of the three-dimensional model of the instrument; and generating and displaying a visualization comprising: an overlay of at least a portion of the three-dimensional model on the two-dimensional image, and a virtual mark in the visualization at a location that corresponds to the location on the surface of the three-dimensional model of the bony anatomy that is proximate to the tip of the aligned three-dimensional model of the instrument.

Clause 79. A method for guiding bone removal comprising: displaying, by a computing system, a visualization comprising: a first two-dimensional image that comprises anatomy and a surgical instrument, and a three-dimensional model aligned with the anatomy in the first two-dimensional image, the three-dimensional model comprising a visual indication of planned bone removal, wherein the display of the first two-dimensional image and the three-dimensional model visually indicates to a user a position of the surgical instrument relative to the planned bone removal; in accordance with the surgical instrument being positioned at a first location of the anatomy that corresponds to a first location of a perimeter of the planned bone removal, using, by the user, the surgical instrument to mark the first location of the anatomy; updating, by the computing system, the visualization to include a second two-dimensional image that comprises the anatomy and the surgical instrument, the surgical instrument having been repositioned by the user; and in accordance with the surgical instrument being positioned at a second location of the anatomy that corresponds to a second location of a perimeter of the planned bone removal, using, by the user, the surgical instrument to mark the second location of the anatomy.

Clause 80. The method of clause 79, wherein the surgical instrument comprises an RF ablation instrument or a bur.

Clause 81. The method of clause 79 or 80, comprising resecting the bone based on the markings of the first and second locations.

## Claims

1. A system for guiding bone removal for a surgical procedure, the system comprising one or more processors, a memory, and one or more programs, wherein the one or more programs are stored in the memory and configured to be executed by the one or more processors, the one or more programs including instructions for:
receiving a two-dimensional image of bony anatomy;
determining an alignment of a pre-generated three-dimensional model of the bony anatomy to the bony anatomy in the two-dimensional image; and
generating and displaying a visualization comprising:
a first portion comprising an overlay of at least a portion of the three-dimensional model on the two-dimensional image, wherein the overlay of at least a portion of the three-dimensional model is displayed according to the determined alignment, and
a second portion comprising a representation of at least a portion of the three-dimensional model and a representation of the determined alignment of the three-dimensional model of the bony anatomy to the bony anatomy in the two-dimensional image.

2. The system of claim 1, wherein the representation of the determined alignment comprises a plane intersecting the at least a portion of the three-dimensional model according to the determined alignment.

3. The system of claim 1 or 2, wherein the representation of the determined alignment comprises a representation of the two-dimensional image intersecting the at least a portion of the three-dimensional model according to the determined alignment.

4. The system of any one of claims 1-3, wherein the orientation of the representation of at least a portion of the three-dimensional model is fixed relative to the orientation of the representation of the determined alignment of the three-dimensional model of the bony anatomy to the bony anatomy in the two-dimensional image and the two representations together form a combined representation that is rotatable.

5. The system of any one of claims 1-4, wherein the at least a portion of the three-dimensional model comprises a representation of planned bone removal.

6. The system of any one of claims 1-5, wherein the representation of the determined alignment corresponds to a clock face line of the bony anatomy.

7. The system of claim 6, wherein the one or more programs include instructions for:
cutting a cross-section of the three-dimensional model according to the clock face line; and
displaying the cross-section of the three-dimensional model.

8. The system of claim 7, wherein the one or more programs include instructions for displaying the representation of the determined alignment with the cross-section of the three-dimensional model.

9. A method for guiding bone removal for a surgical procedure, the method comprising:
receiving a two-dimensional image of bony anatomy;
determining an alignment of a pre-generated three-dimensional model of the bony anatomy to the bony anatomy in the two-dimensional image; and
generating and displaying a visualization comprising:
a first portion comprising an overlay of at least a portion of the three-dimensional model on the two-dimensional image, wherein the overlay of at least a portion of the three-dimensional model is displayed according to the determined alignment, and
a second portion comprising a representation of at least a portion of the three-dimensional model and a representation of the determined alignment of the three-dimensional model of the bony anatomy to the bony anatomy in the two-dimensional image.

10. The method of claim 9, wherein the representation of the determined alignment comprises a plane intersecting the at least a portion of the three-dimensional model according to the determined alignment, and/or a representation of the two-dimensional image intersecting the at least a portion of the three-dimensional model according to the determined alignment.

11. The method of claim 9 or 10, wherein the orientation of the representation of at least a portion of the three-dimensional model is fixed relative to the orientation of the representation of the determined alignment of the three-dimensional model of the bony anatomy to the bony anatomy in the two-dimensional image and the two representations together form a combined representation that is rotatable.

12. The method of any one of claims 9-11, wherein the at least a portion of the three-dimensional model comprises a representation of planned bone removal.

13. The method of any one of claims 9-12, wherein the representation of the determined alignment corresponds to a clock face line of the bony anatomy.

14. The method of claim 13, comprising:
cutting a cross-section of the three-dimensional model according to the clock face line; and
displaying the cross-section of the three-dimensional model.

15. The method of claim 14, comprising displaying the representation of the determined alignment with the cross-section of the three-dimensional model.
